# EUROPEAN PATENT SPECIFICATION

(11) **EP 0 836 482 B1**
(45) Date of publication and mention of the grant of the patent: **13.11.2002**
(21) Application number: 96917828.4
(22) Date of filing: 24.05.1996
(51) Int. Cl.: A61K 39/12, A61K 39/193, A61K 39/295, C07H 21/04, C07K 14/18, C12P 21/06, C12N 1/20

(54) **SUBUNIT VACCINE AGAINST FLAVIVIRUS INFECTION**
UNTEREINHEITSIMPFSTOFF GEGEN FLAVIVIRUS INFEKTION
VACCIN PURIFIE CONTRE UNE INFECTION PAR FLAVIVIRUS

(30) Priority: 24.05.1995 US 448734; 07.06.1995 US 488807; 10.07.1995 US 500469
(43) Date of publication of application: 22.04.1998
(73) Proprietor: HAWAII BIOTECHNOLOGY GROUP, INC., Aiea, HI 96701 (US)
(72) Inventor: IVY, John, M., Kailua, HI 96734 (US); NAKANO, Eileen, Honolulu, HI 96818 (US); CLEMENTS, David, Honolulu, HI 96816 (US)
(74) Representative: Vossius, Volker, Dr.
(86) International application number: PCT/US96/07627
(87) International publication number: WO 96/037221

(56) References cited:
- WO-A-88/02779
- WO-A-90/01946
- WO-A-92/02548
- WO-A-92/03161
- GB-A- 2 191 201
- US-A- 5 514 375
- JIANMIN ET AL.: "Analysis of functional epitopes on the dengue 2 envelope (E) protein using monoclonal IgM antibodies" ARCHIVES OF VIROLOGY, vol. 140, no. 5, May 1995 (1995-05), pages 899-913, XP000199967 Austria
- ARCH. VIROL., Volume 139, issued 1994, DELENDA et al., "Protective Efficacy in Mice of a Secreted Form of Recombinant Dengue-2 Virus Envelope Protein Produced in Baculovirus Infected Insect Cells", pages 197-207.
- P. W. Mason et al., Journal of General Virology, Vol. 71, 1990, pages 2107-2114
- Robert Putnak, "Progress in the Development of recombinant vaccines against dengue and other Arthropod-Borne-Flaviviruses, page 231-252, Chapter 11 IN: Modern Vaccinology, ed: E. Kurstak, Plenum Medical, New York, 1994.

## Description

### Technical Field

The invention relates to protection against and diagnosis of the conditions caused by flavivirus. More specifically, the invention concerns subunits of the flavivirus envelope protein secreted as mature recombinantly produced proteins from eucaryotic cells. Additional viral proteins or subunits, also produced in this way, provide additional active ingredients. These E-proteins or subunits, alone or in combination including combination with additional viral-derived peptides are protective against infection by flavivirus, raise antibodies useful in passive immunization, and are useful in diagnosis of infection by the virus.

### Background Art

The flavivirus family, Flaviviridae, includes the dengue viruses Japanese encephalitis (JEV) virus, Tick-borne encephalitis (TBE) virus, and the initially discovered prototype of this class, the yellow fever (YF) virus. The flaviviruses contain a single positive strand genomic RNA and are small enveloped viruses affecting animals, but generally transmitted to vertebrates by chronically infected mosquito or tick vectors. Flaviviruses are enveloped by host cell membrane and contain the three structural proteins capsid (C), membrane (M), and envelope (E). The E and M proteins are found on the surface of the virion where they are anchored in the membrane. Mature E is glycosylated, whereas M is not, although.its precursor, prM, is a glycoprotein. Glycoprotein E, the largest structural protein, contains functional domains responsible for cell surface attachment and intraendosomal fusion activities. It is also a major target of the host immune system, inducing neutralizing antibodies, protective immunity, as well as antibodies which inhibit hemagglutination.

Dengue virus is the causative agent of dengue fever and is transmitted to man by Aedes mosquitoes, principally Aedes aegypti and Aedes albopictus. Classic dengue fever is an acute illness marked by fever, headache, aching muscles and joints, and rash. A fraction of cases, typically in children, results in more extreme forms of infection, i.e., dengue hemorrhagic fever (DHF) or dengue shock syndrome (DSS). Without diagnosis and prompt medical intervention, the sudden onset and rapid progress of DHF/DSS can be fatal.

Dengue is one of the most important virus groups transmitted to man by arthropods in terms of global morbidity; it has been estimated that dengue is responsible for up to 100 million illnesses annually. With the advent of modern jet travel, dengue has spread globally in the tropics and subtropics, and multiple dengue serotypes in a region are common.

Every flavivirus genome is a single positive-stranded RNA of approximately 10,500 nucleotides containing short 5' and 3' untranslated regions, a single long open reading frame (ORF), a 5' cap, and a nonpolyadenylated 3' terminus. The ten gene products encoded by the single, long open reading frame are contained in a polyprotein organized in the order, C (capsid), prM/M (membrane), E (envelope), NS1 (nonstructural), NS2a, NS2b, NS3, NS4a, NS4b, and NS5 (Chambers, T.J. et al. Ann Rev Microbiol (1990) 44:649-688). Processing of the encoded polyprotein is initiated cotranslationally, and full maturation requires both host and viral-specific proteases. The sites of proteolytic cleavage in the YF virus have been determined by comparing the nucleotide sequence and the amino terminal sequences of the viral proteins. Subsequent to initial processing of the polyprotein, prM is converted to M during virus release (Wengler, *G. et al*. J Virol (1989) 63:2521-2526), and anchored C is processed during virus maturation (Nowak *et al.* Virology (1987) 156:127-137).

There are four antigenically related dengue viruses which, however, can be recognized as distinct serotypes. The complete genomic sequence for at least one strain of each of the four dengue serotypes has been reported (DEN-1, Fu, J. *et al.* Virology (1992) 188:953-958; DEN-2, Deubel, V. *et al.* Virology (1986) 155:365-377; Hahn, Y.S. *et al.* Virology (1988) 162:167-180; DEN-3, Osatomi, K. *et al.* Virus Genes (1988) 2:99-108; Osatomi, K. *et al.* Virology (1990) 176:643-647; DEN-4, Zhao, B.E. et *al.* Virology (1986) 155:77-88; Mackow, E. *et al.* Virology (1987) 159:217-228). In addition, the complete genomic sequences of other flaviviruses are known (e.g., YF virus: Rice *et al.*, Science (1985) 229:726-733).

It does not appear that infection by one dengue serotype can confer long-term immunity on the individual with respect to other serotypes. In fact, secondary infections with heterologous serotypes are quite common. In general, antibody responses in infected subjects to primary infection are mostly IgM antibodies and these antibodies are directed against type-specific determinants. On the other hand, secondary infections by heterologous serotypes generate IgG antibodies which are flavivirus crossreactive.

Helpful reviews of the nature of the dengue disease, the history of attempts to develop suitable vaccines, and structural features of flaviviruses in general as well as the molecular structural features of the envelope protein of flaviviruses are found in Halstead, S.B. Science (1988) 239:476-481; Brandt, W.E. J Infect Disease (1990) 162:577-583; Chambers, T.J. *et al.* Annual Rev Microbiol (1990) 44:649-688; Mandl, C.W. *et al.* Virology (1989) 63:564-571; and Henchal, E.A. and J.R. Putnak, Clin Microbiol Rev (1990) 3:376-396.

Other flavivirus family members, i.e., JEV, TBE and YF appear presently to represent single serotypes -- i.e., antibodies elicited in response to one strain are cross reactive with other known strains.

A successful vaccine for protection against flavivirus infection has never been developed. However, there have been a number of preliminary efforts, many of which focus on the envelope protein, since this protein is exposed at the surface and is believed to be responsible for eliciting immunity.

Monoclonal antibodies (Mabs) directed against purified E of several flaviviruses, DEN-2 (Henchal *et al.* Am J Trop Med Hyg (1985) 34:162-169, TBE (Heinz, F.X. *et al.* Virology (1983) 126:525-537), St. Louis encephalitis (SLE, Mathews, J.H. *et al.* J Immunol (1984) 132:1533-1537), Murray Valley encephalitis (MVE, Hawkes, R.A. *et al.* J Gen Virol (1988) 69:1105-1109), and JEV (Takegami, T. *et al.* Acta Virologica (1982) 26:312-320) are neutralizing in vitro. Some of these Mabs can also passively confer protection in vivo (Heinz, F.X. *et al.* (1983, *supra*)); Mathews, J.H. *et al.* (1984, *supra*)); Kimuro-Kuroda and Yasui, J Immunol (1988) 141:3603-3610).

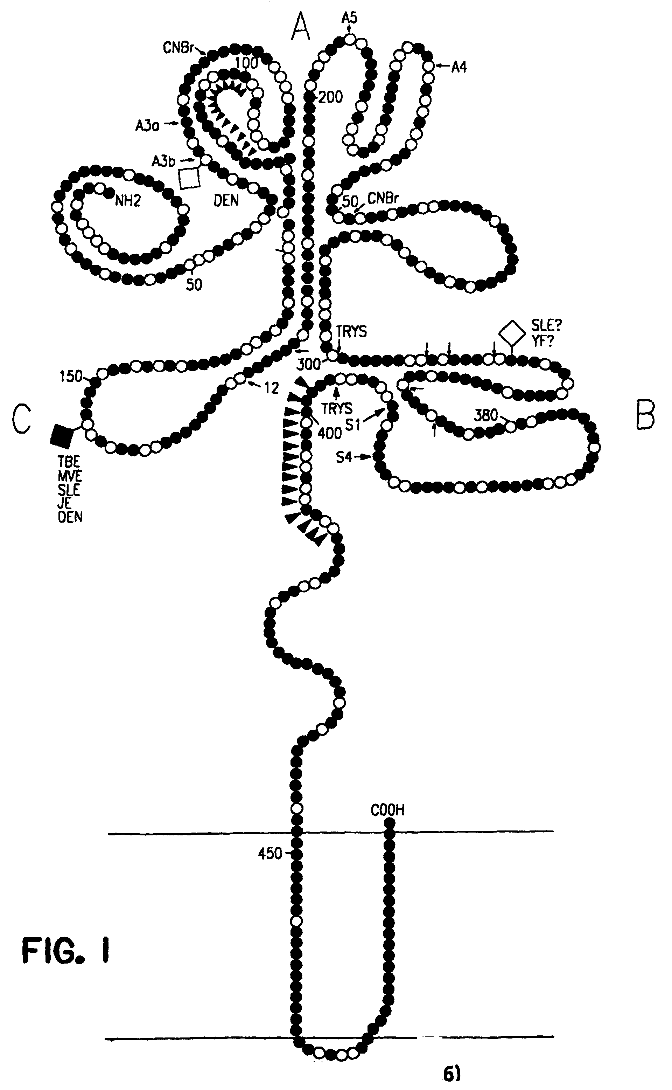

Although the primary amino acid sequence of the flavivirus E glycoprotein is variable (45-80% identity), all have twelve conserved cysteine residues, forming six disulfide bridges, and hydrophilicity profiles are nearly superimposable, suggesting that they may all have similar secondary and tertiary structures. Based on the position of the 12 conserved cysteines (determined for West Nile virus, Nowak and Wengler, Virology (1987) 156:127-137), monoclonal antibody competitive binding studies, monoclonal antibody binding to purified proteolytic fragments, and analysis of neutralizing antibody escape mutants of Tick-Borne Encephalitis Virus, glycoprotein E was divided into three antigenic domains (A, B, and C) and two transmembrane segments at its carboxy-terminus. See, for example, Mandl, C.W. *et al*. J Virol (1989) 63:564-571. Figure 1, reproduced from this article, shows the locations of these domains.

Domain A was defined as a denaturation sensitive, highly folded, and disulfide stabilized discontinuous domain composed of the amino acids from 50-125 and 200-250 containing five of the six disulfide bridges. Neutralization and hemagglutination inhibition epitopes are found within domain A, and, for dengue viruses, one of the two N-linked glycosylation sites. A conserved hydrophobic amino acid sequence within domain A has been postulated to provide fusogenic properties after low pH treatment. Amino acid sequences conserved among the flavivirus family are located within this region; thus, broadly flavivirus-cross-reactive epitopes lie within this domain.

Domain B was identified as a continuous domain composed of amino acids 301-395 (an approximate region between amino acids 300-400 for all flaviviruses). The domain can be isolated as a single immunoreactive proteolytic fragment. It has been postulated that this domain forms part of the receptor binding site (Heinz, F.X. *et al.* APMIS (1993) 101:735-745), and attenuating mutations have been mapped to sequences within domain B Heinz *et al.* (*supra*). A variety of neutralizing antibodies have been shown to specifically map to Domain B (Heinz *et al.* (1983, *supra*)); Trirawatanapong *et al*., 1992; Megret *et al*., 1992; Lin *et al.,* 1994). The binding of these neutralizing monoclonal antibodies is dependent on formation of a disulfide bond, and in some cases also is sensitive to detergent denaturation. Species-specific monoclonal antibodies bind this domain.

Domain C represents a hypervariable loop between the two segments of Domain A. Its antigenicity is insensitive to denaturation or reducing agents, and contains one N-linked glycosylation site. Predominantly sub-type specific monoclonal antibodies react with this domain. No specific activity has been assigned to this domain.

Many strategies are currently under investigation to develop an effective and safe dengue vaccine; however, to date, no single strategy has proven completely satisfactory. Attempts to generate live attenuated dengue vaccine strains have not been entirely successful, although research into this area continues. In the absence of effective, live attenuated dengue vaccines, a significant effort has been invested in the development of recombinant, dengue subunit or viral-vectored vaccines.

Recombinant dengue proteins have been expressed in several systems to date (see Putnak, R.A. (1994) Modern Vaccinology, E. Kurstak ed., Plenum Medical, New York, pp. 231-252, for review). Most efforts using Escherichia coli have yielded poor immunogen unable to elicit neutralizing antibodies. This may reflect nonnative conformation of dengue proteins expressed in the bacteria and the necessity to process the viral proteins through the secretion pathway in order to form the proper disulfide bonds, glycosylate the proteins, or both.

Several reports have described vaccinia-flavivirus recombinants expressing envelope protein as part of a polyprotein (e.g. C-prM-E-NS1; [Dengue] Zhao, B.G. *et al.* J Virol (1987) 61:4019-4022; Deubel, V. *et al.* J Gen Virol (1988) 69:1921-1929; Bray, M. *et al.* J Virol (1991) 63:2853-2856; [YF] Hahn, Y.S. *et al.* Arch Virol (1990) 115:251-265), as a single protein (e.g. 100%E; [Dengue] Bray, M. *et al*., J Virol (1989) 63:2853-2856), or as polypeptides (e.g. 79%E-RKG; Men, R. *et al*. J Virol (1991) 65:1400-1407). The most successful recombinant vaccinia viruses, those capable of inducing neutralizing antibodies and protecting mice from virus challenge, were those which were secreted E extracellularly or accumulated E on the cell surface.

Men, R. *et al*. (1991, *supra*) described the recombinant production of various C-terminal truncations of the DEN-4 envelope protein using a recombinant Vaccinia virus vector and infecting mammalian CV1 cells. The results showed that the recombinants that contain greater than 79% of the coding sequence produced an intracellular protein that could be immunoprecipitated with anti-dengue virus antibodies contained in hyperimmune mouse ascitic fluid (HMAF). Although there was a reduced level of detection for protein which contained 79% of envelope or less, this did not appear to result from reduced production of the protein. It was also found that only truncations which contained 79% of E or less were secreted efficiently; E polypeptides equal to or larger than 81%E were not secreted efficiently.

Men *et al.* (1991, *supra*) constructed additional C-terminal truncations between 79%E and 81%E to map the amino acids responsible for the difference in secretion and immunoreactivity with HMAF of these two truncated E polypeptides. The results demonstrated that 79% E containing the additional tripeptide sequence RKG was also secreted. Although both 59% E and 79% E-RKG were secreted, only 79% E-RKG was detected at the cells' surface. The recombinant Vaccinia viruses containing various truncations were also used to immunize mice. Mice immunized with recombinants expressing 79%E-RKG or larger portions of the envelope protein were protected. However, except for 59% E, mice immunized with 79% E or a smaller product were only partially protected. The 59%E elicited high protection rates (>90%) comparable to 79%E-RKG and larger C-terminal truncated E polypeptides. Protection correlated with binding to HMAF.

Combinations of immunogenic structural and nonstructural JEV virus, DEN-1, DEN-2, and DEN-4 proteins have been expressed by baculovirus recombinants(Matsuura, Y. *et al.* Virology (1989) 173:674-682; Putnak, R.A. *et al.* Am J Trop Med Hyg (1991) 45:159-167; Deubel, V. *et al.* Virology (1991) 180:442-447). Baculovirus-expressed dengue and JE E glycoprotein elicited neutralizing antibodies, protected mice from a lethal dengue virus challenge, or both. In spite of these successes, the expression levels reported in baculovirus are low and the recombinant protein is less immunogenic than the viral protein (Putnak, R.A. *et al.* Am J Trop Med Hyg (1991) *supra*).

Research with purified polypeptides released by proteolysis of flavivirus envelope proteins, with recombinant polypeptides, and with synthetic peptides has indicated where protective epitopes may map. The isolated 9000 dalton domain B trypsin fragment from TBE virus spontaneously refolds and is stabilized by disulfide bridges (Winkler, G. *et al.* J Gen Virol (1987) 68:2239-2244). This disulfide stabilized fragment elicited neutralizing antibodies in mice (Heinz, F.X. *et al.* Virology (1984) 130:485-501). In contrast, a 28,000 dalton trypsin fragment from WN virus containing domain B sequences was unable to spontaneously refold and did not elicit neutralizing antibodies (Wengler and Wengler, 1989).

A cyanogen bromide-cleaved 8 kD fragment (amino acids 375-456) overlapping domain B from JEV envelope protein was found to induce neutralizing antibodies in mice (Srivastava, A.K. *et al*. Acta Virol (1990) 34:228-238). Immunization of mice with a larger polypeptide (JE E amino acid 319 to NS1 amino acid 65) spanning the 8 kD peptide expressed in Escherichia coli as a fusion to protein A elicited neutralizing antibodies and protected mice from lethal virus challenge (Srivastava, A.K. *et al*. Microbiol Immunol (1991) 35:863-870). This polypeptide begins between the two cysteines within domain B, and, therefore, cannot form the stabilizing disulfide bond that earlier reports suggest is necessary for presentation of protective epitopes.

Immunization of mice with synthetic peptides corresponding to amino acids within domain B, aa 356-376 from MVE (Roehrig, J.T. *et al.* Virology (1989) 171:49-60) or aa 352-368 from DEN-2 virus (Roehrig, J.T. *et al.* Virology (1990) 177:668-675), elicited low levels of neutralizing antibodies in mice, suggesting the presence in domain B of a weak linear neutralizing epitope (Roehrig, J.T. *et al.* 1989 and 1990, *supra*).

Mason, P.W. *et al.* J Gen Virol (1990) 71:2107-2114 identified two domains of the DEN-1 envelope protein: domain I which includes amino acids 76-93 of the E protein and domain II (equivalent to domain B) which includes amino acids 293-402. These domains were identified from deletion analysis using recombinant fusion proteins expressed in *E. coli* and reacted with antiviral monoclonal antibodies. Recombinant fusion proteins containing *E. coli* trpE sequences fused to the envelope protein (amino acids 1 to 412) elicited antibodies in mice which reacted with a portion of the protein containing domain II.

In addition, Mason, P.W. *et al*. (J Gen Virol (1989) 70:2037-2049) expressed a collection of *E. coli trpE* fusion proteins to segments of JEV virus envelope protein spanning domain B. The *trpE* fusion proteins containing the smallest JEV E fragments that retained immunoreactivity with a panel of neutralizing monoclonal antibodies included amino acid residues from methionine 303 through tryptophan 396. However, animals immunized with immunoreactive *trpE* fusion polypeptides did not produce neutralizing antibodies nor were they protected from lethal challenge.

Trirawatanapong, T. *et al.* Gene (1992) 116:139-150 prepared several truncated forms of dengue 2 envelope proteins in *E. coli* for epitope mapping, and used the neutralizing monoclonal antibody 3H5 to locate its corresponding epitope. This was first localized between amino acids 255 and 422. Targeted gene deletions in the plasmid constructs encoding the truncated proteins permitted mapping of the binding site to the 12 amino acids between positions 386 and 397. The mapping was apparently confirmed by the ability of a synthetic peptide containing E protein amino acids 386-397 to bind 3H5 specifically.

Megret, F. *et al.* Virology (1992) 187:480-491 prepared 16 overlapping fragments of DEN-2 envelope protein as trpE fusion products in *E. coli* for epitope mapping. The fusion proteins are produced intracellularly and obtained from the lysates. These products were used to map epitopes defined by a panel of 20 monoclonal antibodies. Six antigenic domains were described: nonneutralizing antibodies bound to peptides containing amino acids 22-58, amino acids 304-332, amino acids 60-97, and amino acids 298-397. Neutralizing antibodies bound to peptides containing amino acids 60-135, 60-205, and 298-397.

Significantly, Megret *et al.* (1992, *supra*) demonstrated that all MAbs (including 3H5), with two exceptions (below), that recognize "full-length" domain B (amino acids 298-397) are unable to recognize slightly shorter polypeptides. For example, in contrast to the findings of Trirawatanapong *et al.* Gene (1992, *supra*), MAb 3H5 was unable to bind to trpE fusion proteins containing DEN-2 E amino acids 304-397, 298-385, or 366-424. The two exceptional MAbs in the findings of Megret *et al.* are MAbs 5A2 and 9D12. The pattern of binding of MAb 5A2 suggests that it recognizes a linear epitope between amino acids 304 to 332, while MAb 9D12 binds to a polypeptide, amino acids 298-385, which is slightly shorter than the smallest polypeptide (amino acids 298-397) to which MAb 3H5 binds. These results indicate that both the disulfide bond in domain B and the domain B C-terminal amino acids are involved in forming the immunodominant domain B epitopes.

U.S. Patent No. 5,494,671, issued 27 February 1996 discloses C-terminally truncated dengue and JEV envelope proteins for use in vaccines. This patent is the U.S. counterpart of PCT Application WO 92/03161 published 5 March 1992. The truncated E proteins were either generated in situ using viral vectors, or in insect cells, also using viral vectors.

Although it appears established from the art that the B domain and subunits representing at least about 80% E (truncated at the C-terminus), of the flavivirus envelope protein contain epitopes which bind neutralizing antibodies, problems have arisen with respect to producing recombinant polypeptides, all of which contain the B domain, in a form which mimics the native protein and is thus capable of eliciting an immune response. The only recombinantly produced E polypeptides containing the B domain that elicited a protective immune response in mice were expressed from Vaccinia and baculovirus vectors. Generally, recombinantly produced proteins lack the appropriate glycosylation, folding, and disulfide bond formation for producing a proper immune response.

It has now been found that the B domain and other shortened forms of the envelope protein can be successfully secreted from yeast and from *Drosophila* in a form which elicits the production of neutralizing antibodies. This permits, for the first time, the production of a successful recombinantly produced subunit flavivirus vaccine.

### Disclosure of the Invention

The invention provides vaccines containing, as an active ingredient, a secreted recombinantly produced the flavivirus envelope protein or a subunit thereof. The vaccines are capable of eliciting the production of neutralizing antibodies against the relevant flavivirus. In the illustrations below, Peptide subunits representing 80%E are secreted from *Drosophila* cells using the human tissue plasminogen activator secretion signal sequence for the propeptide (tPA_{L}) or from the homologous premembrane (prM) leader. The secreted products can easily be purified and prepared as a vaccine.

Thus, in one aspect, the invention is directed to a vaccine for protection of a subject against infection by flavivirus. The vaccine contains, as active ingredient, the envelope protein of a flavivirus serotype or a subunit thereof. The E or subunit is secreted as a recombinantly produced protein from eucaryotic cells under the control of a nonviral promoter. The vaccine preferably contains portions of more than one flavivirus serotype E protein similarly produced, and may further include additional flavivirus proteins, particularly NS-1, which can be similarly recombinantly produced.

In other aspects, the invention is directed to methods to protect subjects against infection by administering the invention vaccines, to antibodies generated in a mammalian subject administered an immunogenic amount of the vaccine; immortalized B cell lines which generate monoclonal antibodies of this type; methods to effect passive immunization by administering the antibodies of the invention; methods to detect the presence or absence of antiflavivirus immunoglobulins utilizing the secreted recombinantly produced peptides of the invention and the recombinant materials important in, methods for, their production.

### Brief Description of the Drawings

Figure 1 is a drawing reproduced from Mandl, *et al.* (*supra*) showing a model of the envelope protein of flaviviruses.
Figure 2 (SEQ ID NO:1) shows the partial nucleotide sequence for DEN-2 PR159 S1 mutant strain and differences from the wild-type strain reported by Hahn (1988, *supra*).
Figure 3 (SEQ ID NO:2 and SEQ ID NO:3) shows the partial nucleotide sequence and deduced amino acid sequence of the genome of DEN-2 PR159/S1 strain in comparison with wild-type.
Figure 4 shows the survival times of mice immunized with *D. melanogaster* Schneider cell-secreted 80%E.

### Modes of Carrying Out the Invention

The invention provides, for the first time, a subunit vaccine that can be efficiently produced recombinantly and secreted that is effective in protecting subjects against infection with flavivirus. Although many attempts have been made to obtain such a subunit vaccine, either the subunit itself is resistant to recombinant production techniques which permit it to be secreted in a processed form so as to render it effective as an immunogen, or, if its recombinant production is facile, it fails to elicit neutralizing antibodies. The present applicants have found that certain portions of the envelope protein of dengue virus type 2, such as 80%E is effectively secreted by certain convenient eucaryotic recombinant hosts, in a form that permits processing to mimic the native conformation of the protein. The hosts generally utilize nonviral control sequences. The secretion of the protein into the culture medium facilitates purification. Furthermore, this form is able, when administered, especially in the presence of adjuvant, to raise neutralizing antibodies in animals. Thus, this subunit represents a useful component of a vaccine for protecting subjects against dengue infection.

Other portions of the E protein illustrated below are self-explanatory. 80%E is the N-terminal 80% of the protein from residue 1 to approximately residue 395. These precentages are approximate and may include 1-4 amino acid residues more or less than that calculated precisely. These subunits are produced from vectors containing the DNA encoding the mature subunit or protein or may be included in a prM fusion which results in secretion of the 80%E *per se.*

For practical large-scale production of the subunits used as active ingredients in the vaccines of the invention, recombinant techniques provide the most practical approach. However, to be useful as active ingredients, the subunits as produced must assume a conformation and undergo processing under conditions which render them similar to the native envelope portion as it exists in the envelope protein of the virus. In order to achieve this, the recombinant production must be conducted in eucaryotic cells, preferably yeast or *Drosophila* cells. In addition to the *S. cerevisiae* and *P. pastoris* yeasts illustrated below, other yeasts, such as *Kluveromyces sp Yarrowia lipolytica,* and *Schizosaccharomyces pombe* may be used. Other insect cells besides the *Drosophila melanogaster Schneider* cells illustrated below may also be employed. Additional appropriate eucaryotic cells include mammalian expression cells such as Chinese hamster ovary cells. 80%E must be produced as a correctly processed protein and secreted.

It has been found, as demonstrated hereinbelow, that particularly efficient secretion of biologically active mature protein can be, achieved in several ways. First, this can be done by expressing the protein in yeast in operable linkage with the α-mating factor signal sequence. Constructs which place the nucleotide sequence encoding the E protein or subunit disposed so as to encode a fusion protein with an upstream α-mating factor signal sequence are therefore included within the scope of the invention. An additional preferred embodiment employs *Drosophila* cells and the human tissue plasminogen activator leader sequence for secretion of 80%E, and other E proteins and subunits. This general expression system is disclosed in EP 290261 published 11 September 1988. Envelope protein subunits that represent N-terminal portions of truncated protein may also be secreted from the homologous prM fusion. Other secretion signal peptides or secretion leader pre/pro peptides, such as those associated with invertase or acid phosphatase of *S. cerevisiae* or with glucoamylase of *C. albicans* or of *A. niger* or the bovine chymosin prepropeptide secretion leader can also be used. Secretion leaders in general that occur at the amino terminus of secreted proteins and function to direct the protein into the cellular secretion pathway generically can be used. In general, the invention includes expression systems that are operable in eucaryotic cells and which result in the formation of envelope protein or a subunit secreted into the medium. Preferably, nonviral control sequences are employed. Thus, useful in the invention are cells and cell cultures which contain expression systems resulting in the production and secretion of the E protein or subunit.

The properly processed E protein or subunit is recovered from the cell culture medium, purified, and formulated into vaccines. Purification and vaccine formulation employ standard techniques and are matters of routine optimization; however, particularly advantageous and novel purification methods are described below. Suitable formulations are found, for example, in Remington's Pharmaceutical Sciences, latest edition, Mack Publishing Company, Easton, PA. In particular, formulations will include an adjuvant, such as alum or other effective adjuvant. Alternatively, the active ingredient and the adjuvant may be coadministered in separate formulations.

The active vaccines of the invention can be used alone or in combination with other active vaccines such as those containing attenuated forms of the virus. The vaccines may contain only one subunit as an active ingredient, or additional isolated active components may be added. Corresponding or different subunits from one or several serotypes or flavivirus types may be included in a particular formulation.

More specifically, the subunits utilized in the vaccines of the invention can include the E protein or subunits thereof from each or a subset of the four dengue serotypes, and/or E protein or subunits from alternative flavivirus such as Japanese encephalitis virus, tick-borne encephalitis virus and the yellow fever virus. Multiple subunits of the E protein can also be included, as well as, optionally, other viral proteins, particularly the NS1 protein. These alternative subunits derived from other proteins of the virus can be recombinantly produced in a manner similar to the E protein and subunits described above.

Thus, typical combinations might include:
80%E of the four dengue serotypes plus 60%E of JEV;
80%E of JEV;
80%E of TBE plus 60%E of dengue;
80%E of the four dengue serotypes plus NS1 protein of four dengue serotypes;
80%E of two dengue seroptypes plus NS1 protein or fragment from JEV;
Domain B of JEV plus 80%E of TBE;
Domain B of YF plus 80%E of JEV plus 80%E of dengue type 2;
60%E of JEV plus domain B of four dengue seroptypes plus NS1 or fragment of both dengue and JEV;
80%E of JEV plus NS1 of JEV;
80%E of TBE plus NS1 of TBE
80%E of YF plus NS1 of YF.

To immunize subjects against dengue fever or other flavivirus infections, the vaccines are administered to the subject in conventional immunization protocols involving, usually, multiple administrations of the vaccine. Administration is typically by injection, typically intramuscular or subcutaneous injection; however, other systemic modes of administration may also be employed. Although the technology is not as well developed, transmucosal and transdermal formulations are included within the scope of the invention as are effective means of oral administration. The efficacy of these formulations is a function of the development of formulation technology rather than the contribution of the present invention.

Since the subunit vaccines elicit the production of neutralizing antibodies, the antibodies thus raised can themselves be used as passive vaccines. For production of passive vaccine, a suitable mammalian subject is immunized with the E protein or subunit of the invention and antibodies are either recovered directly as a composition from the antisera or indirectly as monoclonal antibodies from immortalized B cells of the subject. For production of monoclonal antibodies, the conventional techniques of Kohler & Milstein, for example, or treatment with Epstein Barr virus, are used in immortalizing peripheral blood lymphocytes or spleen cells and screening for antibodies immunoreactive with the immunogen. These antibodies may further be screened for their ability to effect plaque reduction in infected sera or cultures.

The polyclonal antisera are generally subjected to purification techniques such as standard size separation and chromatographic techniques to obtain purified immunoglobulins. The recombinantly produced proteins of the invention are particularly useful affinity ligands for chromatographic purification. A multiplicity of techniques is available for the purification of immunoglobulins for use in passive vaccines. If monoclonal antibodies are to be purified from small volumes of a medium ascites fluid, a protein A affinity column is particularly useful. For larger volumes, additional standard chemical techniques, such as ammonium sulfate precipitation and DEAE chromatography can be used. These immunoglobulins or the monoclonal antibodies generated by the immortalized B cells are then used in vaccine formulations as is understood in the art.

As is the case with active vaccines, the passive vaccines of the invention may be used in combination with additional antibodies or tandem administration of the antibodies of the invention and additional antibodies may be employed.

In the event that the passive vaccine is intended for use in a species other than that of the subject in which the antibodies were prepared, it may be desirable to modify the antibodies to minimize any immunogenic response. For example, it may be possible to use only the variable regions of these antibodies, such as the F_{ab}, F_{ab'}, or F_{(ab')₂} regions. These fragments can be prepared either from polyclonal antisera or from the supernatants of hybridoma cultures by treating with proteolytic enzymes and recovering the desired fragments. The fragments are readily separated by using the relevant protein of the invention as an affinity reagent.

Alternatively, chimeric antibodies can be produced wherein the constant region corresponding to the species to be protected is substituted for the constant region characteristic of the species of antibody origin. The availability of recombinant techniques makes the production of chimeric antibodies a relatively trivial exercise. Briefly, a hybridoma or cell line producing the antibody of interest is used as a source for the genes encoding the antibody. The genes are recovered from, for example, the hybridoma using standard cloning procedures. The genes are then manipulated *in vitro* to remove the constant region and replace it with a constant region of a different species origin. The modified genes are then ligated into expression systems and expressed in recombinant host cells, such as CHO cells, monkey cells, yeast cells, and the like.

Further modifications in the variable regions can also reduce immunogenicity. Again, since recovery of the genes encoding the antibody is within the skill of the art, the variable regions, too, can be manipulated to replace the framework regions with framework regions more representative of the desired species, leaving intact the complementarily determining regions responsible for antigen specificity. In still another embodiment, the variable heavy chain and variable light chain regions can be linked through a peptide linker and produced as a single chain Fᵥ molecule.

Thus, if the passive vaccines are intended for humans, the foregoing various techniques of humanizing antibodies can be employed to minimize any immunogenic response even though the original antibodies are raised in nonhuman species.

In addition to use in vaccines or in the generation of passive vaccines, the mature recombinant E protein and subunits of the invention may be used as analytical reagents in assessing the presence or absence of antiflavivirus antibodies in samples. The interest in doing this may be diagnosis of infection with flavivirus, monitoring response to infection or may simply reside in the use of immunoassays as part of standard laboratory procedures in the study of the progress of antibody formation or in epitope mapping and the like. The antigen is employed in standard immunoassay formats with standard detection systems such as enzyme-based, fluorescence-based, or isotope-based detection systems. Preferably, the antigen is used coupled to solid support or in sandwich assays, but a multiplicity of protocols is possible and standard in the art.

Thus, the secreted protein, such as 80%E may be adsorbed onto solid support and the support then treated with a sample to be tested for the presence of antiflavivirus antibodies. Unbound sample is removed, and any bound antibodies are detected using standard detection systems, for example, by treating the support with an antispecies antibody with respect to the species represented in the sample to be tested, the antispecies antibody coupled to a detection reagent, for example, horseradish peroxidase (HRP). The presence of the HRP conjugated antispecies antibody is then detected by supplying a suitable chromogenic substrate.

Alternatively, the anti-subunit or antiflavivirus antibody may be adsorbed to the solid support and detected by treating the solid support with the recombinant domain B, either directly labeled, or labeled with an additional antibody in a sandwich-type assay.

In addition, both the mature peptide, such as 80%E of the invention and the antibodies immunoreactive with it can be used in standard purification procedures as affinity reagents. Thus, purification of the subunits from recombinantly produced cultures can be effected by affinity columns using antibodies raised against these antigens. Conversely, immunoglobulins useful in passive vaccines can be readily purified from antisera using the peptides of the invention.

A subunit of the invention may be used to detect the presence or absence of antibodies of various isotypes, including IgG and IgM isotypes. As set forth above, detection of IgM isotypes is significant since this is an index of primary infection.

In the examples below, particular subunits of the dengue Type 2 envelope protein, in particular 80%E are illustrated as representative of effective subunits of the envelope protein in flavivirus in general. For the 80%E constructs in general, secretion can be obtained from constructions designed to express the prME subunit fusion or under control of the human tissue plasminogen activator leader sequence. The mature N-terminus of the envelope protein is then secreted into the culture medium. Whether the N-terminus of the envelope protein subunits were fused to a heterologous leader, such as the human tissue plasminogen activator leader sequence, or to the homologous prM sequence or C-terminal portion thereof, the mature form of the truncated envelope protein is secreted. The secreted truncated Es are expressed at high levels in *Drosophila*, efficiently processed, and secreted into the medium. The products are glycosylated and processed to an endo-H resistant form. The secreted form of truncated E produced cotranslationally with prM generally represents about 20-30% of the total protein in the medium. Furthermore, based upon reactivity with conformationally sensitive monoclonal antibodies, using a ELISA and immunofluorescence formats, the secreted E products are shown to have a native conformation. Immunization of mice with crude medium from transformed cells expressing prM-truncated E induces a potent virus-neutralizing response.

The following examples are intended to illustrate but not to limit the invention. Any references to 60%E, to the B-domain or to 100%E in the following examples are given for illustrative purposes only.

### Example 1

### Preparation of Envelope Proteins in Saccharomyces cerevisiae

A cDNA clone derived from dengue serotype 2 (DEN-2) described by Hahn, Y.S. *et al.* Virology (1988, *supra*) was used as the starting material. This cDNA derives from strain PR159/S1. This strain has a small plaque, temperature-sensitive phenotype; the complete sequence of the cDNA derived from the genomic RNA for PR159/S1 is set forth in this publication.

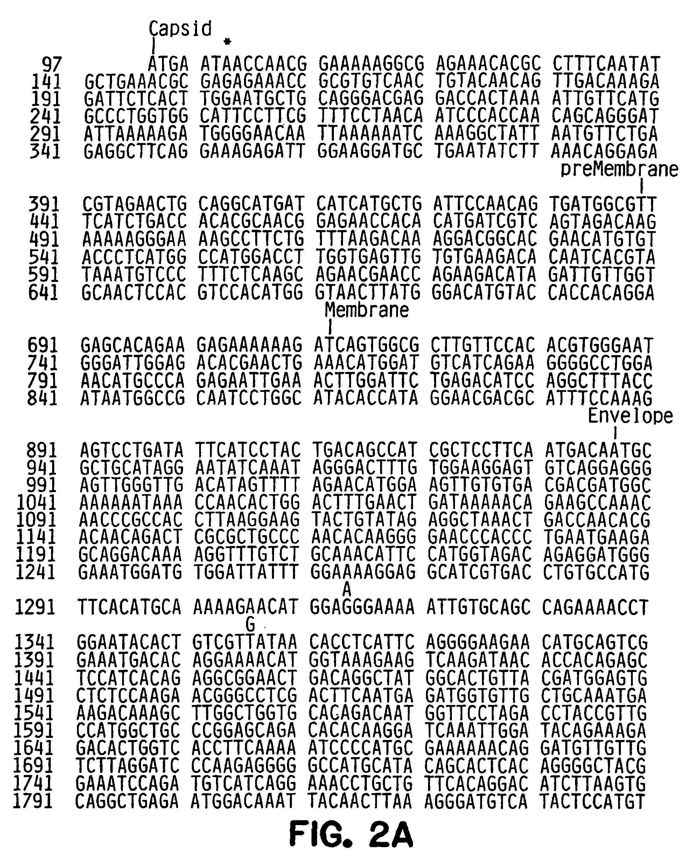

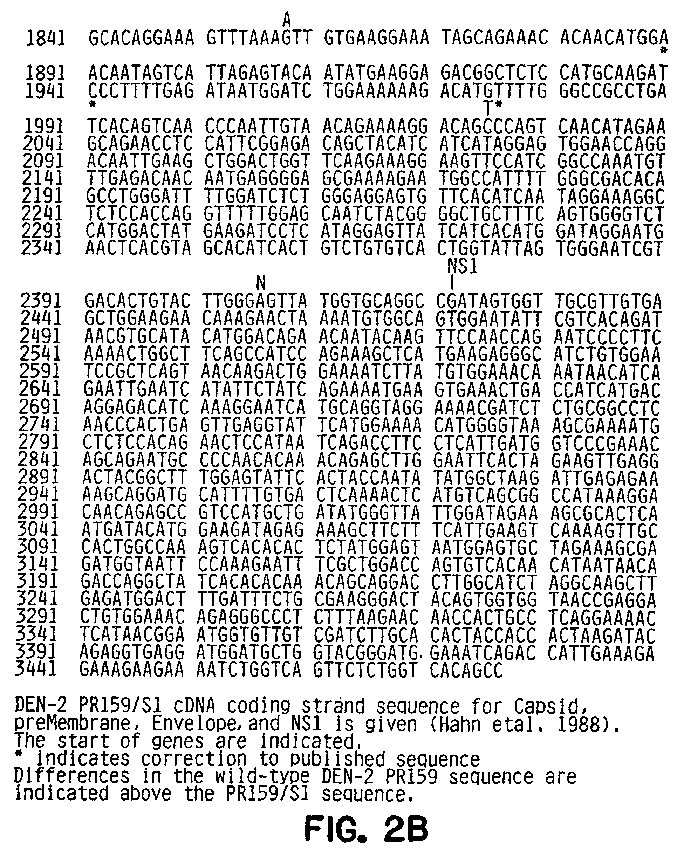

Figure 2 shows the sequence of the cDNA derived from genomic RNA of DEN-2 PR159/S1 for the Capsid, preMembrane, Envelope, and NS1 genes. Shown in bold at nucleotides 103, 1940, 1991, and 2025 are corrections to the Hahn published sequence. Differences in the S1 sequence from the wild-type sequence are noted above the wild-type sequence. There are no nucleotide differences in the Capsid and preMembrane protein-encoding portions and there are four in the E encoding portion.

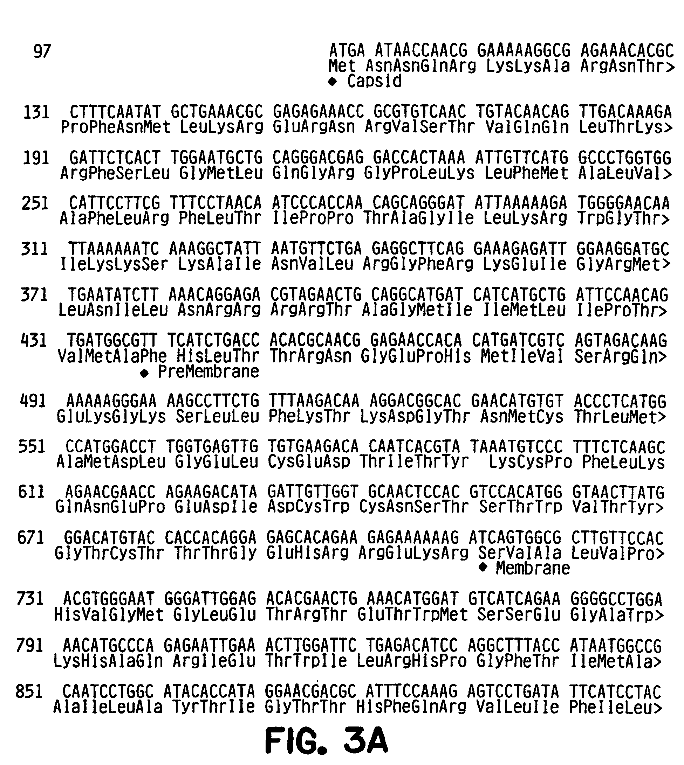

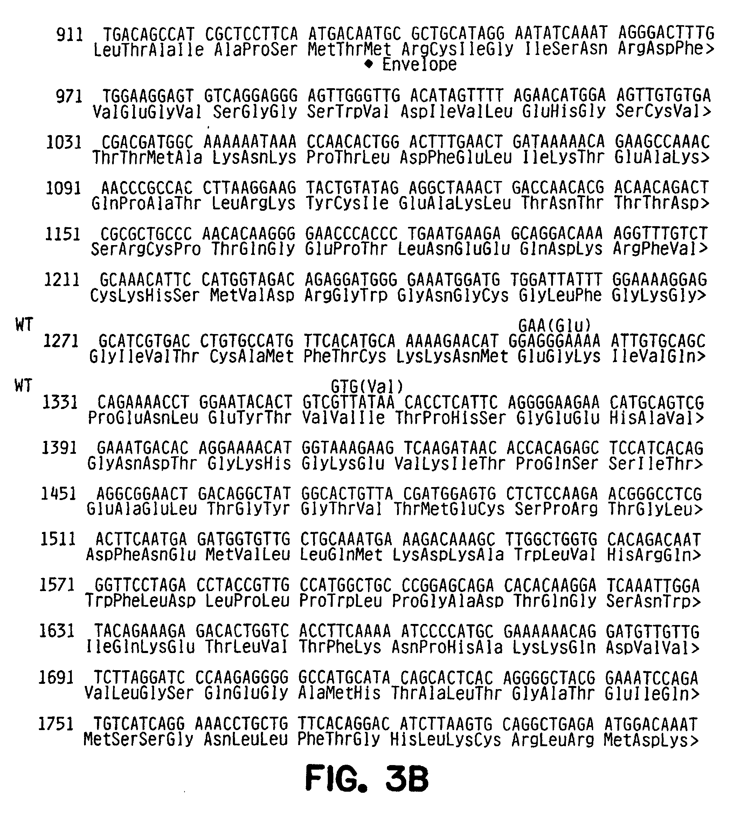

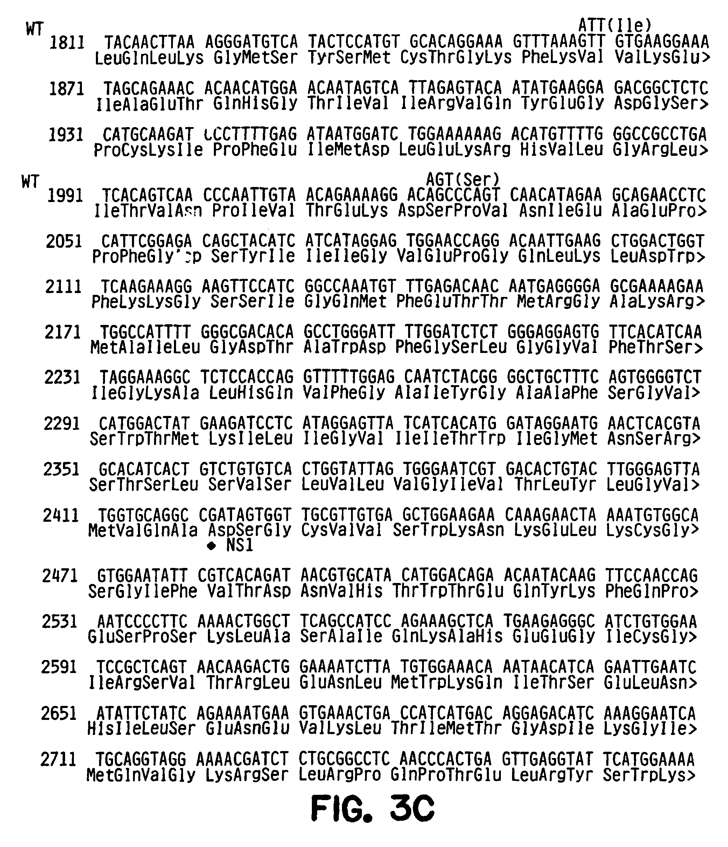

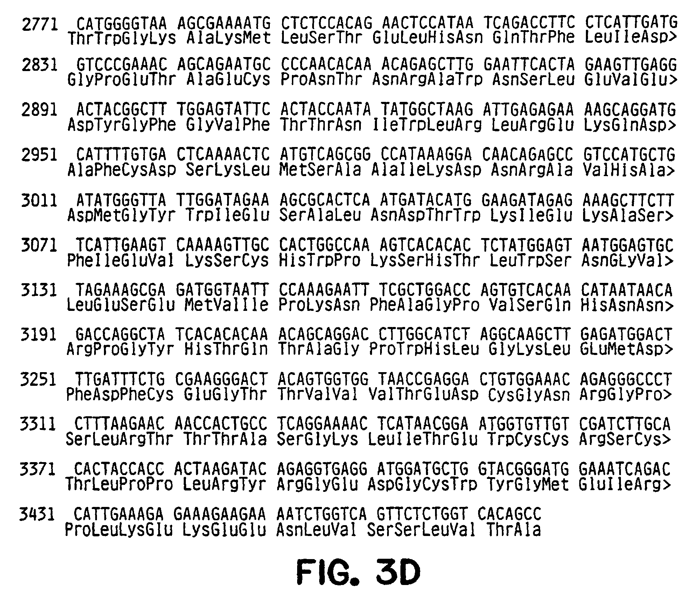

Figure 3 shows the cDNA sequence of DEN-2 PR159/S1 for the Capsid, preMembrane, Envelope, and NS1 genes and the inferred translation of those four genes, which is part of the larger dengue polyprotein. The four differences between wild-type DEN-2 PR159 and the S1 strain are shown above the S1 nucleotide sequence. Also shown is the context of the codons in which the nucleotide differences occur and the encoded amino acid.

In the E gene, three of the four mutations are silent; S1 has G instead of A at position 1314, T rather than G at position 1356, and C rather than T at position 2025. The mutation at position 1858, a G in S1 rather than A, results in a coding change from Ile in wild-type to Val in S1. There is, therefore, a one amino acid difference in the B domain in the S1 strain as compared to wild-type. Although the amino acid substitution conferred by the mutation at 1858 is conservative, mutagenesis studies of other viral structural proteins (Coller, B.G. *et al.* (1994) Mutagenesis Studies on the Predicted VP2 E-F Loop of Coxsackievirus B3, Abstract, 13th Annual Meeting of the American Society for Virology) have demonstrated that even relatively conservative single amino acid changes in surface loops can have profound effects on viral biology, including infectivity and viral stability. Thus, it is not without foundation that the conservative mutation in domain B of DEN-2 PR159/S1 may be involved in the attenuation of this small-plaque, temperature-sensitive variant.

Subsequent determinations on the S1 strain used herein (S1/HBG) showed that the A at position 103 in wild-type was retained in position 103 in S1/HBG and C was substituted for T at position 2025 (a silent mutation). A deduced amino acid sequence from DEN-2 PR159/S1 and differences occurring in the wild-type are shown in Figure 3.

Various E gene subclones were obtained which represented the amino-terminal 90% of the envelope, 80% of the envelope, 60% of the envelope and classical domain B. Using the assignment of Mandl, C.W. *et al*. J Virol (1989) 63:564-571, classical domain B starts at Gly296 and ends at Gly395. Classical domain B contains only one disulfide bond and is encoded by the sequence following domains A and C. This classical domain B does not contain the potential T cell epitope described by Mandl *et al.* at its carboxy end which can be included in some forms of the domain B of the invention, e.g., DomB+T.

The portion of the genome that encodes 80% of the envelope protein (80%E) was amplified using the Polymerase Chain Reaction, primers D2E937p and D2E2121m, and plasmid pC8 (Hahn *et al.* (1988, *supra*) as template.

In this notation of the primers, the virus serotype is first indicated (D2 for DEN-2), then the corresponding dengue gene -- i.e., in this case envelope, E, is noted. Then is noted the number in the dengue cloned sequences of Figures 2 or 3 for the first dengue nucleotide in the 5'-3' direction of the oligonucleotide, i.e., using the numbering of Hahn *et al.* (1988, *supra*), and finally the notation shows whether the oligonucleotide primes the plus (p) or the minus (m) strand synthesis. The sequence in the primers corresponding to dengue cDNA are written in uppercase letters; nondengue sequence is written in lowercase letters.

D2E937p2 (SEQ ID NO:13)

BglII
5'-cttctagatctcgagtacccgggacc ATG CGC TGC ATA GGA ATA TC-3'
XbaI XhoI SmaI

D2E2121m (SEQ ID NO:14)

SalI
5'-gctctagagtcga cta tta TCC TTT CTT GAA CCA G-3'
XbaI End End

The D2E2121m primer placed two stop codons after the 395th codon of E. The 80%E amplified cDNA fragment was digested at the *Xba*I sites in the cloning adapters and cloned into the *Nhe*I site of pBR322 to obtain p29D280E. Double-strand sequence for 80%E was determined, which identified a single silent PCR-introduced mutation at nucleotide 2001 (AAC/Asn to AAT/Asn).

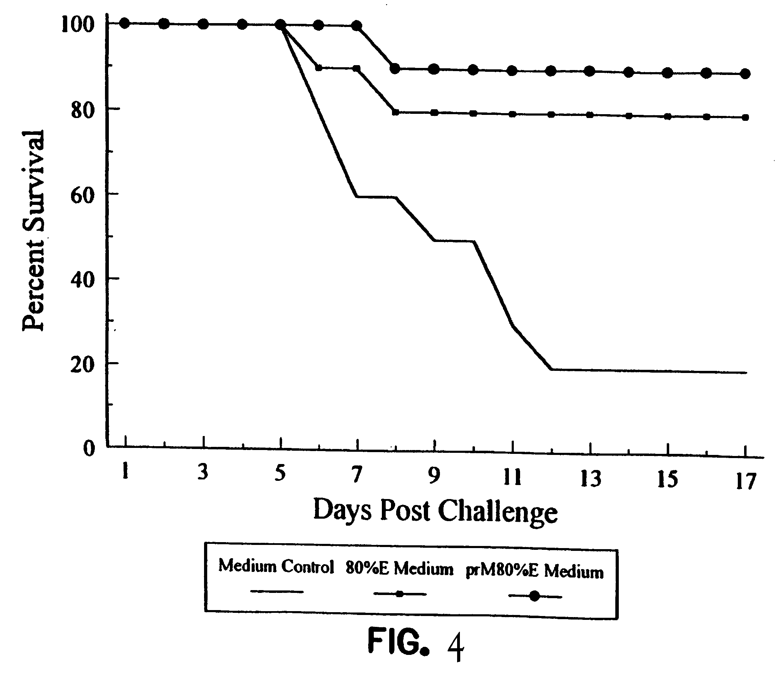

A subclone representing domain B was obtained from the 80%E subclone by oligonucleotide-directed mutagenesis. In the mutagenesis, stop codons and restriction endonuclease sites were inserted between domain C- and domain B-encoding sequences. The stop codons were positioned to terminate domain A+C translation and SalI and PvuII restriction sites were added to facilitate subcloning of domains A+C and domain B fragments, respectively, into yeast expression vectors. As shown in Figure 4, to avoid a high AT content in the mutagenic oligonucleotide, the stop codons defining the carboxy-terminus of 60%E containing domains A and C were positioned four codons upstream of the beginning of domain B, i.e., following Lys291. The original and altered nucleotide sequences of the mutagenized region and the corresponding amino acid translation are shown in Figure 4.

To perform the mutagenesis, a 580 bp BamHI fragment spanning domain B from the pBR322-80%E clone p29D280E was subcloned into pGEM3Zf (Promega) to yield p29GEB2. (See Figure 5.) This BamHI fragment encodes the 3' end of domains A and C and all of domain B. The plasmid obtained following mutagenesis, p29GEB24PS, was confirmed by DNA sequencing. As shown in Figure 6, the introduced PvuII site permits direct subcloning of domain B cDNA into yeast expression vectors as a fusion with yeast secretion leader.

The cloned cDNA fragments encoding B domain and 80%E were inserted into expression vectors so as to maintain the translational frame of fusions to secretion leaders as described below. The sequence of the fusions were confirmed by DNA sequencing.

### Example 2

### Secretion of Domain B from Saccharomyces cerevisiae

The expression vector constructed to secrete classical domain B from *Saccharomyces cerevisiae* to include envelope protein amino acids 296-395 was constructed so that processing by the proteases normally involved in preproMFα_{L} processing would yield a precisely processed domain B. One of these proteases, Kex2p, the gene product of the *Kex2* gene, cleaves following dibasic peptides, such as LysArg, LysLys, ArgArg, and ArgLys. A second protease involved, a dipeptidyl amino peptidase, removes GluAla and AspAla dipeptides from the amino terminus after Kex2p processing.

The MFα prepropeptide (MFα_{L})-domain B fusion was operably linked to the constitutive promoter from the gene (*TDH3*) encoding glyceraldehyde phosphate dehydrogenase (GAPDH) contained in pLS5 and, alternatively, to the copper sulfate-inducible *CUPI* gene promoter contained in pLS6. These vectors were provided by SmithKline Beecham. Both contain the MFα_{L} sequence, and StuI, BglII, and SalI cloning sites, and use the *TRP1* gene as a selectable marker. They contain sequences derived from pBR322 to provide an *E. coli* origin of replication, the ampicillin resistance gene, and sequences derived from the 2-micron plasmid of *S. cerevisiae* to enable replication in *S. cerevisiae.* The insertion of the classical domain B coding sequence into the appropriate reading frame in pLS5 was accomplished by digesting pLS5 with StuI and Sail and by digesting p29GEB24PS with PvuII and Sail and gel purifying the small PvuII-SalI domain B fragment and ligating it into the thus opened pLS5 as shown in Figure 6. A domain B expression vector derived from pLS6 was constructed in a similar manner.

The nucleotide and amino acid sequence of the MFα_{L}-domain B fusion is shown in Figure 7.

The resulting expression vectors were transfected into spheroplasts of *Saccharomyces cerevisiae* strain GL43 (*MATatrp1Δ1 ura3-52 proA::URA3*) and transformants were selected by their ability to grow on minimal medium without tryptophan supplementation. *S. cerevisiae* strain GL43 was supplied by SmithKline Beecham. Strain GL43 pLS5-DomB and pLS6-DomB transformants were grown in small (<5ml) cultures i Casamino acid-supplemented minimal medium, and proteins secreted into the culture medium and cellular proteins were analyzed by Coomassie-stained SDS-PAGE and Western blot. A novel Coomassie-staining band of approximately 12 kD, which was weakly immunoreactive with DEN-2 HMAF, was observed in the culture media of domain B transformants. No novel Coomassie-staining bands and little or no immunoreactive protein not found in negative controls was observed in cellular protein extracts of domain B transformants. When cultured in Casamino acid supplemented minimal medium, comparable levels of domain B were secreted into the medium by pLS5-DomB and pLS6-DomB transformants. About 20% of the total secreted protein was domain B as estimated by the intensity of Coomassie brilliant blue staining of protein in an SDS-PAGE polyacrylamide gel.

The composition of the medium was optimized; when *S. cerevisiae* containing pLS5-DomB were grown in minimal medium (SD) or Casamino acids (Difco)-supplemented minimal medium, the yields of protein were less than when cultured in rich (YEPD) medium. The pLS5-domain B transformant was preferred since this transformant can be cultured in rich medium whereas pLS6-domain B transformants require the use of the less preferred supplemented minimal medium in order to induce with copper sulfate.

The secreted protein was confirmed as domain B having a Glu-Ala dipeptide appendage to the N-terminus, designated herein Glu-Ala-Domain B or Glu-Ala-DomB. For this demonstration, proteins secreted by a pLS5-DomB transformant were separated by SDS-PAGE and electroblotted to Immobilon P membrane (Millipore), and the amino terminal amino acid sequence was determined by microsequencing. That sequence is: H₂N-Glu Ala Gly Met Ser Tyr Ser Met Xxx Thr Gly Lys Phe Xxx Val Val (SEQ ID NO:15). The persistence of the GluAla dipeptide indicates that the dipeptidyl aminopeptidase incompletely processed the domain B N-terminus following proteolysis by Kex2p.

### Example 3

### Purification of Domain B

### A. Initial Purification Method:

Pilot purifications of domain B from *S. cerevisiae* culture medium used size exclusion chromatography on acrylamide Biogel P100.

For these purifications, an *S. cerevisiae* GL43 pLS5-DomB transformant was cultured in minimal medium overnight and then transferred to either Casamino acid-supplemented minimal (SD) medium or rich (YEPD) medium for expression. After culturing at 30°C for 2-3 days and feeding daily with buffered glucose to final concentrations of 0.4% glucose and 5 mM sodium phosphate (pH 6.7), the cells were pelleted by centrifugation and the medium was clarified by filtration through a 0.45 µm pore filter. The filtered medium was concentrated about 30-fold either by tangential flow or by centrifugal ultrafiltration using Minitan (Millipore) or Centricon (Amicon) devices, and the concentrate was exchanged into PBS azide buffer (2.7 mM KCl, 1.2 mM KH₂PO₄, 138 mM NaCl, 4.3 mM Na₂HPO₄·7H₂O, 0.02% azide) by diafiltration or dialysis.

In one example, the concentrated secreted proteins from approximately 500 ml of culture of GL43 pLS5-DomB grown in SD plus Casamino acids were loaded onto a 2.5 x 75 cm Biogel P100 column and domain B was eluted from the column at approximately 1 bed volume. Domain B was pooled based on SDS-PAGE analysis of column fractions. The pooled domain B fractions were brownish in color and could not be decolorized by dialysis. The brown colored preparation also was not immunoreactive in ELISAs. DEAE chromatography of this sample resulted in binding of the brown color and a mostly colorless flow-through containing domain B. For DEAE chromatography, the brown-colored pooled fractions were dialyzed against 0.1 M acetic acid, pH 5.1 and loaded onto a 1.4 x 15 cm DEAE (Biorad) column. Domain B was eluted using a 0.01 M acetic acid, pH 5.1, 0.1 M NaCl step gradient.

The purified domain B was of high purity as assessed by silver stain on SDS-PAGE. The resulting Glu-Ala-DomB also gained weak reactivity with antidengue mouse polyclonal HMAF and with antidengue MAbs 3H5 and 9D12 in an ELISA format. The reactivities of earlier colorless preparations from smaller cultures were greater than this initial large-scale preparation. For the ELISA, 96-well microtiter plates were coated with domain B by overnight incubation at 4°C and then blocked with BSA in 50 mM tris-HCl, pH 7.0, 0.15 M NaCl, 0.05% Tween for 1 hour at room temperature. The plates were then treated with either DEN-2 HMAF or monoclonal antibody. Bound antibody was detected with alkaline phosphatase conjugated goat antimouse IgG and measured by the increase in optical density at 414 nm using the alkaline phosphatase substrate paranitrophenylphosphate.

The domain B frequently eluted from the Biogel P100 in two equal fractions, the first with the bulk of the secreted yeast proteins and the second as 70-90% pure domain B. Treatment with 1M NaCl, 1 and 2 M urea, and 1% DDAPS, a zwitterionic detergent, were ineffective in completely disaggregating domain B during size exclusion chromatography. Since the domain B of both fractions had the same electrophoretic mobility in the presence of detergent and absence of DTT, this polypeptide was neither covalently cross-linked to another protein nor self-polymerized via disulfide bonds.

### B. Improved Purification Method:

A standard culture and purification method was developed that has reproducibly yielded immunoreactive domain B expressed and secreted by *S. cerevisiae* GL43 pLS5-DomB. For that method, a primary culture is prepared by inoculating 60 ml of SD medium in a 250 ml baffled flask with a single colony of yeast strain GL43 pLS5-DomB grown on an SD plate. SD medium is prepared by autoclaving 0.67% Bacto yeast nitrogen base without amino acids, cooling and providing 2.0% w/v dextrose and 12 mM K₂HPO₄ using autoclave stock solutions. Solid SD medium is prepared by including . Bacto agar at 1.8%. The culture is grown at 30°C for 18-24 hours with shaking at 240 rpm.

Secondary cultures are then grown in 300 ml of SD medium supplemented with 0.2% casamino acids in 2 L baffled flasks. The secondary cultures are inoculated with 30 ml of the primary culture. The secondary cultures are incubated at 30°C for 18-24 hours with shaking at 300 rpm.

Large-scale tertiary cultures are then prepared by inoculating 1 L of YEPD medium in a 4 L baffled flask with 100 ml of the secondary culture. YEPD is prepared by autoclaving 1% yeast extract plus 2% Bactopeptone, to which sterile dextrose is added to a final concentration of 2% after cooling. The large-scale cultures are incubated at 30°C for 48 hours with shaking at 300 rpm. After 24 hours, the culture is supplemented with 0.01 vol of sterile 40% w/v glucose and 0.02 vol of sterile 1 M phosphate buffer, pH 6.7.

After growth in the 1 L culture, the cells are removed by centrifugation at 5,000 rpm at 4°C for 5 min. in a Sorvall GS-3 rotor. EDTA and EGTA are added to final concentrations of 1 mM each to the cleared medium, and the resulting solution is filter sterilized using a 045 µm pore filter membrane (Millipack-20 or Opticap-50, Millipore). Glycerol is added to 10% v/v to the filtrate which is then concentrated 20-30 fold using tangential flow ultrafiltration (Millipore Minitan System) with two membrane cartridges (four regenerated cellulose membranes) of a 10 kD MW cutoff. The retentate is kept on ice during ultrafiltration in the coldroom at 4°C for about 10-15 hours.

The concentrated medium is directly used or rapidly frozen on dry ice/ethanol in 10 ml aliquots in 15 ml polypropylene or polystyrene tubes with a screw cap and stored at -70°C.

Concentrated medium of the tertiary culture is dialyzed at 4°C using a membrane with a 6-8 kD cutoff (Spectra/Pore-1 Membrane) against 10 mM acetate, pH 4.5 (4X4 liters; 2-3 hours each; 1 overnight).

For purification, the concentrated, buffer-exchanged medium is loaded onto a 2.5 x 22 cm CM Biogel A (Biorad) column at 0.8 ml/min. at 4°C. The column is washed with 150-250 ml of 10 mM acetate, pH 4.5 until the optical density at 280 nm of the eluent drops to baseline. Domain B is then eluted with 10 mM acetate, 300 mM NaCl, pH 4.5 at 0.8 ml/min. Fractions of approximately 8 ml are collected and analyzed by SDS-PAGE (15%) and visualized by silver staining. Domain B migrates on the trailing edge of the main peak of eluted material as monitored by 280 nm.

The domain B-containing fractions are pooled and concentrated about 11-fold by centrifugal ultrafiltration using, for example, a Centriprep-10 (Amicon) and loaded onto a 5 x 60 cm Sephadex G-75 (Superfine, Pharmacia) column at 4°C. The column is eluted using phosphate-buffered saline (PBS: 8 g/l NaCl, 0.2 g/l KCl, 1.44 g/l Na₂HPO₄, 0.24 g/l KH₂PO₄) at a flow rate of 0.8 ml/min. Nine ml fractions are collected and analyzed by SDS-PAGE as above and by indirect ELISA as above using Mab 9D12.

The Sephadex G-75 fractions containing pure immunoreactive domain B from 2-4 purification runs are concentrated about 50-fold by centrifugal ultrafiltration using a Centriprep-10 (Amicon; final volume 2-5 ml). The pooled material is stored at 4°C.

Glu-Ala-DomB purified as above typically is immunoreactive with the virus-neutralizing monoclonal antibodies 3H5 and 9D12. The above-described culturing and purification protocol has been performed several times with reproducible results. The number of cultures of the above-indicated volumes can be increased, and we have grown simultaneously up to six 1 L tertiary cultures. The culture medium from the multiple cultures is pooled for clarification and concentration, and domain B from the concentrate is purified in batches and pooled following verification of immunoreactivity. The amount of protein in the purified domain B preparations may be quantitated by total amino acid analysis using HCl hydrolysis with on-line ninhydrin reaction and optical detection. Table X presents the yield of protein in purified domain B from four cultures.

**Table X**

| **Total Protein Yields in Purified Domain B Preparations** | | | |
|---|---|---|---|
| **Lot No.** | **Culture Volume** | **Final Yield*** | **Yield mg/l** |
| 198.76 | 850 ml | 4 mg | 4.7 |
| 198.71 | 1500 ml | 35 mg | 23 |
| 244.68 | 5750 ml | 90 mg | 16 |
| 244.190 | 5000 ml | 25 mg | 5 |
| Based on amino acid composition of total protein. | | | |

### Example 4

### Production of Domain B in Pichia pastoris

In addition to *Saccharomyces cerevisiae* as used in the previous examples, the methylotrophic budding yeast *P. pastoris* was used as a host. The domain B coding sequence was subcloned into the expression vector pPIC9 (Invitrogen, San Diego, CA) which contains the alcohol oxidase (AOX1) promoter sequence from *P. pastoris,* the MFα prepropeptide secretion leader from *S. cerevisiae*, the AOX1 transcriptional termination sequence, and the *HIS4* gene from *S. cerevisiae* as a selectable marker. To construct pPIC9-DomB, the domain B-encoding XhoI-SalI fragment from pLS5DomB was inserted into XhoI-digested pPIC9 as shown in Figure 8. *XhoI* cuts at identical positions within the MFα_{L}-encoding DNA of both pLS5 and pPIC9, and *Sal*I cuts immediately following the translational stop codons following domain B-encoding sequences. After verification by DNA sequence analysis, the expression cassette, consisting of the *AOX1* promoter-MFα_{L}-domain B-*AOX1* terminator, plus HIS4 was released from pPIC9-DomB by BglII digestion and the linear DNA was transformed into competent *P. pastoris* cells according to the supplier's instructions (Invitrogen, San Diego, CA). Transformants were selected for the ability to grow on medium lacking histidine (His^{±} phenotype), and specific integration of the transforming DNA at the AOX1 locus was indicated in some transformants by slow growth on medium containing methanol (Mut^{s} phenotype) as the sole carbon source.

The expression and secretion of domain B from eleven *P. pastoris* pPIC9-DomB transformants in 3 ml cultures was surveyed by Coomassie-stained SDS-PAGE analysis. A protein of molecular weight equal to domain B secreted by *S. cerevisiae* was evident in the culture media of ten of the eleven *P. pastoris* transformants. Immunoblots of a comparable polyacrylamide gel demonstrated that the protein secreted by *P. pastoris* pPIC9-DomB transformants was immunoreactive with polyclonal antibodies made to domain B secreted by *S. cerevisiae* (see Example 6). The SDS-PAGE analysis indicated that *P. pastoris* secreted domain B at a level comparable to or higher than that achieved in *S. cerevisiae.*

For further comparison of the secreted expression levels of domain B by *S. cerevisiae* and *P*. *pastoris,* the *S. cerevisiae* transformant and the two best *P*. *pastoris* transformants, Nos. 5-8 (Mut^{±}) and 6-16 (Mut^{s}), were cultured in 100 ml shake flask cultures. *S. cerevisiae* pLS5-DomB was cultured 48 hrs in YEPD medium, and 100 ml precultures of the *P. pastoris* pPIC9-DomB transformants were grown in BMGY medium (1.34% yeast nitrogen base without amino acids, 2% Bacto peptone, 0.4 µg/ml biotin, 1% glycerol, and 100 mM potassium phosphate, pH 6.8) for 24 hrs and then harvested and resuspended in 25 ml of BMMY heterologous protein-inducing medium (the same as BMGY, except with 0.5% methanol replacing the glycerol) and cultured 48 hrs. Culture media with cells removed by sequential centrifugation and filtration through a 0.45 µm pore size membrane were buffer exchanged by diafiltration into TEEN (10 mM Tris, pH 8.0, 1 mM EDTA, 1 mM EGTA, and 150 mM NaCl). The relative levels of domain B secreted by the three cultures were estimated by size fractionating the proteins in 3, 6, 9, and 12 µl of each sample on a 15% SDS-PAGE gel and staining with Coomassie blue. As estimated by the intensity of Coomassie staining, *P. pastoris* secreted at least four-fold more domain B per volume of induction culture medium than *S. cerevisiae* in YEPD. Correcting for the four-fold concentration of culture volume of the *P. pastoris* when transferred into the induction medium, we conclude that *P. pastoris* secretes slightly more than *S. cerevisiae* in shake flask cultures on a per volume basis. However, on a per gram cell dry weight basis, *P. pastoris* (0.80 gm total cell dry weight) secreted approximately 1.6-fold more domain B than *S. cerevisiae* (1.33 gm total cell dry weight).

### Example 5

### Effect of Extending Domain B

In a manner similar to that set forth in the preceding examples, two plasmid constructs for expression in *S. cerevisiae* were made that include additional domain B-carboxy terminal sequence downstream of amino acid 395 with the domain B coding sequence. One construct, designated DomB+stem, represents Gly296-Gly445 and was expressed as a fusion with the MFα_{L}. This construct includes those sequences that lie between domain B and the transmembrane anchor of E. This region contains a potential T cell epitope (Mandl *et al.* J Virol (1989) 63:564-571) and additional hydrophobic sequences, a peptide from this region elicits virus-recognizing antibodies (Roehrig *et al.* 1922 in Vaccines 92, pp. 2777-2281), and this region may contribute to the proper folding and presentation of the domain B B-cell epitope to the immune system.

The domain B+stem cDNA fragment was constructed in *E. coli* cloning vectors by combining the domain B cDNA fragment and the 3' end of a 90%E clone. As introduced in Example 1, an E gene subclone representing the amino terminal 90% of E was constructed from DEN-2 PR159/S1 cDNA plasmid pC8 of Hahn *et al.* (1988, *supra*) using the PCR. The 90%E polypeptide contains all of E except for the C-terminal membrane anchor comprising two transmembrane domains. The 90%E cDNA clone was made as follows. The 90%E fragment was amplified by the PCR using pC8 as template and primers D2E937p and D2E2271m. The sequence of D2E937p is given in Example 1. The sequence of D2E2271m is: 
D2E2271m (SEQ ID NO:16):
SalI
5'-gctctagagtcga cta tta CCC GTA GAT TGC TCC G-3'
XbaI End End
 Primer D2E2271m placed two stop codons after Gly445 of #, and the two primers positioned useful restriction enzyme sites at both ends of the fragment. The PCR-amplified 90%E cDNA fragment was made blunt at both ends and cloned into the *Sma*l site of a modified pUC13 cloning vector, yielding pVZ90E.

Combining domain B and the 90%E 3' end made use of a unique *Afl*III restriction enzyme site found in most pUC-like cloning vectors and a unique *Afl*III site in domain B sequences. This combining was accomplished by first subcloning the 90%E fragment from pVZ90E into pBluescript to reverse the orientation of 90%E relative to the vector sequences, yielding pBS90E. Then, p29GEB24PS, containing domain B sequences in pGEM (Example 1), and pBS90E were digested with *Afl*III, and the vector-domain-B5' fragment and the domain-B3'-stem-vector fragment from the two digestions, respectively, were purified, ligated, and recovered in *E. coli* yielding pBS-Bstem.

For expression in *S. cerevisiae,* the *Pvu*II*-Sal*I domain B+stem fragment from pBS-Bstem was subcloned into the pLS5 yeast expression vector at its *Stu*I and *Sal*l sites, putting the MFα_{L} and B+stem in translational frame. The sequence of the ligated junctions were verified by DNA sequence determination. The domain B+stem expression vector, pLS5-BStem, was transformed into *S*. *cerevisiae* strain GL43. The resulting transformants grew more slowly than did transformants with pLS5-DomB containing classical DomB and the plasmid appeared to be rapidly lost during growth under nonselective conditions. The DomB+stem product may be toxic to the cells, and the hydrophobicity of the stem may inhibit secretion.

However, a similar construct representing Gly296-Ala413 analogously inserted into pLS5 resulted in substantial amounts of secreted domain B protein. This form of domain B, designated herein DomB+T, contains the downstream T cell epitope. As stated above, the DomB proteins of the present invention include classical DomB and all or part of the amino acid sequence downstream from Gly395 to the Ala residue at position 413.

The DomB+T cDNA fragment was amplified by the PCR from pC8 plasmid DNA using oligonucleotide primers D2E1822p and D2E2175m. The primer sequences are: 
D2E1822p KpnI XhoI (SEQ ID NO:17)

5'-cta gcg gta ccc tcg aga AAA GGG AGG CCG GGA TGT CAT ACT CCA TGT GC-3'
 
D2E2175m SalI NotI (SEQ ID NO:18)

5'-cgt gtg tcg acg cgg ccg cta tta GGC CAT TCT TTT CGC TCC-3'
 The PCR product was cloned into pUC18 following *Kpn*I and *Sal*I digestion of the product. The DomB+T cDNA fragment was then released from pUC18-DomB+T by XhoI-SalI digestion and cloned into the XhoI site in pLS5 within the MFα_{L} and Gly395 of domain B+T. *S. cerevisiae* strain GL43 secrets approximately ten-fold more domain B than domain B+T.

### Example 6

### Preparation of Antibodies to the Recombinant Domain B Protein

The Glu-Ala-DomB peptide which had been column-purified, denatured by heating in SDS, and reduced by treatment with DTT was used to immunize Swiss Webster mice following removal of the excess SDS. The mice yielded high-titre antibodies that were highly immunoreactive when used to probe Western blots displaying the same antigen or DEN-2 viral envelope protein.

### Example 7

### Recombinantly Produced Glu-Ala-Domain B in ELISA Assays

The serological diagnosis for dengue infection is based on the detection of anti-dengue IgM and IgG in primary and secondary viral infection using standard Enzyme Linked Immunosorbent Assay (ELISA) techniques. Current assays are based on the ability of anti-dengue immunoglobulins to recognize semi-purified virus. Primary and secondary infections can be distinguished by the IgM:IgG ratios. (Innis *et al.,* 1989; Kuno *et al.,* 1991).

The recombinantly produced Glu-Ala-domain B purified as in paragraph B of Example 3 was tested as an antigen preparation in both IgM and IgG tests. In these ELISAs, the antigen was coated on plates, followed by sera positive for dengue antibodies and then detection by goat antihuman antibody.

Microtiter plates were coated overnight at 4° C with 100 µl of a 5 µg/ml solution of domain B. After blocking with 3% normal goat serum, primary infected dengue 2 positive serum at a 1:100 dilution was added per well. The bound IgM was detected by the addition of goat anti-human IgM (Fc) conjugated to horseradish peroxidase. Under these conditions, the recombinant Glu-Ala-domain B did not provide positive results.

For IgG, high titer secondary dengue 2 infected sera were supplied at a 1:270 dilution and detected with goat anti-human IgG (Fc) conjugated to horseradish peroxidase. Two of the five sera gave positive reactions in this assay.

The recombinant Glu-Ala-domain B purified by the improved procedure of Example 3 is not recognized by murine polyclonal hyperimmune ascitic fluid (HMAF) to other dengue serotypes and to other flaviviruses, when assayed using a similar ELISA format. Flavivirus infected murine sera tested include, Japanese Encephalitis virus, Tick-Borne Encephalitis virus, Yellow Fever virus, Saint Louis Encephalitis virus, West Nile virus, three viral isolates of dengue serotype 1, two viral isolates of dengue serotype 3, and two viral isolates of dengue serotype 4.
Sandwich assay for the detection of any domain B-containing envelope antigen.

In an alternative enzyme immunoassay format, anti-domain B or anti-Dengue capture antibody, polyclonal or monoclonal, may be absorbed to the solid support, and sample containing an unknown quantity or serotype of dengue antigen may be added and then detected by reacting with a second anti-domain B or anti-Dengue antibody, either conjugated to a signal-generating enzyme or to be detected using a appropriate signal generating system, of which there are a multitude. This immunoassay is useful for the quantitation of recombinantly produced envelope protein or whole virus by comparing the immunoreactivity of a known concentration of domain B with that of the unknown antigen preparation. In addition, the conformational sensitivity or epitope binding site of the anti-domain B capture antibody can be varied to garner additional information regarding the conformation of dengue protein in the preparation.

To perform the sandwich enzyme immunoassay, 100 µl of anti-Dengue monoclonal antibody 9D12 or 3H5 (Henchal, E.A. *et al.,* Am J Trop Med Hyg (1985) 34:162-169) was used to coat microtiter wells. The monoclonal antibodies were purified by Protein-A affinity chromatography and used at 10 µg/ml concentration (diluted in PBS: 50 mM sodium Phosphate, pH 7.0, 0.15 M NaCl). After a one hour incubation, the wells were washed three times with TBS-T (50 mM Tris-HCl pH 7.0, 0.15 M NaCl, 0.05% Tween-20), and blocked with 200 µl/well of 1% BSA in PBS for 1 hour at room temperature. Following three washes with TBS-T, 100 µl per well of a standard purified solution of domain B or an unknown sample was added to each well and captured by the bound monoclonal antibody. The domain B solution was allowed to bind the antibody for one hour at room temperature, and after three washes with TBS-T, 100 µl of Protein A affinity purified polyclonal rabbit anti-domain B serum (25 µg/Ml) diluted in PBS-T (PBS, 0.25% BSA, 0.05% Tween-20) was added to each well and allowed to bind to the bound antigen. Following three washes with TBS-T, 100 µl of alkaline phosphatase-labeled goat anti-rabbit IgG+IgM (H and L) conjugate (Caltag) diluted 1:2500 in PBS-T (previously established by titration) was added to each well. After incubation for one hour at room temperature, the plates were washed four times with TBS-T and 200 µl/well of 1 mg/ml *p*-nitrophenylphosphate (pNPP) substrate in alkaline phosphatase substrate buffer (25 mM Trizma base, pH 9.5, 0.15 M NaCl, 5 mM MgCl₂; 0.02% NaN₂) was added. The plates were incubated for one hour at room temperature and the absorbance of each well was measured on a Dynatech MR5000 using a sample wavelength of 410 nm and a reference wavelength of 630 nm. A typical domain B standard curve is presented in Figure 12.

### Example 8

### Immunization of Mice to Raise Neutralizing Antibodies

A. Crude DomB Immunogenicity in Mice: Media from yeast cultures secreting DomB and a negative control yeast culture were buffer-exchanged with PBS, and total secreted proteins were concentrated for injection into mice. Six groups of five mice each (Outbred Swiss strain, Simonsen) were inoculated with the crude DomB preparation, negative control secreted protein, or saline, as listed below.

| **Group** | **Immunogen** | **Adjuvant** |
|---|---|---|
| I | Saline | Freund's |
| II | Saline | none |
| III | control medium | Freund's |
| IV | control medium | none |
| V | DomB medium | Freund's |
| VI | DomB medium | none |

Primary immunizations consisted of 50 µg of the immunogen diluted in phosphate buffered saline, with or without complete Freund's adjuvant as indicated above. Three boosts of 25 µg each were delivered at two-week intervals with no adjuvant (Groups II, IV, and VI) or Freund's incomplete adjuvant (Groups I, III, and V). Test bleeds were obtained one week after the first and second boosts, and the mice were bled out one week after the third boost.

Western blot and ELISA analysis of the first test bleeds confirmed a strong immune response to DomB among the Group VI (DomB, no adjuvant) mice. After the second bleed, this response was found to be titratable to greater than 1:6,400 by ELISA. Group VI was the only group that developed high titer DomB antibodies. The mice immunized with DomB in Freund's adjuvant (Group V) mounted a weak immune response; based on reactivity to the immunogen in Western blot format and DomB-ELISA antibody titers in the 100-400 range, Group V mice appeared to be immunosuppressed. The control mice (Groups I-IV) were all negative for antibody to DomB in both the Western and ELISA screens. In spite of high DomB antibody titers in Group VI mice, plaque reduction neutralization tests (PRNT) revealed that none of the mice immunized with the crude DomB produced neutralizing antibody (Table 1).

**Table 1**

| Plaque Reduction Neutralization Tests of Sera from Mice Immunized with DomB in Concentrated, Total Secreted Yeast Proteins | | | | | | | |
|---|---|---|---|---|---|---|---|
| Group | Trial | Dilution Plaque Counts | | | | | |
| PBS+ Freund's Adjuvants | | 1:100 | 1:200 | 1:400 | 1:4 | 1:8 | 1:16 |
| | 1 | 109, 105 | ∼100 | ∼100 | | | |
| | 2 | | | | 34, 33 | 38, 30 | 44, 44 |
| PBS | | 1:10 | 1:20 | 1:40 | 1:4 | 1:8 | 1:16 |
| | 1 | 100, 106 | ∼100 | ∼100 | | | |
| | 2 | | | | 29, 33 | 33, 40 | 39, 37 |
| Negative secreted proteins + Freund's | | 1:10 | 1:20 | 1:40 | 1:4 | 1:8 | 1:16 |
| | 1 | 106, 95 | ∼ 100 | ∼ 100 | | | |
| | 2 | | | | 40, 35 | 37, 39 | 39, 47 |
| Negative secreted proteins | | 1:10 | 1:20 | 1:40 | 1:4 | 1:8 | 1:16 |
| | 1 | 109, 94 | ∼ 100 | ∼ 100 | | | |
| | 2 | | | | 38, 42 | 44, 40 | 45, 42 |
| Crude DomB+ Freund's | | 1:10 | 1:20 | 1:40 | 1:4 | 1:8 | 1:16 |
| | 1 | 100, 92 | ∼ 100 | ∼ 100 | | | |
| | 2 | | | | 37±5* | 35±6* | 39±3* |
| Crude DomB | | 1:10 | 1:20 | 1:40 | 1:4 | 1:8 | 1:16 |
| | 1 | 105, 91 | ∼ 100 | ∼ 100 | | | |
| | 2 | | | | 39±3* | 41±3* | 39±3* |
| DEN-2 HMAF | | 1:100 | 1:200 | 1:400 | 1:800 | 1:1600 | 1:3200 |
| | 1 | 0,0 | 0,0 | 0,0 | 11, 8 | 31, 19 | 81, 75 |
| | | | | | 1:250 | 1:1000 | 1:4000 |
| | 2 | | | | 3, 6 | 12, 15 | 21, 21 |
| PRNT assays performed on VERO cells (Trial 1) or BHK-21 C15 cells (Trial 2) with DEN-2 NGC strain. All plaque counts indicate the number of plaques obtained with the sera from five animals were pooled and assayed in duplicate, except those indicated (*) where each serum sample was individually assayed in duplicate and the number of plaques averaged (mean ± SD). | | | | | | | |

B. Pure DomB Immunogenicity in Mice: In contrast to mice immunized with crude DomB, outbred ICR mice (Charles River) immunized with purified DomB demonstrated high titer DEN-2 virus neutralizing antibodies. Purified DomB, at a concentration of 3.5 mg/ml, was used to immunize mice (three per group) with 175 µg of purified DomB mixed 1:1 with Freund's adjuvant, with alum, or without adjuvant (PBS). Test bleeds taken after three inoculations were assayed by PRNT (Table 2).

**Table 2**

| Plaque Reduction Neutralization Tests of Sera from Mice Immunized with Purified DomB | | | | | | | | |
|---|---|---|---|---|---|---|---|---|
| Group | Trial | Dilution | | | | | | 80% PRNT Titer⁻¹ |
| | | Plaque Counts | | | | | | |
| Negative Ascites Fluid | | 1:10 | 1:20 | 1:40 | | | | |
| | 1 | 36,40 | 35,34 | 40,38 | | | | |
| | 2 | 39,38 | 47,45 | 41,44 | | | | |
| DEN-2 HMAF | | 1:100 | 1:200 | 1:400 | 1:800 | 1:1600 | 1:3200 | |
| | 1 | 0,0 | 0,0 | 1,0 | 2,1 | 8,9 | 13,14 | 1500 |
| | 2 | 0,1 | 1,0 | 1,2 | 1,1 | 7,6 | 16,17 | >1600 |
| DomB no adjuvant | | 1:10 | 1:20 | 1:40 | 1:80 | 1:160 | 1:320 | |
| | 1 | 36,29 | 28,36 | 29,27 | 36,30 | 40,25 | 28,31 | >10 |
| | 2 | 9,17 | 20,15 | 22,26 | 33,26 | 41,36 | 42,47 | ~10 |
| DomB alum | | 1:10 | 1:20 | 1:40 | 1:80 | 1:160 | 1:30 | |
| | 1 | 8,6 | 10,9 | 10,12 | 21,20 | 32,26 | 30,28 | 10 |
| | 2 | 1,0 | 5,4 | 8,7 | 12,15 | 20,25 | 27,35 | 40 |
| DomB Freund's | | 1:40 | 1:80 | 1:160 | 1:320 | 1:640 | 1:1200 | |
| | 1 | 0,0 | 1,0 | 1,4 | 9,2 | ND | ND | >320 |
| | 2 | 0,0 | 3,4 | 5,5 | 5,20 | 13,9 | 23,20 | ∼640 |
| PRNT assays performed on VERO cells with DEN-2 NGC strain. ND = Not Determined. | | | | | | | | |

Mice immunized with purified DomB in the absence of adjuvant lacked neutralizing antibodies. DomB administered in combination with alum elicited low titer (80T PRNT ^{~}1:10) neutralizing antibodies, and mice receiving purified DomB in combination with Freund's adjuvants had a high PRNT titer (80T PRNT >1:320).

Three groups of 3 mice each, 5-6 week-old outbred ICR strain (Charles River) were used. Inoculation was intramuscularly in the rump at one site using 10 µg of antigen in 0.1 ml administered solution. Three inoculations were given to each group on days 1, 20, and 43. In one group, inoculation on day 1 incorporated complete Freund's adjuvants, on day 20 incomplete Freund's adjuvant, and that at day 43, no adjuvant. In a separate group, no adjuvant was supplied and in a third group, alum was supplied with all three inoculations. The sera were withdrawn on day 57 and the sera from each group were pooled, heat-inactivated at 56°C for 30 minutes and tested for their ability to reduce plaques formed from VERO cells.

C. DomB Protection from Virus Challenge: Suckling mice were immunized with purified DomB in Freund's, Alum, Hunter's TiterMax (Vaxcel), or no adjuvant for protection against an intracerebral injection of DEN-2 New Guinea C (NGC) strain. DomB administered in all adjuvants conferred comparable moderate survival against dengue virus challenges although survival was statistically significant (P< 0.5 G test) only for mice immunized with DomB and Hunter's TiterMax. The results are shown in Figure 10.

D. KLH-DomB Immunogenicity in Mice: Two series of mouse immunizations were initiated to determine the 50% effective immunizing dose (EID₅₀) of unconjugated and KLH-conjugated DomB. Effects of alum and Freund's adjuvants to a no-adjuvant control are compared.

DomB was conjugated to KLH via EDC (1-ethyl-3-(3-dimethylaminopropyl) carbodiimide HCl) using the carbodiimide coupling method at a 1.5:1 DomB-to-KLH mass ratio. The amount of unconjugated and conjugated DomB used for these immunizations was normalized, relative to the amount of unconjugated DomB. This normalization was based upon the specific immunoreactivity of each preparation as assayed by indirect ELISA.

Mice were immunized with the priming does of 174, 52, or 5.2 µg (total protein) of the KLH-conjugated DomB in Freund's, alum, or no adjuvant. Additional mice were immunized with 87, 26, or 2.6 µg (total protein) of unconjugated DomB in Freund's adjuvant to allow direct comparison of conjugated and unconjugated material. Boosts consisting of one-half the priming dose are being given at two-week intervals. Test bleeds are assayed for the induction of anti-DomB antibodies by ELISA and Western blot. Final bleeds are tested for induction of a neutralizing immune response by PRNT assay as well as for the production of binding antibodies by ELISA and Western blot. The results are summarized in Table 3.

The response to unconjugated DomB with Freund's adjuvants was low compared to the results in Table 2, which had shown that unconjugated DomB induced a strong virus neutralizing response in outbred ICR mice when administered with Freund's adjuvants. This apparent difference may be grounded in testing pooled sera for the data in Table 2. Pooling sera may mask individual variability. The variability in Table 3 may be attributed to the limited epitopes in DomB and to differences in the MHC genes for the outbred Swiss mice used in Table 3.

**Table 3**

| **Mouse Immune Response to Unconjugated and KLH-Conjugated Purified Domain B** | | | | | |
|---|---|---|---|---|---|
| **mouse** | **antigen** | **adjuvant** | **Final Titer Western**^{**c**} | **Final Titer ELISA**^{**c**} | **Final Titer PRNT**_{**80**}^{**c**} |
| 1 | saline | none | N/T^{a} | <1:100 | N/T |
| 2 | | | N/T | <1:100 | <1:10 |
| 3 | | | N/T | <1:100 | <1:10 |
| 4 | 87 µg B | none | N/T | <1:100 | <1:10 |
| 5 | | | N/T | <1:100 | N/T |
| 6 | | | N/T | <1:100 | <1:10 |
| 7 | 87 µg B | Freund's | >1:100,000 Dblt^{b} | >1:102,400 | 1:40 |
| 8 | | | N/T | <1:100 | <1:10 |
| 9 | | | 1:1,000,000 Dblt | >1:409,600 | <1:10 |
| 10 | | | N/T | <1:100 | <1:10 |
| 11 | | | 1:100,000 Dblt | 1:25,600 | <1:10 |
| 12 | 26 µg B | Freund's | N/T | <100 | <1:10 |
| 13 | | | 1:100,000 Dblt | 1:102,400 | <1:10 |
| 14 | | | 1:10,000 Dblt | 1:102,400 | N/T |
| 15 | | | 1:10,000 Dblt | >1:25,600 | <1:20^{c} |
| 16 | | | 1:10,000 Dblt | 1:409,600 | <1:10 |
| 17 | 2.6 µg B | Freund's | 1:1,000 Dblt | 1:25,600 | <1:10 |
| 18 | | | N/T | <1:100 | <1:46 |
| 19 | | | N/T | <1:100 | <1:48 |
| 20 | | | N/T | <1:100 | <1:24 |
| 21 | | | N/T | <1:100 | <1:10 |
| 22 | "87 µg" KLH-B | none | N/T | 1:100 | <1:10 |
| 23 | | | <1:100 | 1:400 | <1:24 |
| 24 | | | N/T | <1:100 | <1:24 |
| 25 | | | N/T | <1:100 | <1:24 |
| 26 | | | N/T | <1:100 | <1:20 |
| 27 | "26 µg" KLH-B | none | N/T | <1:100 | <1:34 |
| 28 | | | N/T | <1:100 | <1:70 |
| 29 | | | N/T | <1:100 | <1:34 |
| 30 | | | N/T | <1:100 | N/T |
| 31 | | | N/T | <1:100 | <1:10 |
| 32 | "2.6 Tg" KLH-B | none | N/T | <1:100 | N/T |
| 33 | | | N/T | <1:100 | <1:10 |
| 34 | | | N/T | <1:100 | <1:10 |
| 35 | | | N/T | <1:100 | <1:10 |
| 36 | | | N/T | <1:100 | <1:10 |
| 37 | "87 Tg" KLH-B | Freund's | N/T | >1:1,600 | 1:20 |
| 38 | | | N/T | >1:100 | <1:10 |
| 39 | | | N/T | <1:100 | <1:10 |
| 40 | | | 1:1,000 | >1:1,600 | 1:20 |
| 41 | | | N/T | <1:100 | <1:10 |
| 42 | "26 Tg" KLH-B | Freund's | 1:10,000 Dblt | >1:25,600 | 1:10 |
| 43 | | | 1:10,000 Dblt | >1:25,600 | 1:2560 |
| 44 | | | 1:10,000 Dblt | >1:25,600 | 1:5120 |
| 45 | | | N/T | <1:100 | <1:10 |
| 46 | | | N/T | 1:100 | <1:10 |
| 47 | "2.6 Tg" KLH-B | Freund's | N/T | <1:100 | <1:10 |
| 48 | | | N/T | <1:100 | <1:10 |
| 49 | | | <1:50 | 1:6,400 | <1:10 |
| 50 | | | N/T | <1:100 | <1:10 |
| 51 | | | <1:50 | 1:25,600 | <1:10 |
| 52 | "87 Tg" KLH-B | Alum | <1:100 | <1:400 | 1:60 |
| 53 | | | N/T | <1:100 | <1:10 |
| 54 | | | <1:100 | >1:1,600 | 1:320 |
| 55 | | | <1:100 | >1:1,600 | 1:80 |
| 56 | | | 1:1000 | >1:6,400 | 1:640 |
| 57 | "26 Tg" KLH-B | Alum | N/T | >1:100 | <1:10 |
| 58 | | | N/T | <1:100 | <1:10 |
| 59 | | | N/T | <1:100 | <1:10 |
| 60 | | | N/T | <1:100 | <1:10 |
| 61 | | | N/T | <1:100 | <1:10 |
| 62 | "26 Tg" KLH-B | Alum | <1:100 | 1:1,600 | 1:120 |
| 63 | | | 1:1000 | >1:1,600 | 1:120 |
| 64 | | | N/T | <1:100 | <1:10 |
| 65 | | | 1:100 | >1:1,600 | 1:80 |
| 66 | | | N/T | <1:100 | <1:10 |

| | | | | | |
|---|---|---|---|---|---|
| ^{a}N/T - not tested | | | | | |
| ^{b}Dblt - a doublet at approximately 12 kD | | | | | |
| ^{c}If serum was insufficient for testing at 1:10 dilution then higher initial dilutions were used and are indicated. | | | | | |

E. DomB Immunizations for Hybridoma Generation: Six BALB/c mice were immunized with 87 µg of unconjugated DomB or 174 µg KLH-conjugated DomB in Freund's adjuvant. Upon demonstration of a strong anti-DomB response, the mice are sacrificed and their spleen cells fused to hybridoma cells for monoclonal antibody production.

### Example 9

### Production of Domain B in Drosophila

The cloning vector p29GEB2.4PS, containing the domain B-encoding nucleotide sequence was digested with PvuII and the short fragment ligated into pMttSma, which is derived from pMttbns by digesting with BglII and inserting an adapter oligonucleotide containing an Sma site. The duplex linker adapter inserted has the sequence pGATCCCGG. pMttsma then contains a unique SmaI site at the 3' end of sequences encoding the tPA leader. The parent vector pMttbns SmithKline contains the *Drosophila* metalothionein gene (*mtn*), the human tissue plasminogen activator secretion signal (tPA_{L}) and the SV40 early polyadenylation signal. The resulting expression vector, pMttDomB is thus obtained as shown in Figure 11. The sequence of the fusion is shown in Figure 9.

The resulting pMttDomB vector was transfected into Schneider S2 cells which were maintained in Schneider medium (Life Technologies, Grand Island, NY) supplemented with 10% heat-inactivated FBS at 25°C. The cells were plated the day prior to transfection at 5 x 10⁵ cells in 60 mm tissue culture dishes in a total volume of 4 ml Schneider medium plus 10% FBS. The Schneider cells were then cotransfected with pMttDomB and pCOHygro (a hygromycin B phosphotransferase-based selection vector obtained from SmithKline Beecham) using standard calcium phosphate transfection. 48 hours after transfection, the medium was supplemented with hygromycin at 300 µg/ml; hygromycin-resistant cells grew in 2-3 weeks. Varying levels of the cotransformed plasmids were used in the transfection procedure ranging from expression plasmid: PCOHygro, 1:1 to 100:1.

Cells cotransfected at ratios of 1:1, 5:1 and 20:1 were induced with 200 µM copper sulfate and the media and cells were harvested at days 1, 4 and 7. Western blots showed secretion of domain B into the medium.

### Example 10

### Production of 60%E and 80%E in Drosophila

Using the adapted expression vector of Example 9, containing the *Drosophila* metalothionein gene, the human tissue plasminogen activator signal and the SV-40 early polyadenylation signal, the nucleotide sequences encoding 80%E, prM 80%E, 60%E and prM 60%E are inserted and the resulting vectors used to transform Schneider cells as described in Example 9. The mature truncated forms of the envelope protein are secreted into the medium, or properly processed, and are conformationally correct with respect to the corresponding native portions of the envelope protein.

Furthermore, crude media from these transformants employed in the protocol set forth in paragraphs A and B of Example 8 produce antibodies which are neutralizing against dengue virus.

### Example 11

### Expression of 80%E in Saccharomyces cerevisiae

An expression vector (pLS6-80%E) was constructed for secretion of the N-terminal 80% (codons 1-395) of the DEN-2 PR-159 S1 envelope glycoprotein (80%E) from *S. cerevisiae.* The 80%E DNA sequences were obtained from plasmid p29D280E, described in example 1, by restriction endonuclease digestion with both *Bgl*II and *Sal*I. The released fragment was isolated by agarose gel electrophoresis and subcloned between the *Bgl*II and *Sal*l sites of pLS6, a yeast expression vector described in example 2. The resulting recombinant plasmid, pLS6-80%E, contains truncated E as a translationally in-frame fusion to the leader region of mating-factor α (MFα), a secreted yeast protein. The MFα leader (MFα_{L}) contains a 19 amino acid secretion signal peptide followed by a 66 amino acid propeptide. Cleavage of the signal peptide is expected . to occur concomitantly with translocation across the endoplasmic reticulum membrane. Maturation of MFα normally involves removal of the propeptide by Kex2p, a golgi protease, and subsequent trimming of N-terminal (Glu/Asp)Ala dipeptides by dipeptidyl aminopeptidase (DPAP). The herein described MFα_{L}-80%E fusion was made such that processing of the MFα_{L} propeptide and trimming of a GluAla dipeptide results in 80%E with eight additional N-terminal amino acids derived from sequences present in the multiple cloning site of the pLS6 vector or in the PCR primer-adapter used to synthesize the 80%E cDNA (see below) 
ATG .....GCT.....GAG GCC TTT AGA TCT CGA GTA CCC GGG ACC ATG ...GAA TAA
TAG
Met-18aa Ala-65aa Glu Ala Phe Arg Ser Arg Val Pro Gly Thr Met1...Gly395End
End
▲ ▲ ▲
80%E
Signalase Kex2p DPAP
(SEQ ID NO:19, SEQ ID NO:20)
 Transcription of the gene fusion is driven by the *S. cerevisiae* copper-inducible copper metallothionein (*CUP1*) promoter.

After confirming the DNA sequence of the ligated junctions of expression vector pLS6-80%E, the recombinant DNA was transformed into *S. cerevisiae* strain GL43 (*MATa trp1Δ1 ura3-52 pep4::URA3;* SmithKline Beecham) according to standard protocols (Gutherie & Fink, eds. 1991; Rose et al, 1990). Transformants were selected by their ability to grow on minimal medium (SD medium: Guthrie and Fink, 1991) without tryptophan supplementation.

In order to test for expression and secretion of 80%E, several transformants were grown as small-scale cultures (5 ml medium in 17x150 mm tubes). Single colonies were used to inoculate SD medium and the inoculated cultures were grown to saturation (overnight, 30°C, 220 rpm). 1x10⁸ cells from the overnight culture were used to inoculate 5 ml of minimal SD medium supplemented with Casamino acids (2 g/l; Difco) and CuSO₄ (200 µM). This expression culture was fed with glucose (4 g/l, final concentration) and sodium phosphate (pH 6.7, 20 mM, final concentration) at 24 hours post inoculation and was harvested by centrifugation at 48 hours of growth (30°C, 220 rpm, in 17x150 mm tubes). Cell-free spent medium was buffer-exchanged with TEEN+PIC (50 mM Tris, 10 mM EDTA, 10 mM EGTA, 150 mM NaCl, pH 8.0 with 1 µg/ml each of pepstatin and leupeptin and 1 mM phenylmethylsulfonylfluoride) and concentrated 250-fold using Centricon-30 (Amicon) ultrafiltration. An extract of cellular protein was prepared by lysing the yeast cells with vigorous agitation in the presence of glass beads (425-600 µm) and TEEN+PIC using a Mini Beadbeater apparatus (BioSpec Products, Bartlesville, OK). Samples were endoglycosidase H_{f} digested according to the manufacturer's (New England Biolabs, Beverly, MA) protocol prior to SDS-PAGE analysis. Protein gels were Coomassie-stained directly as well as Western blotted and immunoprobed using mouse polyclonal serum raised against recombinant domain B (described in example 15). Negative control yeast carrying the expression vector without a Dengue gene insert secreted no proteins recognized by the anti-domain B serum, while the major immunoreactive band from pLS6-80%E medium had a relative mobility matching that of other recombinant 80%E proteins (see Example 17). The pLS6-80%E medium also contained a minor immunoreactive species with an apparent molecular weight 6-8 kD higher than 80%E; this is likely to be unprocessed MFα propeptide-80%E. The pLS6-80%E cellular protein extract contained many immunoreactive polypeptides not observed in negative control cells, two of which match the secreted products discussed above. A Coomassie-stained protein corresponding to recombinant 80%E could not be identified in either the pLS6-80%E secreted sample or the total cellular protein sample. This indicates the relatively low levels of recombinant protein expression in these preparations.

### Example 12

### Construction of expression vector pPIC9-80%E and secretion of 80%E by P. pastoris expressing MFα_{L}-80%E

The expression vector constructed to secrete 80%E from *P*. *pastoris* was engineered to express amino acids 1-395 of the DEN-2 PR-159 S1 envelope glycoprotein as a fusion to the MFα_{L}. The DNA sequences encoding 80%E were obtained from the clone p29D280E, described in example 1, by digestion with the restriction enzymes *Sma*I and *Sal*I. The isolated fragment was treated with the Klenow DNA polymerase I fragment enzyme to make the *Sal*I end blunt. This 80%E fragment was then cloned into the *Pichia* expression vector pPIC9 (Invitrogen, San Diego, CA) which contains the MFα secretion leader (MFα_{L}) sequence, *Sna*BI, *Eco*RI, and *Not*I cloning sites, and uses the *HIS4* gene as a selectable marker. The described 80%E fragment was ligated with pPIC9 plasmid DNA that was previously digested with the restriction enzyme *Sna*BI. The orientation and genetic'integrity of the resulting gene fusion expression vector, pPIC9-80%E, was confirmed by restriction digestion and DNA sequence analysis. The organization, partial nucleotide and predicted amino acid sequences of the MFα_{L}-80%E fusion gene are shown below: 
(SEQ ID NO:21 and SEQ ID NO:22)
ATG ......... GAG GCC TTTAGATCTCGAGTACCCGGGACC ATG ...GGA TAA
Met-18aa-65aa Glu Ala PheArgSerArgValProGlyThr Met1...Gly395 END
80%E
Signalase Kex2p DPAP
 The location of the signalase and *Kex2* cleavage sites which remove the pre and pro portions of the MFα leader peptide, respectively, are indicated. The dengue sequences are indicated in bold type. The Met₁ residue is the N-terminal amino acid of the E glycoprotein and Gly₃₉₅ is residue 395 from the amino terminal end of the envelope glycoprotein. The expression of a recombinant product in *Pichia* from the pPIC9 vector is driven by the methanol inducible promoter derived from the *Pichia AOX1* (alcohol oxidase 1) gene.

The pPIC9-80%E expression vector was transformed into spheroplasts of *P. pastoris* strain GS115 (*his4*) and transformants were selected for their ability to grow on minimal medium without histidine supplementation. Strain GS115 and the protocol used for transformation were obtained from Invitrogen (San Diego, CA). Transformants were tested for their ability to express and secrete 80%E by growing selected clones in small cultures (5 to 50 ml). The transformants were first grown to saturation (24 to 36 hrs.) in BMGY medium (1% yeast extract, 2% peptone, 100 mM potassium phosphate, pH 6.0, 1.34% yeast nitrogen base without amino acids, 4 x 10⁻⁵% biotin, 1% glycerol). The cells were collected by centrifugation and suspended in one half the original culture volume with BMMY (identical to BMGY except the glycerol component of BMGY is replaced in BMMY with 0.5% methanol) medium and cultured for 48 hrs. Proteins secreted into the culture medium as well as cellular proteins were treated with endoglycosidase H_{f}, (EndoH_{f}, New England Biolabs, Beverly, MA) and separated by SDS-PAGE. Protein gels were analyzed by both Coomassie staining and immunoprobing of Western blots. When nonEndoH treated samples are compared to EndoH treated samples on Coomassie stained gels of proteins prepared from the culture medium, a unique staining band of approximately 50 kD is present in the EndoH treated lanes of all the pPIC9-80%E transformants. Immunoprobing with anti-domain B serum (see example 6) detected a smear ranging from 50 to 90 kD in the nonEndoH treated samples and a unique band of approximately 50 kD in the EndoH treated samples on Western blots of proteins prepared from the culture medium. No bands were detected by the anti-domain B serum in negative control lanes. Variable amounts of the corresponding immunoreactive band were detected in EndoH treated samples of cellular protein samples. The approximately 50 kD 80%E product produced by the MFα_{L}-80%E construct is consistent with the approximate molecular weight as determined by SDS-PAGE of other recombinant 80%E proteins (see Example 17). The amount of secreted 80%E in the culture medium is about 1% of the total secreted protein as estimated by the intensity of the Coomassie staining band detected. In one liter cultures, the amount of 80%E secreted into the culture medium was determined to be 500 ng/ml by use of a sandwich ELISA method.

### Example 13

### Construction of Expression vector pMttbns-80%E and secretion of 80%E by Drosophila melanogaster Schneider cells expressing tPA_{L}-80%E

The expression vector constructed to secrete 80%E from *Drosophila melanogaster* tissue culture cells included the sequences encoding the DEN-2 PR159/S1 envelope glycoprotein amino acids 1-395. The DNA sequences for 80%E were obtained from the clone p29D280E (described in Example 1) by digestion with the restriction enzymes *Bgl*II and *Sal*I. The released 80%E fragment was cloned into the *Bgl*II plus *Xho*I-digested *D. melanogaster* expression vector pMttΔXho. The expression vector pMttΔXho is a derivative of the vector pMttbns (SmithKline Beecham) that contains a *D. melanogaster* metallothionein gene promoter (*Mtn*), the human tissue plasminogen activator leader sequence (tPA_{L}), and the SV40 early polyadenylation signal. A 15 bp *Bam*HI DNA fragment containing a *Xho*I site was deleted from pMttbns to make pMttΔXho, in which the *Bgl*II and another *Xho*I restriction endonuclease sites are unique. This construction resulted in the 80%E fragment being fused to the tPA_{L} sequence. During normal maturation of tissue plasminogen activator the 20 amino acid prepeptide region of the leader sequence is removed by signalase in the endoplasmic reticulum and the 11 amino acid propeptide region is enzymatically removed in the Golgi. The genetic integrity of the gene fusion expression vector, pMtt80%E, was confirmed by restriction digestion and DNA sequence analysis. The nucleotide and predicted amino acid sequences of the tPA_{L}-80E fusion gene are shown below: 
(SEQ ID NO:23 and SEQ ID NO:24)
ATG ............... GGA GCC AGA TCT CGA GTA CCC GGG ACC ATG ... GGA TAA
Met- 20 aa - 11 aa -Gly Ala Arg Ser Arg Val Pro Gly Thr Met1 ... Gly395 END
pre▲ pro-tPA▲ 80%E
 The tPA pre- and propeptide regions are delineated by pre^{▲} and pro-tPA^{▲}, respectively, and the dengue sequences are indicated in bold type. The Met₁ residue is the N-terminal amino acid of the envelope glycoprotein and Gly₃₉₅ is residue 395 from the amino terminal end of the envelope glycoprotein.

The selection plasmid, pCOHygro (SmithKline Beecham), carries the *E. coli* hygromycin B phosphotransferase gene under the transcriptional control of a *D. melanogaster copia* transposable element long terminal repeat and confers resistance to hygromycin B. Others have demonstrated that Schneider cells that have been cotransfected with both the pMtt expression vector and the pCOHygro plasmid and are then selected for hygromycin B resistance produce stable cell populations that contain multiple copies of the cotransfected gene of interest. *Drosophila melanogaster* Schneider cells (ATCC, Rockville, MD) were cotransfected with the pMtt80%E and pCOHygro plasmids at a ratio of 20:1 using the calcium phosphate coprecipitation method (Gibco BRL). Transformants were selected by outgrowth in Schneider medium (Gibco BRL) supplemented with 10% fetal bovine serum (Hyclone) and 300 µg/ml hygromycin (Boerhinger Mannheim). Transformants were split to a cell density of 2 X 10⁶ cells/ml in serum free Excell medium (JRH Biosciences) and induced with 200 µM CuSO₄. The medium and cells from induced cells were separately harvested after 6-7 days of induction. Proteins secreted into the culture medium were separated by SDS-PAGE, and proteins were analyzed by both Coomassie staining and immunoprobing of Western blots. The Coomassie blue-stained SDS-PAGE gels shows that the approximately 50 kD secreted 80%E product is one of the predominant proteins in the unconcentrated medium, comprising as much as 20% of the total protein. Immunoblots probed with anti-DEN2 hyperimmune mouse ascites fluid (HMAF; from Robert Putnak, Walter Reed Army Institute of Research) and polyclonal anti-domain B antisera (Example 6) revealed a single immunoreactive polypeptide of approximately 50 kD present in unconcentrated medium. In addition, immunoblots revealed that the 80%E produced by the tPA_{L}-80%E construct was slightly larger than that obtained upon expression of a tPA_{L}-prM80%E construct (described in detail in Example 17). This additional mass may owe to the nine adapter amino acids at the amino terminus of 80%E (GARSRVPGT-80%E) (SEQ ID NO:25) when expressed from pMtt80%E versus 80%E expressed from pMttprM80%E (Example 17). The tPA propeptide, if not proteolytically removed, may also contribute to the additional molecular weight of 80%E expressed from pMtt80%E.

### Example 14

### Subcloning of dengue prM100%E and prM80%E cDNAs and mutagenesis of E secretion signal-encoding sequence (mutSS)

A cDNA clone of DEN-2 PR159/S1 designed to encode the preMembrane, Membrane, and Envelope genes (prM100%E) was constructed by PCR amplification essentially as described in Example 1 for the subcloning of 80%E. This cDNA clone includes nucleotides 439 to 2421 of the DEN-2 genome. The dengue cDNA fragment was generated using synthetic oligonucleotide primers D2pM439p and D2E2421m (see example 1 for nomenclature) and plasmid pC8 (Hahn et al, 1988, *supra*) as template. In addition to DEN-2 specific sequences, the primers contained the identical adapter sequences described in Example 1, except that a methionine codon (ATG) was included immediately preceding the first codon of the preMembrane sequence (phenylalanine). The primers are:

D2prM439p (SEQ ID NO:26)
BglII
5'-attctagatctcgagtacccgggacc atg TTT CAT CTG ACC ACA CGC-3'
XbaI XhoI SmaI

D2E2421m (SEQ ID NO:27)
SalI
5'-tctctagagtcga cta tta GGC CTG CAC CAT AAC TCC
XbaI END END

The PCR-generated prM100%E cDNA fragment was digested with the restriction endonuclease *Xba*I and ligated into the *Xba*I site of pBluescript SK⁺ (Stratagene, San Diego, CA) to obtain the plasmid p29prME13. DNA sequence analysis of the PCR-generated cDNA clone identified three PCR-introduced nucleotide differences between pC8 and p29prME13. A T→C transition at nucleotide 1255 resulted in a silent mutation, while the A→G transition at nucleotide 1117 resulted in the conservative amino acid substitution of a valine for an isoleucine. The final transition observed at nucleotide 1750 replaced a methionine, adjacent to a cysteine involved in a disulfide bond in domain A, with a valine. The effect of this substitution on the ability of the disulfide to form or its stability is not known.

To generate a cDNA subclone representing prM80%E, a 794 bp *Bam*HI-*Sal*I fragment from p29prME13 representing the envelope carboxy terminal-encoding fragment was removed. This fragment was replaced with a 431 basepair *Bam*HI-*Sal*I fragment from p29D280E (described in Example 1) that encodes a 20% carboxy end truncation of the envelope glycoprotein. The *Bam*HI site is a naturally occurring site within the envelope cDNA, and the *Sal*I site is an engineered site that immediately follows the stop codons encoded by the PCR primers. The resulting truncated cDNA clone, p48BSprM80E, was confirmed by restriction digestion and DNA sequence analysis to encode amino acids 1 through 395 of the envelope glycoprotein following prM.

Expression of the prM80%E cDNA in *S. cerevisiae* (Example 15) demonstrated absence of proteolytic processing between the prM and 80%E proteins in this yeast. To improve processing of E from prM, oligonucleotide-directed mutagenesis was performed to alter the naturally occurring signalase cleavage site between the prM and E proteins. Based on the algorithm of Von Heijne (1986, Nucl. Acids Res. 14:4683-4690), the natural DEN-2 E secretion signal peptide receives a poor predictive score for its function as a secretion signal. The algorithm of von Heijne is based on N-terminal amino acid sequences found in secreted and nonsecreted proteins. Scores for functional secretion signal peptides range from 3.5 to 17, with a mean of ^{~}10. The score for the secretion signal peptide of E of DEN-2 PR159/S1 is 5.2, near the lower end of the range for signal peptides. In the mutagenesis, the sequence encoding the four amino acids immediately preceding and the amino acid immediately following the signalase cleavage site were altered to change these five amino acids. The modified signal sequence has a score of 12.4 based on von Heijne's algorithm. The original and altered nucleotide sequences of the mutagenized region and the encoded amino acid sequences relative to the signalase site are (SEQ ID NO:30 through SEQ ID NO:33):

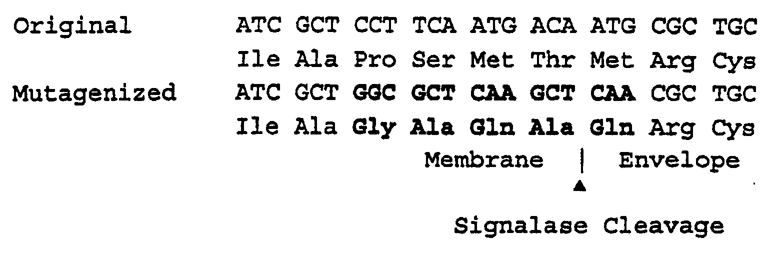

The mutagenized sequence and the resulting amino acid changes are indicated in bold.

To perform the mutagenesis, a 1,122 bp *Sma*I-*Hin*dIII fragment spanning the prM-E signalase cleavage site from the p29prME13 cDNA clone was subcloned into pAlter1 (Promega, Madison, WI) to yield the plasmid pAltSmaH3prME. The 1,122 bp *Sma*I-*Hin*dIII fragment contains all of prM and 611 bp of the E sequence. The *Hin*dIII site is a naturally occurring site within the E sequence that is located at nucleotide 1547 of the genomic sequence. The mutagenized clone, pAltSmaH3prME(mutSS), was verified by DNA sequence analysis.

### Example 15

### Construction of expression vectors pLS6-prM80%E and pLS6-prM(mutSS)80%E, expression of MFα_{L}-prM80%E and MFα_{L}-prM(mutSS)80%E in Saccharomyces cerevisiae, and secretion of 80%E by Saccharomyces cerevisiae expressing MFα_{L}-prM(mutSS) 80%E

For expression of DEN-2 PR159/S1 preMembrane protein amino acids 1-166 and Envelope glycoprotein amino acids 1-395 as a single continuous open reading frame in *S. cerevisiae,* DNA sequences encoding these proteins were obtained by digestion of plasmid p48BSprM80E (see Example 14) with restriction endonucleases *Bgl*II and *Sal*I. This fragment was cloned into the pLS6 yeast expression vector (see Example 2) that had been digested with *Bgl*II and *Sal*I. The structure of the resulting gene fusion expression vector, pLS6-prM80%E, was confirmed by restriction digestion and DNA sequence analysis. The nucleotide and predicted amino acid sequences of the MFα_{L}-prM80%E fusion gene are shown below (SEQ ID NO:28 and SEQ ID NO:29): 
BglII SmaII
ATG ......... GAG GCC TTTAGATCTCGAGTACCCGGGACCATG TTT ...ACA ATG ...GGA TAA
Met-18aa-65aa Glu Ala PheArgSerArgValProGlyThrMet Phe1...Thr166 Met1...Gly395End
▲ ▲ ▲
prM 80%E
Signalase Kex2p DPAP
 The location of the signalase, Kex2p, and DPAP cleavage sites involved in the processing of the MFα leader peptide are indicated (See Example 1 of this application for a detailed explanation). The dengue sequences are indicated in bold. The Phe₁ and Thr₁₆₆ residues are the N-terminal and C-terminal amino acid residues of prM, respectively. The Met₁ residue is the N-terminal amino acid of the envelope glycoprotein and Gly₃₉₅ is residue 395 from the amino terminal end of the envelope glycoprotein.

The pLS6prM80%E plasmid was transformed into *Saccharomyces cerevisiae* strain GL43 (MATa *ura3-52 trp1Δ1 pep4:URA3*) and screened for 80%E expression as described in Example 1. Proteins secreted into the culture medium as well as total cellular proteins were treated with Endoglycosidase H_{f} (EndoH_{f}, New England Biolabs, Beverly, MA) prior to analysis by SDS-PAGE followed by Coomassie Blue staining or Western blot immunoprobing. A novel secreted protein could not be identified either on Coomassie Blue stained SDS-PAGE gels or Western blots probed with anti-DEN2 hyperimmune mouse ascitic fluid (HMAF; from R. Putnak, Walter Reed Army Institute for Research) or anti-DomB antiserum (see Example 6). Western blot analysis of total cellular protein revealed a HMAF immunoreactive band of approximately 90 kD suggesting that the recombinant product had not been processed to prM and E. Probing of companion Western blots with polyclonal antisera that recognized the MFα leader peptide (from J. Rothblatt, Dartmouth University) confirmed that the product recognized by the anti-DEN2 HMAF was identical to that recognized by anti-MFα serum, demonstrating that the MFα_{L}-prM80%E fusion protein was not processed into its individual components (MFα_{L}, prM, and 80%E).

The unsuccessful processing of E from prM in the MFα_{L}-prM80%E fusion protein may be an obstacle to the proper folding and secretion of E. To assess whether the optimized dengue signal sequence (see Example 16) facilitated the processing of the envelope protein at the prM-E junction, the altered E signal sequence from pLS6prM(mutSS)100E-TGA was introduced into pLS6prM80%E to create plasmid pLS6prM(mutSS)prM80%E. This procedure replaced the native E signal sequence (Pro-Ser-Met-Thr₋₁-Met₊₁) (SEQ ID NO:34) with the optimized E signal sequence (Gly-Ala-Gln-Ala₋₁-Gln₊₁) (SEQ ID NO:35).

Plasmid pLS6prM(mutSS)100E-TGA was obtained by homologous replacement of a *Sma*I-*Sac*I fragment between plasmids pAlterSmaH3prME(mutSS) (see Example 14) and pLS6prM100E. DNA sequencing of pLS6prM(mutSS)100E-TGA identified an unintended TGA stop codon within E downstream of the mutated secretion signal. To transfer the altered secretion signal encoding sequence to pLS6prM80%E and to separate the cDNA fragment containing the altered secretion signal of E from the TGA stop codon, a *Bgl*II-*Eco*NI fragment from pLS6prM(mutSS)100E-TGA, encompassing prM and the first 430 nucleotides of E and lacking the TGA stop codon, was transferred to plasmid pLS6prM80%E which had been similarly digested to yield the expression plasmid pLS6prM(mutSS)80%E. The sequence of the expression plasmid was confirmed by restriction digestion and DNA sequence analysis. The nucleotide and predicted amino acid sequences of the MPα_{L}-prM junction are identical to the sequences listed above.

As described for the nonmutagenized plasmid, the DNA was transformed into the *S. cerevisiae* GL43 strain and transformants were selected based upon their ability to grow on unsupplemented minimal medium (see Example 11). Transformants were cultured, induced, and evaluated as described above for the nonmutated MFα_{L}-prM80%E transformants. Proteins secreted into the culture medium as well as total cellular proteins were treated with EndoH_{f} prior to separation by SDS-PAGE. Protein gels were analyzed by Coomassie staining and immunoprobing of Western blots. SDS-PAGE analysis of concentrated culture medium failed to identify a novel Coomassie staining band. Immunoprobing with anti-DEN2 HMAF and anti-DomB antiserum, however, revealed a small amount of processed immunoreactive E protein in the medium. The size of the immunoreactive protein (approximately 50 kD) was similar to the secreted protein from pLS6-80%E expression vector. Evaluation of intracellular expression of the fusion protein containing the optimized secretion signal by SDS-PAGE and Western blot demonstrated that the transformed cells produce immunoreactive product recognized by anti-DEN2 HMAF and anti-DomB antiserum. Unlike the immunoreactive product seen in pLS6prM80%E transformants, the immunoreactive band found in pLS6(mutSS)prM80%E transformants was not recognized by MFα_{L} anti-serum suggesting that processing had occurred at the prM-E junction. Thus, the mutagenesis of the signalase cleavage site resulted in greatly enhanced processing of the MFα_{L}-prM80%E product at the prM-E junction.

### Example 16

### Construction of expression vector pPIC9-prM(mutss)80%E and secretion of 80%E by P. pastoris expressing MFα_{L}-prM(mutss) 80%E

The expression vector constructed to express preMembrane-mutated secretion signal-80%Envelope (pPICprM(mutSS)80%E) in *P. pastoris* from a single continuous open reading frame utilized the DEN-2 PR159/S1 prM and E gene sequences described above (Example 14). The plasmid, pPIC9-prM(mutSS)80%E, was constructed by transferring a prM(mutSS)80%E fragment from the *S*. *cerevisiae* expression plasmid pLS6prM(mutSS)80%E into pPIC9. The *P. pastoris* expression vector pPIC9 (Example 4) and the *S. cerevisiae* expression vector pLS6 (Example 2) both use the MFα prepropeptide leader (MFα_{L}) sequence to direct the secretion of expressed proteins, and the two MFα_{L} sequences share an *Xho*I restriction endonuclease site, encoding amino acids leucine and glutamic acid, just upstream of the Kex2 protease site. The transfer of the dengue cDNA fragment made use of this *Xho*I site.

Prior to transferring the prM(mutSS)80%E cDNA fragment, sequences encoding extraneous amino acids and an extraneous *Xho*I site at the MFα_{L}-prM fusion were first removed. This was accomplished by digesting pLS6-prM(mutSS)80%E with restriction endonuclease *Xho*I and *Xma*I into which was ligated a synthetic oligonucleotide duplex {5'-TCGAGAAGAGAGAAG-3' (SEQ ID NO:36) and 5'-CCGGCTTCTCTCTTC-3' (SEQ ID NO:37) }. This manipulation deleted the extraneous *Xho*I site, the *Xma*I site, and six extraneous codons and preserved the MFα_{L} proteolytic processing sites and the *Xho*I site within MFα_{L} required for the cDNA fragment transfer from pLS6 to pPIC9. The nucleotide and predicted amino acid sequence at the MFα_{L}-prM fusion junction from pLS6-prM(mutSS)80%E and pLS6Δ-prM(mutSS)80%E are:

MFαL-prM junction of pLS6-prM(mutSS)80%E
(SEQ ID NO:38 and SEQ ID NO:39)
XhoI XhoI XmaI
ATG ...CTC GAG AAA AGG GAG GCC TTTAGATCTCGAGTACCCGGGACCATG TTT ..
Met ...Leu Glu Lys Arg Glu Ala PheArgSerArgValProGlyThrMet Phe1..
) ▲
Kex2p DPAP

MFαL-prM junction of pLS6Δ-prM(mutSS)80%E
(SEQ ID NO:40 and SEQ ID NO:41)
XhoI
ATG ...CTC GAG AAA AGG GAG GCC GGGACCATG TTT ..
Met ...Leu Glu Lys Arg Glu Ala GlyThrMet Phe1..
)
Kex2p DPAP

To construct the clone pPIC9-prM(mutSS)80%E, a *Xho*I*-Sal*I fragment encoding prM(mutSS)80%E sequences was obtained from pLS6Δ-prM(mutSS)80%E and was inserted into the pPIC9 vector that had been digested with *Xho*I. The genetic integrity of the expression plasmid, pPIC9-prM(mutSS)80%E, was confirmed by restriction digestion.

The pPIC9-prM(mutSS)80%E expression vector was transformed into spheroplasts of *P*. *pastoris* strain GS115 (*his4*), and His⁺ transformants were selected for their ability to grow on minimal medium without histidine supplementation. The transformants were screened for expression and secretion of 80%E as described in Example 12. No unique Coomassie staining bands were detected in the culture medium of either nonEndoH_{f} or EndoH_{f} treated samples (similar to that observed for culture medium from pPIC9-80%E transformants - see Example 12). Western immunoblots of proteins from the culture medium probed with anti-domain B serum detected multiple bands with a diffuse area of reactivity ranging from 50 to 90 kD in the nonEndoH treated samples and a unique band of approximately 50 kD in the EndoH treated samples. No immunoreactive polypeptides were detected by the anti-domain B serum in negative control samples. Various amounts of the corresponding immunoreactive protein were detected in EndoH treated samples of cellular proteins. We estimate that the amount of secreted 80%E in the culture medium is less than 1% of the total amount of secreted protein based on the observation that no polypeptide of the appropriate molecular weight was detected by Coomassie staining of EndoH_{f} treated samples.

### Example 17

### Construction of pMttbns-prM80%E and Secretion of 80%E by Drosophila melanogaster Schneider cells Expressing tPA_{L}-prM80%E

For expression of DEN-2 PR159/S1 preMembrane protein amino acids 1-166 and Envelope glycoprotein amino acids 1-395 as a single continuous open reading frame in *Drosophila melanogaster* Schneider 2 tissue culture cells, DNA sequences encoding these proteins were obtained by digestion of the p48BSprM80%E clone (described in Example 14) with the restriction enzymes *Bgl*II and *Sal*I. This fragment was cloned into the unique *Bgl*II and *Xho*I restriction sites of plasmid pMttΔXho (described in Example 13). The cloning junctions of the resulting gene fusion expression vector, pMttprM80E, were confirmed by restriction endonuclease digestion and DNA sequence analysis. The partial nucleotide and predicted amino acid sequences of the tPA_{L}-prM80E fusion gene are (SEQ ID NO:42 and SEQ ID NO:43): 
ATG ............. GGAGCCAGATCTCGAGTACCCGGGACCATG TTT ..ACA ATG ..GGA TAA
Met-20 aa- -11 aa-GlyAlaArgSerArgValProGlyThrMet Phe1..Thr166 Met1..Gly395 END
pre▲pro-tPA▲ prM 80%E
 The tPA pre- and propeptide regions are delineated by pre^{▲} and pro-tPA^{▲}, respectively, and the dengue sequences are indicated in bold type. The Phe₁ and Thr₁₆₆ residues are the N-terminal and C-terminal amino acid residues of prM, respectively. The Met₁ residue is the N-terminal amino acid of envelope glycoprotein and Gly₃₉₅ is residue 395 from the amino terminal end of the envelope glycoprotein.

As described previously in Example 13, Schneider 2 cells were cotransfected with pMttprM80%E DNA at ratios of 1:1, 5:1, and 20:1 relative to pCOHygro DNA. Transformants were induced with 200µM CuSO₄ and expression of prM80%E was examined at various times after induction. Proteins secreted into the culture medium as well as cellular proteins were separated by SDS-PAGE. Protein gels were analyzed by both Coomassie Blue staining and immunoprobing of corresponding Western blots. Analysis of Coomassie Blue-stained SDS-PAGE gels identified a novel band of approximately 50 kilodalton in all transfectants. This novel band was also recognized by anti-DEN-2 HMAF in Western blot analysis. This ^{~}50 kD immunoreactive band is roughly the same size as the secreted EndoH-treated product from pLS6-80%E transformed yeast cells (Example 11) and slightly smaller than the secreted 80%E from pMttbns80%E-transformed *D. melanogaster* Schneider cells (Example 13), suggesting the Envelope protein had been processed away from the preMembrane protein. (The size discrepancy between 80%E secreted by pMtt80%E and pMttprM80%E Schneider cells is discussed in Example 13.) Polyclonal antisera to the pr portion of prM (from Peter Wright, Monash University, Australia) did not recognize the ^{~}50 kD protein, confirming that the 80%E produced in the transfected cells was processed from prM. In fact, no evidence of a higher molecular weight band that might correspond to unprocessed prM80%E was detected in any sample, suggesting that the proteolytic processing of prM from E is extremely efficient in Schneider cells. The fate of the prM portion of the fusion remains unresolved as no distinct immunoreactive band was detected by probing with the anti-pr antisera.

The secreted 80%E glycoprotein was partially purified (judged by the presence of a single major band on a sliver stained SDS-PAGE gel) and its N-terminal amino acid sequence was determined. To purify the secreted glycoprotein, culture medium was concentrated and buffer exchanged against 20 mM succinate pH 5.7. The buffer exchanged material was loaded onto a CM-BioGel column and eluted in 150 mM NaCl. The 150 mM NaCl eluant was separated on an SDS-PAGE gel and electro-transferred to Immobilon-P membrane (Millipore). The 80%E band was excised, and the N-terminal amino acids were determined by Edman sequencing. Two amino acid sequences were obtained. One, Me.t-Xxx-Xxx-Ile-Gly-Ile (SEQ ID NO:44), had an individual residue yield of 7.9 -10.0 picomoles, while the other, Val-Xxx-Val-Gly-Ala-Val (SEQ ID NO:45), had a 3.2- 4.2 picomole yield. Incomplete reduction of the Cys at position three may account for lack of its detection. the first sequence is consistent with the expected sequence, Met-Arg-Cys-Ile-Gly-Ile (SEQ ID NO:46), supporting the interpretation that the ^{~}50 kD secreted immunoreactive glycoprotein is correctly processed 80%E of DEN-2.

Sensitivity of the secreted 80%E to endoglycosidases was evaluated by molecular weight shift of the protein in SDS-PAGE and Western immunoblots following endoglycosidase treatment. Resistance of the secreted 80%E to Endoglycosidase H_{f} (Endo H_{f}; New England Biolabs) and sensitivity to N-glycosidase F (PNGase F; New England Biolabs) digestion indicated that the secreted product contains N-linked glycosylation, and that the glycosylation is probably complex and is neither high mannose nor hybrid in composition.

The secreted protein is one of the predominant proteins in the unconcentrated medium, comprising as much as 20% of the total secreted protein. Estimates of the concentration of the 80%E product in unconcentrated medium based upon sandwich ELISA assays (described in detail in Example 7) and Coomassie blue staining range from 3-16 µg/ml depending on the preparation. Immunoblots probed with polyclonal anti-dengue 2 hyperimmune mouse ascites fluid (DEN-2 HMAF; from R. Putnak, WRAIR) demonstrated that the amount of secreted 80%E produced by the transfectants increased over time from day 1 post induction to 7 days post induction. The amount of 80%E detected intracellularly in the transfectants correlated with the cotransfection ratio, but the increase in intracellular 80%E with time was not as dramatic as for secreted 80%E, suggesting efficient secretion of 80%E and accumulation in the medium.

### Example 18

### Induction of anti-Dengue 2 antibodies in mice by Pichia pastoris-secreted 80%E

*P. pastoris* cells transformed with pPIC-80%E (described in Example 12) were induced with 0.5% methanol and the medium was collected after 40 hours of induction (for additional details on culture conditions see Example 12). The medium was filtered through a 0.5 µm low protein binding filter (Opticap, Millipore), then buffer exchanged with phosphate buffered saline (10 mM sodium phosphate, 2 mM potassium phosphate, 0.15 M sodium chloride, and 27 mM potassium chloride, pH 7.5) and concentrated approximately 40 fold using a combination of tangential flow (Minitan; Millipore) and centrifugal ultrafiltration (Centriprep 30; Amicon). The concentrated medium was analyzed by Western blot and assayed by sandwich ELISA (described in detail in Example 7) prior to injection into mice. Five week-old Swiss Webster outbred mice (Simonsen) were immunized by intraperitoneal (I.P.) injection with 100 µg total protein of the crude concentrated 80%E medium with or without complete Freund's adjuvant. Controls for this experiment included a negative control medium prepared from a nonrecombinant *P. pastoris* culture as described above for the 80%E medium. Protein precipitation was observed during the concentration of the negative control medium, consequently the final protein concentration of the concentrated medium was lower than that from the 80%E medium. (For this reason, 12.5 µg of total protein in Freund's complete adjuvant was used for immunization with the negative control medium.) Additional controls included saline and KLH-domain B, a recombinant dengue product previously shown to induce neutralizing antibodies (Example 8), both were administered with Freund's complete adjuvant. The mice received three I.P. boosts consisting of one half the priming dose with or without Freund's incomplete adjuvant, and consistent with the specific priming immunization. The boosts were administered at two week intervals.

Following the second boost, the animals were test bled (tail vein) and the immune response was monitored using an indirect ELISA. In the ELISA assay, plates were coated with a bovine serum albumin (BSA)-domain B conjugate, blocked with BSA, and serial dilutions of the mouse sera were then incubated with the coating antigen. Alkaline phosphatase-labeled goat anti-mouse IgG was used as the secondary detecting antibody, and the color development upon addition of an alkaline phosphatase chromogenic substrate was monitored. The ELISA titer is that which corresponded to the highest dilution of serum resulting in an optical density twofold above background (reactivity of the serum against BSA only). Following the third boost, the animals were bled-out and the serum was tested for anti-DEN2 responsiveness by indirect ELISA and the plaque reduction neutralization assay (PRNT). In the PRNT assay, the mouse sera were serially diluted in Eagles minimal essential medium (EMEM; BioWhittaker) supplemented with 10% fetal bovine serum (FBS; Hyclone) and mixed with 100 plaque forming units (pfu) of DEN2 virus (New Guinea C strain, from Robert Putnak, WRAIR). After allowing one hour for binding and neutralization of the virus, the serum-virus mixtures were plated onto susceptible monkey kidney monolayers (Vero cells, from Robert Putnak, WRAIR) plated in EMEM (BioWhittaker) supplemented with 10% FBS (Hyclone) in 6 well tissue culture dishes (Corning). The cells were overlaid with 0.9% agarose (Seakem; FMC) in EMEM supplemented with 5% FBS and viral cytopathic effect was allowed to develop for 6-7 days. The resulting viral plaques were stained with 0.012% neutral red (Sigma) in 1% agarose (Seakem; FMC). The number of plaques in each cluster were counted and compared to a no-serum viral control. The PRNT₈₀ was the highest dilution of serum that resulted in an 80% reduction in the number of plaques from a given viral inoculum. Results from the ELISA and PRNT assays are summarized in Table 4. The *P. pastoris* expressed 80%E induces a potent anti-DEN2 response in mice, with ELISA titers of up to 1:102,400. The titers obtained in the presence of adjuvant exceeded those obtained without adjuvant. The lack of a strong virus neutralizing response (PRNT₈₀ titers, Table 4) may simply reflect the crude nature of the immunogen, as similar results were obtained when crude domain B was used as an immunogen (see Example 8).

**Table 4**

| **Induction of Anti-DEN2 Immune Response in Mice Immunized with *P. pastoris-* expressed 80%E** | | | | | |
|---|---|---|---|---|---|
| **mouse** | **antigen** | **adjuvant** | **30 titer ELISA** | **40 titer ELISA** | **40 titer PRNT**_{**80**} |
| 30-1 | Saline | Freund's | <1:50 | <1:100 | |
| 30-2 | | | <1:50 | <1:100 | |
| 30-3 | | | <1:50 | <1:100 | |
| 30-4 | | | <1:50 | <1:100 | |
| 30-5 | | | <1:50 | <1:100 | |
| 32-1 | KLH-DomB "26"Tg | Freund's | >1:6400 | 1:25600 | |
| 32-2 | | | >1:1600 | 1:102,400 | |
| 32-3 | | | <1:100 | 1:100 | |
| 32-4 | | | >1:6400 | 1:102,400 | |
| 32-5 | | | <1:100 | <1:100 | |
| 34-1 | 12.5 Tg Pichia negative control medium | Freund's | <1:100 | <1:100 | |
| 34-2 | | | <1:100 | 1:100 | |
| 34-3 | | | <1:100 | < 1:100 | |
| 34-4 | | | <1:100 | <1:100 | |
| 34-5 | | | <1:100 | 1:100 | |
| 36-1 | 100 Tg Pichia 80%E total medium | Freund's | >1:6400 | 1:25,600 | <1:10 |
| 36-2 | | | 1:6400 | 1:25,600 | <1:10 |
| 36-3 | | | >1:6400 | 1:25,600 | <1:10 |
| 36-4 | | | 1:100 | 1:100 | <1:10 |
| 36-5 | | | 1:6400 | 1:102,400 | <1:10 |
| 37-1 | 100 Tg Pichia 80%E total medium | None | < 1:100 | 1:100 | |
| 37-2 | | | 1:1600 | 1:6400 | |
| 37-3 | | | 1:100 | 1:400 | |
| 37-4 | | | 1:400 | 1:6400 | |
| 37-5 | | | 1:100 | <1:100 | |

### Example 19

### Induction of dengue virus-neutralizing antibodies by immunizing mice with 80%E secreted by Drosophila melanogaster Schneider cells expressing tPA_{L}-prM80%E and tPA_{L}-80%E

Schneider cells, transformed with pMtt-prM80%E and pMtt-80%E expressing the tissue plasminogen activator leader fusion proteins tPA_{L}-prM80%E and tPA_{L}-80%E, respectively (Described in detail in Examples 17 and 13, respectively), were cultured in serum-free medium (Excell; JRH Biosciences) and induced by addition of CuSO₄ to a final concentration in the culture medium of 0.2 mM (see examples 13 and 17 for more detail on culture conditions). The cells were maintained in inducing medium for seven days prior to harvesting. The cells were removed by centrifugation at 1000 X G in a Beckman TJ-6 refrigerated centrifuge and the media were filtered through a 0.2 µm cellulose acetate filter (Nalgene). The media containing the recombinant 80%E were concentrated 20-fold using centrifugal concentrators (Centriprep 30; Amicon) and assayed by ELISA (described in detail in Example 7) and Western immunoblots prior to their use as an immunogen in mice. Negative control medium, derived from Schneider cells transformed with pCOHygro only (See Example 13), was produced as described above. In two series of immunizations, outbred Swiss Webster mice (Simonsen) were immunized intraperitoneally (I.P.) with 100 µg total protein in Freund's complete adjuvant. Control animals were immunized with 100 µg of total protein from the concentrated negative control medium, 80 µg of purified *Saccharomyces*-expressed domain B (see Example 8), or saline only, each in Freund's complete adjuvant. In the first series of immunizations, the mice received three I.P. boosts, consisting of one half the priming dose in Freund's incomplete adjuvant, at two week intervals. In the second series of I.P. immunizations, the mice received two boosts, each at one month intervals.

Following the first and second boosts, the animals were test bled (tail bleed) and the immune response was monitored using an indirect ELISA as described in example 18. Following the final boost, the animals were bled out and the sera were tested for anti-DEN2 responsiveness by indirect ELISA and PRNT as described in Example 18. Results for the ELISA and PRNT assays are summarized in Tables 5 and 6. In both series of immunizations, the mice immunized with the crude media containing 80%E, expressed cotranslationally with prM or independently without prM, developed high titer, virus-neutralizing antibodies. These titers are higher than any previously reported titers for any immunogen produced from any flavivirus, suggesting the utility of these immunogens as efficacious vaccine candidates.

**Table 5**

| **Immune Response of Mice Immunized with Crude *Drosophila* Media Containing Dengue 2 Virus 80%E Expressed as a prM80%E Fusion** | | | | | | |
|---|---|---|---|---|---|---|
| **mouse** | **antigen** | **adjuvant** | **20 titer**^{**a**} **ELISA** | **30 titer**^{**b**} **ELISA** | **Final titer**^{**c**} **ELISA** | **PRNT**_{**80**} **titer**^{**c**} |
| 20-1 | Saline | Freund's | <1:50 | <1:50 | <1:100 | <1:10 |
| 20-2 | | | <1:50 | <1:50 | <1:100 | <1:10 |
| 20-3 | | | <1:50 | <1:50 | <1:100 | <1:10 |
| 20-4 | | | <1:50 | <1:50 | <1:100 | <1:10 |
| 20-5 | | | <1:50 | <1:50 | <1:100 | <1:10 |
| 21-1 | pCoHygro | Freund's | <1:50 | <1:50 | <1:100 | <1:10 |
| 21-2 | | | <1:50 | <1:50 | <1:100 | <1:10 |
| 21-3 | | | <1:50 | <1:50 | <1:100 | <1:10 |
| 21-4 | | | <1:50 | <1:50 | <1:100 | <1:10 |
| 21-5 | | | <1:50 | <1:50 | <1:100 | <1:10 |
| 22-1 | prM80%E | Freund's | 1:3200 | >1:25,600 | 1:102,400 | 1:2560 |
| 22-2 | | | > 1:800 | >1:6,400 | 1:25,600 | 1:2560 |
| 22-3 | | | >1:200 | >1:1,600 | 1:25,600 | 1:2560 |
| 22-4 | | | <1:50 | >1:102,400 | >1:102,400 | 1:2560 |
| 22-5 | | | 1:3200 | >1:25,600 | >1:25,600 | 1:640 |

| | | | | | | |
|---|---|---|---|---|---|---|
| ^{a}Determined following the 2nd injection. | | | | | | |
| ^{b}Determined following the 3rd injection. | | | | | | |
| ^{c}Determined following the 4th and final injection. | | | | | | |

**Table 6**

| **Immune Response of Mice Immunized with Crude *Drosophila* Media Containing Dengue 2 Virus 80%E Expressed with or without prM** | | | | | |
|---|---|---|---|---|---|
| **mouse** | **antigen** | **adjuvant** | **20 titer**^{**a**} **ELISA** | **3/ titer**^{**b**} **ELISA** | **Final titer**^{**c**} **PRNT**_{**80**} |
| 25-1 | Saline | Freund's | < 1:50 | >1:50 | < 1:10 |
| 25-2 | | | <1:50 | >1:50 | < 1:10 |
| 25-3 | | | <1:50 | >1:50 | <1:10 |
| 25-4 | | | <1:50 | >1:50 | <1:10 |
| 25-5 | | | <1:50 | > 1:50 | < 1:10 |
| 27-1^{d} | 80Tg Purified Sacchro DomB | Freund's | 1:1600 | 1:102,400 | <1:100 |
| 27-2 | | | 1:100 | 1:6400 | 1:10 |
| 27-3 | | | >1:6400 | 1:102,400 | < 1:100 |
| 27-4 | | | >1:6400 | 1:409,600 | 1:40 |
| 27-5 | | | >1:6400 | 1:409,600 | <1:500 |
| 28-1 | 100Tg Drosophila prM80%E total medium | Freund's | DEAD | NT | NT |
| 28-2 | | | >1:6400 | 1:102,400 | 1:8000 |
| 28-3 | | | >1:6400 | 1:409,600 | 1:8000 |
| 28-4 | | | >1:6400 | 1:102,400 | 1:4000 |
| 28-5 | | | >1:6400 | 1:102,400 | 1:1000 |
| 29-1 | 100Tg Drosophila 80%E total medium | Freund's | 1:6400 | 1:6400 | 1:500 |
| 29-2 | | | 1:6400 | 1:102,400 | 1:4000 |
| 29-3 | | | <1:100 | 1:25,600 | 1:1000 |
| 29-4 | | | >1:6400 | 1:25,600 | 1:8000 |
| 29-5 | | | >1:6400 | 1:102,400 | 1:4000 |

| | | | | | |
|---|---|---|---|---|---|
| ^{a}Determined following the 2nd injection. | | | | | |
| ^{b}Determined following the 3rd injection. | | | | | |
| ^{c}Determined following the 4th and final injection. | | | | | |
| ^{d}mouse 27-1 did not receive second boost. NT - not tested. | | | | | |

### Example 20

### Protection from dengue virus challenge by immunizing mice with 80%E secreted by Drosophila melanogaster Schneider cells expressing tPA_{L}-prM80%E or tPA_{L}-80%E.

Schneider cells, transformed with pMtt-prM80%E and pMtt-80%E expressing the tissue plasminogen activator leader fusion proteins tPA_{L}-prM80%E and tPA_{L}-80%E respectively (described in detail in Examples 17 and 13, respectively), were cultured in serum-free medium (Excell; JRH Biosciences) and induced by addition of CuSO₄ to the culture medium at final concentration of 0.2 mM (see examples 13 and 17 for additional details on culture conditions). The cells were maintained in inducing medium for seven days prior to collecting the medium. The cells were removed by centrifugation at 1000 X G in a Beckman TJ-6 refrigerated centrifuge and the media were filtered through a 0.2 µm cellulose acetate filter (Nalgene). The media were concentrated 20-fold using centrifugal ultrafiltration (Centriprep 30, Amicon) and assayed by ELISA and Western immunoblots prior to use as an immunogen in mice. Negative control medium, derived from Schneider cells transformed with pCOHygro only (see Example 13), was produced as described above. These immunogens were used to immunize 10-13 day old Balb/c mice (Jackson Labs). A total of 0.1 ml of each preparation (corresponding to 70 µg total protein from negative control medium, 230 µg total protein from tPA_{L}-80%E medium, and 150 µg total protein from tPA_{L}-prM80%E medium) were used to subcutaneously inoculate groups of 10 mice each, using Alum as adjuvant. An identical second dose was administered 14 days later as a booster. One week after the second dose the mice were challenged with an intracranial injection of 100 LD₅₀ units of DEN2 New Guinea C strain (10⁵ pfu/injection).

The morbidity and mortality of the mice were monitored for 17 days post challenge. Six days after challenge the mice immunized with the negative control medium began exhibiting symptoms of infection including ruffled fur, hindlimb paralysis, wasting, and death. Eight of the 10 negative control mice were dead by day 12 post-challenge and the remaining two mice recovered by day 15 post-challenge. In contrast, of the 10 mice immunized with the tPA_{L}-80%E, only four exhibited any symptoms of infection, and eight of 10 survived the challenge. Similarly, nine of 10 mice immunized with tPA_{L}-prM80%E survived the challenge, although seven of these mice exhibited mild symptoms of infection during the monitoring period. These survival data are illustrated in Figure 13, and show that both *Drosophila*-expressed 80%E antigens efficiently protected mice from viral challenge. These results emphasize the utility of the *Drosophila* cell expressed 80%E dengue immunogens as vaccine candidates.

### Example 21

### Construction of DEN-2 N-terminal 60%E and prM60%E cDNA Fragments

A subclone encoding the N-terminal 60% of E was constructed using p29GEB24PS and p29D280E. Example 1 of the parent application --- describes construction of p29GEB24PS. Plasmid p29GEB24PS holds a *Bam*HI fragment insert containing, in part, DEN-2 E sequences (nucleotides 1696-2121) starting at a DEN-2 genomic *Bam*HI site and ending with the Gly395 codon, followed immediately by two stop codons and a *Sal*I site. Oligonucleotide mutagenesis had deleted codons for Leu292 through Lys295 (nucleotides 1810-1821) and had inserted two stop codons and a *Sal*I site immediately following the Lys291 codon (see Figure 4 and Example 1).

The N-terminal 80%E insert in p29D280E was then converted to a 60%E insert by replacing a restriction fragment encoding the 3' end of 80%E with a restriction fragment from p29GEB24PS encoding the 3' end of 60%E. To accomplish this, DNA of p29GEB24PS was digested with *Bam*HI, the ^{~}590 bp *Bam*HI fragment was isolated by agarose gel electrophoresis and then digested with *Sal*I, and finally the 119 bp *Bam*HI*-Sal*I fragment released from the ^{~}590 bp *Bam*HI fragment and containing dengue nucleotides 1696-1809 was isolated by agarose gel electrophoresis and ligated into p29D280E prepared as follows. Plasmid p29D280E was digested with *Bam*HI, which cuts the *Bam*HI site (dengue nucleotides 1696-1701) within 80%E, and with *Sal*l, which cuts immediately 3' of 80%E and also within the vector, pBR322, 422 base pairs distal to the 3' end of the 80%E fragment. Following ligation, the desired product, a plasmid containing the cDNA encoding the N-terminal 60% of E in pBR322 (p29D260E), was recovered by transformation of *E. coli* with the ligation mixture and screening transformant colonies for plasmids of the appropriate size and restriction digestion pattern. Proper ligation of the *Bam*HI-*Sal*I fragment in p29D260E was confirmed by DNA sequence determination.

To construct a cDNA encoding prM and the amino terminal 60% of E (prM60%E), we used a strategy identical to that used to construct prM80%E (Example 14). The prM100%E plasmid, p29prME13, was digested with *Bam*HI and Sail to release the 794 bp 3' end fragment of E, which was then replaced with the 119 bp *Bam*HI-*Sal*I fragment encoding a 40% carboxy-end truncation of E from p29D260E. The resulting truncated cDNA clone, p48BSprM60E, encodes a prM-60%E fusion ending with Lys291 of E and was confirmed by restriction digestion and DNA sequence analysis.

### Example 22

### Construction of expression vector pLS6-60%E and secretion of 60%E by Saccharomyces cerevisiae expressing MFα-60%E

An expression vector (pLS6-60%E) was constructed for secretion of the N-terminal 60% (codons 1-291, 60%E) of the DEN-2 PR-159 S1 envelope glycoprotein from *S. cerevisiae.* The 60%E DNA sequences were obtained from plasmid p29D260E, described in example 21, by restriction endonuclease digestion with both *Bgl*II and *Sal*I. The released fragment was isolated by agarose gel electrophoresis and subcloned into the *Bgl*II and *Sal*I sites of pLS6, a yeast expression vector described in example 2. The MFα_{L}-60%E fusion was made such that processing of the MFα propeptide and trimming of a Glu-Ala dipeptide results in 60%E with eight additional N-terminal amino acids encoded by sequences present in the multiple cloning site of the pLS6 vector and the E gene PCR primer adapter (see below) (SEQ ID NO:47 and SEQ ID NO:48). 
ATG .....GCT......GAG GCC TTT AGA TCT CGA GTA CCC GGG ACC ATG ...AAA TAA TAG
Met-18aa Ala-65aa Glu Ala Phe Arg Ser Arg Val Pro Gly Thr Met1...Lys291End End
60%E
Signalase Kex2p DPAP
 Transcription of the gene fusion is driven by the *S. cerevisiae* copper-inducible copper metallothionein (*CUP1*) promoter.

After confirming the DNA sequence of the ligated junctions of expression vector pLS6-60%E, the recombinant DNA was transformed into *S. cerevisiae* strain GL43 (*MATa trp1Δ1 ura3-52 pep4::URA3;* SmithKline Beecham) according to standard protocols (Gutherie & Fink, eds. 1991; Rose et al, 1990). Transformants were selected by their ability to grow on minimal medium (SD medium: Guthrie and Fink, 1991) without tryptophan supplementation.

In order to test for expression and secretion of 60%E, several transformants were grown in small-scale cultures (5 ml medium in 17x150 mm tubes). Single colonies were used to inoculate SD medium and the inoculated cultures were grown to saturation (overnight, 30°C, 220 rpm). 1x10⁸ cells from the overnight culture were used to inoculate 5 ml of minimal SD medium supplemented with Casamino acids (2 g/l; Difco) and CuSO₄ (200 µM). This expression culture was fed with glucose (4 g/l, final concentration) and sodium phosphate (pH 6.7, 20 mM, final concentration) at 24 hours post inoculation and, after 48 hours of growth (30°C, 220 rpm, in 17x150 mm tubes), was harvested by centrifugation. Cell-free spent medium was buffer-exchanged with TEEN (50 mM Tris, 10 mM EDTA, 10 mM EGTA, 150 mM NaCl, pH 8.0) and concentrated 200-fold using Centricon-30 (Amicon) ultrafiltration. An extract of cellular protein was prepared by lysing the yeast cells with vigorous agitation in the presence of glass beads (425-600 µm) and TEEN+PIC (TEEN with 1 µg/ml each of pepstatin and leupeptin and 1 mM phenylmethylsulfonylfluoride) using a Mini Beadbeater apparatus (BioSpec Products, Bartlesville, OK). Samples were endoglycosidase H_{f} digested according to the manufacturer's (New England Biolabs, Beverly, MA) protocol prior to SDS-PAGE analysis. Protein gels were Coomassie-stained directly as well as Western blotted and immunoprobed using anti-DEN2 HMAF.

Negative control yeast carrying the expression vector without a Dengue gene insert secreted no proteins recognized by anti-DEN2 HMAF, while pLS6-60%E medium contained several immunoreactive species. The major band presumably represents full-length 60%E since its apparent molecular weight is approximately 10 kD less than that of recombinant 80%E. A protein band comigrating with this immunoreactive material was visible in Coomassie-stained gels of proteins secreted by pLS6-60%E transformants, but this band was absent in medium of negative control transformants. The immunoblot of proteins secreted by pLS6-60%E transformants evidenced a minor band of apparent molecular weight 6-8 kD larger than 60%E; this likely represents unprocessed MFα propeptide-60%E. The cellular protein extract of pLS6-60%E transformants contained many immunoreactive polypeptides not observed in negative control cells; two of these match the secreted products discussed above.

### Example 23

### Construction of expression vectors pLS6-prM60%E and pLS6-prM(mutSS)60%E, expression of MFα_{L}-prM60%E and MFα_{L}-prM(mutSS)60%E in Saccharomyces cerevisiae, and secretion of 60%B by Saccharomyces cerevisiae expressing MFα_{L}-prM(mutSS)60%E

For expression of DEN-2 PR159/S1 premembrane protein amino acids 1-166 and envelope glycoprotein amino acids 1-291 as a single contiguous open reading frame in *S. cerevisiae,* DNA sequences encoding these proteins were obtained by digestion of plasmid p48BSprM60E (see Example 21) with the restriction enzymes *Bgl*II and *Sal*I. This fragment was cloned into the pLS6 *S. cerevisiae* expression vector (see Example 2) that had been digested with *Bgl*II and *Sal*I. The structure of the resulting gene fusion expression vector, pLS6-prM60%E, was confirmed by restriction digestion and DNA sequence analysis. The N-terminal MFα_{L}-prM fusion amino acid sequence of the MFα_{L}-prM60%E fusion protein are identical to those described for MFα_{L}-prM(mutSS)80%E fusions (Example 15), while the C-terminal amino acid of the fusion protein is Lys₂₉₁ of the dengue envelope glycoprotein.

The pLS6-prM60%E plasmid was transformed into *S. cerevisiae* strain GL43 (*MAT*a *ura*3-52 *trp1Δ1 pep4::URA3*) and screened for 60%E expression as described in Example 11. Proteins secreted into the culture medium as well as cellular proteins were treated with endoglycosidase H_{f} (EndoH, New England Biolabs, Beverly, MA) and separated by SDS-PAGE. Proteins were analyzed by both Coomassie staining of polyacrylamide gels and immunoprobing of Western blots. Similar to the expression of secreted 80%E from pLS6-prM80%E (Example 15), secreted 60%E was not detected on Coomassie stained polyacrylamide gels nor Western blots. An immunoblot of intracellular protein confirmed that the construct (MFα_{L}-prM60%E) was expressed, but the fusion product had not been processed to prM and E (evaluation was performed as described in Example 15).

Because the dengue E signal sequence itself may limit processing of the prME fusion proteins, expression of MFα_{L}-prM(mutSS)60%E was evaluated. The *Bgl*II-*Eco*NI fragment from pLS6-prM(mutSS)100%E-TGA encoding the altered secretion signal peptidase cleavage site (see Example 15) was used to replace the homologous fragment from the pLS6-prM60%E to produce pLS6-prM(mutSS)60%E. The sequence of the expression plasmid was confirmed by restriction digestion and DNA sequence analysis. Plasmid pLS6prM(mutSS)60%E was transformed into the *S. cerevisiae* GL43 strain and transformants were selected as described in Example 11.

Transformants were cultured, induced, and evaluated as described in Examples 11 and 15. In contrast to the expression of MFα_{L}-prM60%E, Western blot analysis of total intracellular proteins from pLS6-prM(mutSS)60%E transformants demonstrated that the transformed cells produce an approximately 40 kilodalton product recognized by anti-DEN2 HMAF. For analysis of secreted proteins, media from induced cultures were concentrated, treated with endoglycosidase H_{f}, and analyzed on Western blots for E antigen. A small amount of processed 60%E could be identified in the culture medium upon immunoprobing with anti-DEN2 HMAF. Thus, the mutagenesis of the signalase cleavage site resulted in greatly enhanced processing of the MFα_{L}-prM(mutSS)60%E product at the prM-E junction which produced secretion of a processed 60%E from the MFα_{L}-prM(mutSS) 60%E in *S. cerevisiae.*

### Example 24

### Construction of expression vector pPIC9-60%E and secretion of 60%E by P. pastoris expressing MFα_{L}-60%E

The expression vector constructed to secrete 60%E from *P. pastoris,* pPIC9-60%E, included the DEN-2 PR-159 S1 envelope glycoprotein amino acids 1-291. As a precursor to this 60%E expression vector, a modified pLS6-60%E plasmid (pLS6-alt60%E), encoding a fusion between MFα_{L} and the amino terminal 60% of the dengue envelope, was constructed that encodes fewer nondengue amino acids between the MFα_{L} and E segments. The 60%E cDNA fragment from pLS6-alt60%E was then transferred to pPIC9.

The sequences in pLS6-alt60%E encoding the dengue E protein amino terminus were derived from pLS6-2x80E, which encodes a tandemly arrayed dimer of 80%E that are linked by a synthetic linker peptide. To convert pLS6-2x80E to pLS6-alt60%E, pLS6-2x80E was digested with *Eco*NI, which cuts within the first member of the 80%E dimer, and *Sal*I, which cuts immediately downstream of the dimer, thereby removing the 3' portion of the first member of the 80E dimer and the second member of the 80E dimer entirely. In its place was ligated an *Eco*NI*-Sal*I fragment from pLS6-prM(mutSS)60E (see Example 23), encoding the 3' portion of 60%E, to complete construction of pLS6-alt60%E.

The minimizing of nondengue codons between MFα_{L} and E, found in pLS6-2x80E, were preserved in pLS6-alt60%E, and, subsequently, in pPIC9-60%E. The codons between MFα_{L} and E were minimized, owing to use of the *Xma*I site in p29D280E (*Xma*I is the first adapter restriction enzyme site upstream of E; see Example 1) and the StuI site in pLS6. To ligate the *Xma*I 5' end of E to the *Stu*I site in the MFα_{L}, the *Xma*I site was treated with Klenow polymerase to make the end blunt during the construction of pLS6-2x80E.

To transfer 60%E from pLS6-alt60%E to pPIC9, pLS6-alt60E was digested by *Xho*I plus *Sal*I, which released a fragment that includes a portion of the MFα leader and the entire 60%E coding region. This fragment was ligated with the *Pichia* expression vector pPIC9 (Invitrogen, San Diego, CA; described in Example 12) that was previously digested with the restriction enzyme *Xho*I. This ligation restored the complete MFα leader sequence including the Kex2 cleavage site. The genetic integrity of the resulting gene fusion expression vector, pPIC9-60%E, was confirmed by restriction digestion and DNA sequence analysis. The partial nucleotide and predicted amino acid sequences of the MFα_{L}-60%E fusion gene are shown below (SEQ ID NO:49 and SEQ ID NO:50): 
ATG ......... GAG GCC GGG ACC ATG ...AAA TAA
Met-18aa-65aa Glu Ala Gly Thr Met1...Lys291 END

Signalase Kex2p DPAP
 The location of the signalase and *Kex2* cleavage sites which remove the pre and pro portions of the MFα leader peptide, respectively, are indicated. The dengue sequences are indicated in bold type. The Met₁ residue is the N-terminal amino acid of the E glycoprotein and Lys₂₉₁ is residue 291 from the amino terminal end of the envelope glycoprotein. The expression of a recombinant product in *Pichia* from the pPIC9 vector is driven by the methanol inducible promoter derived from the *Pichia pastoris aox*1 (alcohol oxidase 1) gene.

The pPIC9-60%E expression vector was transformed into spheroplasts of *P. pastoris* strain GS115 (*his4*) and transformants were selected for their ability to grow on minimal medium without histidine supplementation. Strain GS115 and the protocol used for transformation were obtained from Invitrogen (San Diego, CA). Transformants were tested for their ability to express and secrete 60%E by growing selected clones in small cultures (5 ml). The transformants were grown to saturation (24 to 36 hrs.) in BMGY medium (1% yeast extract, 2% peptone, 100 mM potassium phosphate, pH 6.0, 1.34% yeast nitrogen base without amino acids, 4 x 10⁻⁵% biotin, 1% glycerol). The cells were collected by centrifugation and suspended in one half the original culture volume with BMMY (identical to BMGY except the glycerol component of BMGY is replaced in BMMY with 0.5% methanol) medium and cultured for 48 hrs. Proteins secreted into the culture medium, as well as cellular proteins, were treated with endoglycosidase H_{f} (EndoH, New England Biolabs, Beverly, MA) and separated by SDS-PAGE. Western immunoblots probed with DEN-2 HMAF indicated that the recombinants expressed significant levels of 60%E. Protein gels analyzed by Coomassie staining also showed strong levels of 60%E expression and secretion.

### Example 25

### Construction of expression vector pPIC9-prM(mutSS)60%E and secretion of 60%E by P. pastoris expressing MFα_{L}-prM(mutSS)60%E

To construct clone pPIC9-prM(mutSS)60%E, a strategy identical to that describe in Example 16 was used. Clone pLS6-prM(mutSS)60%E (described in Example 23) was digested with restriction endonuclease *Xho*I and *Xma*I and sequences within the MFα_{L} and dengue cloning adapter were replaced by oligonucleotides to remove an extraneous *Xho*I site, to preserve a critical *Xho*I site, and to regenerate the Kex2 protease process site. The *Xho*I*-Sal*I prM(mutSS)60%E fragment from pLS6Δ-prM(mutSS)60%E was ligated into the unique *Xho*I site of pPIC9. The nucleotide and amino acid sequences at the N-terminus of the fusion protein are identical to that shown in Example 16 for pLS6Δ-prM(mutSS)80%E. The structure of the *Pichia* expression vector pPIC9-prM(mutSS)60%E was confirmed by restriction digestion and DNA sequence analysis.

The pPIC9-prM(mutSS)60%E expression vector was transformed into spheroplasts of *P. pastoris* strain GS115 (*his4*), and transformants were selected and evaluated as described in Example 12. After EndoH_{f} treatment of secreted proteins, a novel Coomassie Blue-staining band could be detected by SDS-PAGE. Immunoprobing with anti-domain B serum of Western blots of proteins prepared from the culture medium detected a diffuse area of reactivity ranging from 40 to 90 kD in the nonEndoH treated samples and a unique band of approximately 40 kD in the EndoH_{f} treated samples that corresponds to the band observed following Coomassie Blue staining.

### Example 26

### Construction of pMttbns-prM100%E, Expression of tPa-prM100%E by Drosophila melanogaster Schneider cells, and Induction of a Virus Neutralizing Response by Immunizing Mice with tPa-prM100%E Membrane Preparations

For expression of DEN-2 PR159/S1 preMembrane protein amino acids 1-166 and Envelope glycoprotein amino acids 1-495 as a single continuous open reading frame in *Drosophila melanogaster* Schneider 2 tissue culture cells, DNA sequences encoding these proteins were obtained by digestion of the p48BSprME13 clone (described in Example 14) with the restriction enzymes BglII and SalI. This fragment was cloned into the unique Bg1II and XhoI restriction sites of plasmid pMttΔXho (described in Example 13).

The cloning junctions of the resulting gene fusion expression vector, pMttprM100E, were confirmed by restriction endonuclease digestion and DNA sequence analysis. The partial nucleotide and predicted amino acid sequences of the tPA₁-prM100E fusion gene are: 
(SEQ ID NO:51 and SEQ ID NO:52)
ATG ............. GGAGCCAGATCTCGAGTACCCGGGACCATG TTT ..ACA ATG ..GCC TAA
Met-20 aa- -11 aa-GlyAlaArgSerArgValProGlyThrMet Phe1..Thr166 Met1 ..Ala495END
pre▲pro-tPA▲ prM 100%E
 The TPA pre- and propeptide regions are delineated by pre^{▲} and pro-tPA^{▲}, respectively, and the dengue sequences are indicated in bold type. The Phe₁ and Thr₁₆₆ residues are the N-terminal and C-terminal amino acid residues of prM, respectively. The Met₁ residue is the N-terminal amino acid of envelope glycoprotein and Ala₄₉₅ is residue 495 from the amino terminal end of the envelope glycoprotein (the carboxy-terminal residue).

As described previously in Example 13, Schneider 2 cells were cotransfected with pMttprM100%E DNA at ratios of 1:1, and 5:1, relative to pCOHygro DNA and selected for growth in medium containing 300 µg/ml hygromycin. Transfectants were induced with 200µM CuSO₄ and expression of prM100%E was examined at various times after induction. Proteins secreted into the culture medium as well as cellular proteins were separated by SDS-PAGE. Protein gels were analyzed by both Coomassie Blue staining and immunoprobing of corresponding Western blots. Analysis of Coomassie Blue-stained SDS-PAGE gels failed to reveal a unique protein band either intracellularly or in the culture medium. However, a novel band was recognized intracellularly by polyclonal anti-dengue 2 hyperimmune mouse ascites fluid (anti-DEN-2 HMAF, gift of R. Putnak, WRAIR) in Western blot analysis. This ^{~}60 kD immunoreactive band comigrated with viral Envelope derived from dengue-2 infected mosquito C6/36 cells suggesting the recombinant 100%E protein had been processed away from the preMembrane protein. Polyclonal antisera to the pr portion of prM (from Peter Wright, Monash University, Australia) recognized a ^{~}20 kD protein, which comigrated with viral prM, confirming that the 100%E produced in the transfected cells was processed from prM. In fact, no evidence of a higher molecular weight band that might correspond to unprocessed prM100%E was detected in any sample, suggesting that the proteolytic processing of prM from E is extremely efficient in Schneider cells. No 100%E was detected in the culture medium indicating that 100%E remains anchored in cell-associated membranes.

Immunoblots probed with anti-DEN-2 HMAF demonstrated that the amount of intracellular 100%E produced by the transfectants increased over time from day 1 post induction to 7 days post induction. The amount of 100%E detected intracellularly in the transfectants correlated with the cotransfection ratio. Sensitivity of the intracellular 100%E to endoglycosidases was evaluated by molecular weight shift of the protein in SDS-PAGE and Western immunoblots following endoglycosidase treatment. Partial resistance of the recombinant 100%E to Endoglycosidase H_{f} (Endo H_{f}; New England Biolabs) digestion indicated that the product contains N-linked glycosylation, and that the composition of the recombinant products probably represents a mixture of complex, high mannose, and/or hybrid glycosylation.

Schneider cells, transformed with pMtt-prM100%E expressing the tissue plasminogen activator leader fusion protein tPA_{L}-prM100%E were cultured in serum-free medium (Excell; JRH Biosciences) and induced by addition of CuSO₄ to a final concentration in the culture medium of 0.2 nM (see Examples 13 and 17 for more detail on culture conditions). The cells were maintained in inducing medium for seven days prior to harvesting. The cells were harvested by centrifugation at 1000 X G in a Beckman TJ-6 refrigerated centrifuge. The cells were washed one time with phosphate buffer saline (PBS), resuspended in PBS and disrupted with a tissue homogenizer. Undisrupted cells were removed by centrifugation at 1000 X G in an Eppendorf microcentrifuge. Cellular membranes were pelleted at 10,000 X G in an Eppendorf microcentrifuge and 100,000 X G in a Beckman L8-80M Ultracentrifuge. The 10,000 X G and 100,000 X G pellets were resuspended in PBS and pooled for mouse immunization. Negative control cells transformed with pCOHygro only (see Example 13) were cultured, induced, and harvested as described for the prM100%E-expressing cells. The immunogens were assayed by Western blot prior to immunization of mice.

Outbred Swiss Webster mice (Simonsen) were immunized intraperitoneally (I.P.) with 75 µg total protein of the prM100%E membrane preparation in Freund's complete adjuvant. Control animals were immunized with either 75 µg of total protein from the negative control membrane preparation or with saline only, each in Freund's complete adjuvant. The mice received three I.P. boosts, consisting of one half the priming dose in Freund's incomplete adjuvant, at two week intervals.

Following the first and second boosts, the animals were test bled (tail bleed) and the immune response was monitored using an indirect ELISA as described in Example 18. One week after the final boost, the animals were bled out and the sera were tested for anti-DEN2 responsiveness by indirect ELISA and PRNT as described in Example 18. Results for the ELISA and PRNT assays are summarized in Table 7. The mice immunized with the membrane preparation containing 100%E developed virus-neutralizing antibodies despite the crude nature of the immunogen, suggesting the utility of these immunogens as efficacious vaccine candidates.

**Table 7**

| **Immune Response of Mice Immunized with Recombinant Dengue prM100%E Membrane Preparation** | | | | | | |
|---|---|---|---|---|---|---|
| **mouse** | **antigen** | **adjuvant** | **20 titer**^{**a**} **ELISA** | **30 titer**^{**b**} **ELISA** | **final titer ELISA** | **PRNT**_{**80**} **titer**^{**b**} |
| 20-1 | Saline | Freund's | <1:50 | <1:50 | <1:100 | <1:10 |
| 20-2 | | | <1:50 | <1:50 | <1:100 | <1:10 |
| 20-3 | | | <1:50 | <1:50 | <1:100 | <1:10 |
| 20-4 | | | <1:50 | <1:50 | <1:100 | <1:10 |
| 20-5 | | | <1:50 | <1:50 | <1:100 | <1:10 |
| 23-1 | pCoHygro membrane | Freund's | <1:50 | <1:50 | <1:100 | <1:10 |
| 23-2 | | | <1:50 | <1:50 | <1:100 | <1:10 |
| 23-3 | | | <1:50 | <1:50 | <1:100 | <1:10 |
| 23-4 | | | <1:50 | <1:50 | <1:100 | <1:10 |
| 23-5 | | | <1:50 | <1:50 | <1:100 | <1:10 |
| 24-1 | prM100%E membrane | Freund's | 1:200 | 1:1,600 | >1:25600 | 1:320 |
| 24-2 | | | <1:50 | >1:1600 | >1:1600 | 1:320 |
| 24-3 | | | <1:50 | >1:1600 | >1:1600 | 1:80 |
| 24-4 | | | <1:50 | >1:1600 | >1:25600 | 1:640 |
| 24-5 | | | <1:50 | <1:50 | 1:400 | 1:20 |

| | | | | | | |
|---|---|---|---|---|---|---|
| ^{a}Indirect ELISA titer using recombinant domain B as coating antigen. | | | | | | |
| ^{b}80% reduction of plaque titer of DEN-2 NGC virus on Vero cells. | | | | | | |

### Example 27

### Immunoaffinity Purification of E and Subunits

### Preparation of immunoaffinity columns:

### Materials:

Cyanogen bromide (CNBr) activated sepharose 4B, lot 17-0430-01 (Pharmacia Biotech, Upsala, Sweden). (HiTrap) Bead size 45-165 µm .
1 mM HCl
Binding buffer (100 mM NaHCO₃, 500 mM NaCl pH 8.3)
Low pH washing buffer (100 mM glycine, pH 2.5)
Equilibration buffer (100 mM phosphate, pH 7.2)
Monoclonal antibody 9D12, 4G2 or other (dengue envelope specific/reactive)

*Procedure:* 0.6g (^{~}2 ml) of freeze-dried HiTrap was suspended in 5 ml of 1 mM HCl for 15 min. The gel was poured onto a sintered glass filter and washed with 120 ml of 1 mM HCl, followed by 10 ml of binding buffer. Monoclonal antibody 9D12 was buffer exchanged into binding buffer using a Centricon 30 (Amicon). The concentration was estimated from its absorbance at 280 nm (A₂₈₀ of 1 mg/ml = 1.4). Approximately 20 mg of antibody was added to washed matrix to a final volume of about 4 ml. The coupling reaction was allowed to proceed at 4°C with end-over-end rotation. The antibody modified gel was then poured into a polypropylene column (Biorad) and washed. Washing involved sequential elution with equilibration buffer - washing buffer - equilibration buffer - binding buffer, for three cycles. The amount of unbound antibody was estimated by measuring absorbance of the eluants at 280 nm. Typically, about 18 mg of antibody bound per 2 ml of gel. The column was stored at 4 °C until used. Prior to running media containing protein, the column was washed with washing buffer followed by equilibration buffer.

*Purification of proteins by immunoaffinity chromatography.* Chromatography was performed using low pressure. Media or buffer was supplied to the antibody-sepharose column by a peristaltic pump (Pharmacia or Gilson). The effluent from the column was monitored for absorbance at 280 nm using a Gilson 112 UV/vis detector equipped with a flow through cell. Media containing classical domain B, domain B+T, MFα80E and 80%E from *Drosophila* cells was circulated through the immunoaffinity column for antigen binding and washed with phosphate buffer pH 7.0. The bound protein is eluted with buffer at pH 2.5.

*Comparison of 9D12, 4G2 and 3H5 MAbs as affinity supports. N*-hydroxysuccinimide (NHS) activated HiTrap columns (1 ml) were reacted with 5 mg of dengue envelope-specific MAb 9D12, 4G2 or 3H5 according to methods provided by the manufacturer. The columns were then tested for their ability to purify DEN-2 80%E from *Drosophila* cell culture medium, using the purification procedure described above. The 4G2 column had a greater capacity than the 9D12 column, while the 3H5 column did not bind significant amounts of DEN-2 80%E. Up to 150 µg of 80%E could be bound by the 4G2 column, while the capacity of the 9D12 column was about 70 µg.

*General protocol for purification of DEN-2 80%E by 4G2 and 9D12 HiTrap columns.* The columns (1 ml or 5 ml bed volume) are perfused at a rate of 1 ml per minute with media containing 80%E adjusted to pH 7.2. Following washing with 10 mM phosphate buffer, pH 7.2, the antigen is eluted with 100 mM glycine pH 2.5. The eluted product is neutralized with 1 M Tris, pH 7.5 (final concentration 0.2 M), and NaCl is added to a final concentration of 150 mM. The sample is then concentrated by membrane ultrafiltration in a Centricon 30 (Amicon).

*Concentration and purity determination.* The concentration of purified 80%E was determined by ELISA, using purified domain B as a standard. The purity of the antigen was estimated by SDS-PAGE followed by Coomassie staining or Western transfer, showing the immunoaffinity purified 80%E antigens to be greater than 98% pure.

*Purification of (MFa*_{*L*}*)80%E from Pichia media.* (MFa_{L})80%E was purified by immunoaffinity chromatography using a MAb 9D12 column as described. For each run, 50 ml of yeast culture medium [^{~}1 µg/ml (MFa_{L})80%E] was cycled for 3 hr through a 2 ml Sepharose column containing 20 mg of MAb 9D12. The column was washed with 5 mM phosphate buffer, pH 7.0, until a baseline absorbance at 280 nm was re-established. The bound protein was eluted with 5 mM phosphate buffer, pH 2.5, then concentrated 20-fold by evaporation using a SpeedVac (Savant), and further concentrated by membrane ultrafiltration using a Centricon 30 (Amicon). The concentration of purified protein was estimated using a MAb 9D12/rabbit anti-domain B sandwich ELISA. This assay incorporates a purified *Pichia*-secreted domain B standard, quantified by amino acid analysis (Protein and Nucleic Acid Facility, Stanford CA). The molar amount of (MFa_{L})80%E eluted from the columns was determined by comparison to a domain B standard curve. The concentration of (MFa_{L})80%E, was estimated by multiplying the concentration of domain B equivalents by five, to compensate for the mass/size difference between the domain B standard and (MFa_{L})80%E. Purity of the (MFa_{L})80%E product was assayed by SDS-PAGE analysis on a 12% gel. Prior to SDS-PAGE analysis, the protein was treated with 5,000 units of Endo Hf (New England Biolabs) for two hours to remove heterogeneous carbohydrates added post-translationally to the molecule by the yeast. The treated protein migrates as a single band of molecular weight approximately 50,000 daltons.

Approximately 0.6 mg of (MFa_{L})80%E was recovered by this method (^{~}50% yield). The purified subunit protein was stored in PBS at 4 °C, until used to immunize mice.

### Example 28

### Ion-Exchange and Size Exclusion Chromatography Purification of Envelope Subunit

Sterile-filtered, frozen stocks of supernatant from *Drosophila* cell cultures grown in a bioreactor were used as starting material for the purification procedure.. The sample was thawed then concentrated and diafiltered using a tangential flow ultrafiltration device (Millipore, Bedford, MA) containing membranes with molecular weight cut-off of 10 kilodaltons. The sample was first concentrated to approximately 200 ml then diafiltered against 10mM Tris-succinate buffer (TSB), pH 8.1. The volume of TSB, pH 8.1, that was used for the diafiltration was at least twice the starting sample volume. Under the described conditions, 80%E was retained by the membranes along with the majority of the *Drosophila* proteins.

The retentate from the diafiltration was loaded directly onto a Fractogel DEAE (E-M Sciences, Gibbstown, NJ) column (1.4cm id. x 5.5cm ht.) that was equilibrated against TSB, pH 8.1. The sample was loaded at a flowrate of 1 ml per minute. Under these loading conditions, all of the 80%E in the sample was bound to the anion-exchange matrix. After the sample was loaded, the column was washed with TSB, pH 8.1, until the absorbance at 280nm was zero. The column was then treated with TSB, pH 8.1, containing 100mM NaCl; this buffer eluted all bound proteins including 80%E. The product from this step contained large amounts of *Drosophila* contaminants; however, a significant contaminating *Drosophila* protein component as well as nonprotein components of the culture media were removed.

The peak fractions from the 100mM NaCl elution profile were then adjusted to pH 5.7 and loaded onto a Fractogel-COO⁻ (E-M Sciences) column (1.4cm id. x 1.6cm ht.) that was equilibrated against TSB, pH 5.7, containing 100mM NaCl. The sample was loaded at a flowrate of 1 ml per minute. Most of the 80%E was bound to the cation-exchange column, while many of the *Drosophila* proteins were not. Once the sample was loaded, the column was washed with TSB, pH 5.7 (no NaCl), followed by TSB, pH 8.1(no NaCl). Bound 80%E was eluted with TSB, pH 8.1, containing 50 mM NaCl. The 80%E fraction from this step was approximately 80% pure.

The Fractogel-COO⁻ column product was further purified by gel filtration chromatography on a BioSep 3000 HPLC column (600x7.8mm, Phenomenex, Torrance, CA). Phosphate buffered saline (25 mM phosphate, 100mM NaCl), pH 6.8, was used as the running buffer at a flow rate of 0.5 ml per minute. Some of the 80%E was eluted in an early peak fraction along with various *Drosophila* contaminants; however, most of the material was eluted in a later peak. The 80%E in the later fraction was >90% pure.

Based on sandwich ELISA and SDS-PAGE analyses, the recombinant Dengue protein retains complete reactivity with MAb 9D12 that recognizes a conformationally sensitive envelope epitope. Reactivity with MAb 9D12 was retained during each step of the purification process.

### Example 29

### Expression and secretion of DEN-2 NS1 from Drosophila cells

A fully sequenced DEN-2 PR159 S1 NS1 clone was used as the starting construct. The plasmid clone, p29D2NS1.2, contains a 1,055 bp fragment that corresponds to nucleotides 2422 and 3477 of the parent DEN-2 genomic map. The 5' end is flanked by a *Bgl*II site and the 3' end is flanked by two stop codons and a *Sal*I site. This *Bgl*II*-Sal*I fragment was cloned into the *Drosophila* expression plasmid pMTT digested with the same two enzymes. Two of the resulting recombinants were selected for further characterization.

Two clones encoding the DEN2 NS1 protein, pMttNS1.1 and pMttNS1.2, were transfected into S2 cells and transfectants selected for hygromycin resistance. The expression of the NS1 protein by the mixed populations was evaluated by Western blot analysis. Immunoreactive product was detected both intracellularly and in the medium, with the vast majority of the product being secreted into the medium. A corresponding Coomassie blue staining band was seen on SDS-PAGE gels. The secreted 80%E product appeared to accumulate in the medium out to at least day 7 post induction. By Western blot analysis, the NS1.2 transfectant population appears to express at slightly higher levels than the NS1.1 transfectant population. The immunoreactive product is recognized by anti-DEN2 HMAF and anti-NS1 MAb 7E11. Digestion of the secreted product with Endoglycosidase H (EndoH) and Peptide:N-glycosidase F (PNGase F) demonstrated that the secreted product is sensitive to both EndoH and PNGase F digestion and therefore is glycosylated. Interestingly, the mobility shift on EndoH digestion is intermediate between the undigested and the PNGase F digested product, although all products are discrete bands on a Western blot. This may suggest that the two glycosylation sites are not identically modified by the secretory pathway of the S2 cells. Estimates of the expression level based upon Coomassie stained gel and Western blot is ^{~}10 µg/ml.

S2 DEN-2 NS1 transfectants were plated at limiting dilutions in supplemented serum-free medium (IPL-41; Life Technologies Inc.). The subcultures were induced with CuSO₄ and the expression levels screened by immuno-dot blot, using MAb 7E11 (NS1 specific) for detection. Several of these cultures expressed and secreted into the medium significant levels of NS1. The highest level expressors will be expanded and a clonal cell population secreting DEN-2 NS1 will be produced.

A bioreactor culture of S2 DEN-2 NS1 cells was grown using the *Drosophila* cell culture methods described above. Based on Western dot blot analysis, we estimate that approximately 3-4-fold more NS1 was produced per unit volume in the bioreactor medium (^{~}10 µg/ml), compared to S2 DEN-2 NS1 cultures grown in tissue culture flasks. Analysis by SDS-PAGE of unpurified S2 DEN-2 NS1 culture medium, heated at 37 °C for 15 minutes in nonreducing buffer, showed a distinct band migrating at ^{~}80-85 kDa, possibly corresponding to NS1 dimer. Western blots employing the anti-NS1 specific MAb, 7E11, confirmed that >90% of the NS1 secreted into culture medium exists as a homodimer. SDS-PAGE/Western blot analysis also indicated that S2 DEN-2 NS1 cells secreted ^{~}3-4--fold higher levels of NS1 dimer when cultured in the bioreactor, compared to culture in flasks.

For nondisruptive HPLC purification preserving the dimer form a Hewlett-Packard Ti-series 1050 HPLC system was employed, with UV detection at 280 nm. Preliminary attempts with hydrophobic interaction chromatography (TOSOHAAS phenyl-5PW) failed to resolve any of the medium components. Thus, an ion exchange method based on Cellufine sulfate (Amicon), was employed. S2 DEN-2 NS1 containing medium from a bioreactor culture was exchanged into 20 mM succinic acid/sodium succinate, pH 6.0, and concentrated using a Minitan tangential flow ultrafiltration unit (Millipore, Bedford, MA) equipped with 10K molecular weight cut-off membranes. The concentrate was applied to a Cellufine sulfate column equilibrated with a running buffer of 20 mM succinic acid/sodium succinate, pH 6.0, and the NS1 protein was eluted with a linear 0--3M NaCl gradient developed over 5 column volumes. NS1 eluted in the 1--1.5 M NaCl fraction and gave nearly complete recovery of ²50% pure NS1 dimer, as judged by SDS-PAGE analysis.

Further purification of the NS1 obtained in the previous paragraph employed Fractogel DEAE (EM Sciences, Gibbstown, NJ) which efficiently removes a major *Drosophila* cell contaminant present in the Cellufine sulfate fractions. Since the calculated isoelectric point of NS1 is similar to that of 80%E, we initially employed Fractogel DEAE column conditions that had been developed for 80%. NS1 medium was exchanged into running buffer, 20 mM Tris succinate, pH 8.1, containing 50 mM NaCl, applied to Fractogel DEAE, then NS1 was eluted with a NaCl step-gradient in pH 8.1 buffer. This protocol resulted in ^{~}50-75% pure NS1 dimer, as determined by SDS-PAGE and Western blot.

## Claims

1. An immunogenic composition which generates protective, neutralizing antibody responses to a *Flavivirus* in a murine host against the homologous *Flavivirus*, said strain of *Flavivirus* selected from the group consisting of a strain of dengue, a strain of Japanese encephalitis virus (JEV), a strain of yellow fever virus (YF), and a strain of tick-borne encephalitis virus (TBE)
which composition contains an adjuvant; and
a portion of the envelope protein (E) of the *Flavivirus* strain against which said responses are sought, which portion is 80% E, wherein said 80% E represents that portion of the envelope protein that constitutes 80% of its length starting from amino acid 1 at its N-terminus and which portion is encoded in a DNA construct operably linked downstream from a secretion leader and secreted as a recombinantly produced protein from *Drosophila* cells.

2. The immunogenic composition of claim 1 wherein the secretion leader is human tissue plasminogen activator prepropeptide secretion leader (tPA_{L}).

3. The immunogenic composition of claim 1 wherein said *Drosophila* cells are D. *melanogaster* Schneider cells.

4. The immunogenic composition of claim 1 or 3 wherein the *Flavivirus* is a dengue virus.

5. The immunogenic composition of claim 1 wherein said adjuvant is an alum adjuvant.

6. The use of a portion of the envelope protein (E), which portion is 80% E, wherein said 80% E represents that portion of the envelope protein that constitutes 80% of its length starting from amino acid 1 at its N-terminus and which portion is encoded in a DNA construct operably linked downstream from a secretion leader and secreted as a recombinantly produced protein from *Drosophila* cells for the preparation of a pharmaceutical composition for protecting a subject against a *Flavivirus* strain, said strain selected from the group consisting of a strain of dengue, a strain of YF, a strain of JEV and a strain of TBE, wherein the composition is administered to a subject in need of such protection in an effective amount.

7. The use of claim 6 wherein said *Flavivirus* is a dengue virus.

8. An immunogenic composition according to claim 1 which generates protective, neutralizing antibody responses to a *Flavivirus* in a murine host which responses confer protection against intracerebral challenge by the homologous *Flavivirus*, said strain of *Flavivirus* selected from the group consisting of a strain of dengue, a strain of Japanese encephalitis virus (JEV), a strain of yellow fever virus (YF), and a strain of tick-borne encephalitis virus (TBE).

9. The immunogenic composition of claim 8 wherein said *Drosophila* cells are Schneider cells.

10. The immunogenic composition of claim 8 wherein said *Flavivirus* is a dengue virus.

11. The immunogenic composition of claim 8 wherein the secretion leader is human tissue plasminogen activator prepropeptide secretion leader (tPA_{L}).

12. The immunogenic composition of claim 8 wherein the adjuvant is an alum adjuvant.

13. The use of a portion of the envelope protein (E), which portion is 80% E, wherein said 80% E represents that portion of the envelope protein that constitutes 80% of its length starting from amino acid 1 at its N-terminus, and which portion is encoded in a DNA construct operably linked downstream from a secretion leader and secreted as a recombinantly produced protein from *Drosophila* cells for the preparation of a pharmaceutical composition for generating a neutralizing antibody response in a subject against a *Flavivirus* strain, said strain selected from the group consisting of a strain of dengue, a strain of YF, a strain of JEV and a strain of TBE, wherein the composition is administered to a subject in need of generating said response in an effective amount.

14. The use of claim 13 wherein said *Flavivirus* is a dengue virus.

15. An expression system for the recombinant production and secretion of a portion of an envelope (E) protein of a *Flavivirus* selected from the group consisting of dengue virus, Japanese encephalitis virus (JEV), tick-borne encephalitis virus (TBE) and yellow fever virus (YF), which expression system comprises *Drosophila* cells modified to contain a DNA molecule which comprises
(a) a first nucleotide sequence encoding said portion of said E protein of the *Flavivirus* strain against which protection is sought, which portion is the N-terminal 80% of the protein from residue 1 to residue 395, and
(b) a second nucleotide sequence which encodes a secretory leader sequence or a secretory signal sequence operably linked to said first nucleotide sequence and positioned so as to produce a fusion protein when said first and said second nucleotide sequences are expressed in a eukaryotic cell, wherein said secretory leader sequence is human tissue plasminogen activator prepropeptide secretion leader (tPA_{L}) and optionally includes the premembrane leader of the E protein,
said encoding sequences operably linked to control sequences capable of effecting expression of said encoding nucleotide sequences in eukaryotic cells.

16. A method to produce a portion of an E protein of a *Flavivirus* selected from the group consisting of dengue virus, Japanese encephalitis virus (JEV), tick-borne encephalitis virus (TBE) and yellow fever virus (YF), which method comprises
(a) culturing the *Drosophila* cells of claim 15 in culture medium under conditions favorable for expression of the encoding nucleotide sequence so that the cells secrete said portion of the E protein of the *Flavivirus* strain against which protection is sought, which portion is the N-terminal 80% of the protein from residue 1 to residue 395 into the medium; and
(b) recovering the portion of the E protein from the culture medium.

17. The expression system of claim 15 wherein the N-terminal 80% of the E protein from residue 1 to residue 395 is dengue virus E protein.

18. The method of claim 16 wherein the N-terminal 80% of the E protein from residue 1 to residue 395 is dengue virus E protein.

19. The expression system of claim 15 wherein the *Drosophila* cells are *Drosophila* Schneider cells.

20. The method of claim 16 wherein the *Drosophila* cells are *Drosophila* Schneider cells.

21. The expression system of claim 17 wherein the *Drosophila* cells are *Drosophila* Schneider cells.

22. The method of claim 18 wherein the *Drosophila* cells are *Drosophila* Schneider cells.

## Patentansprüche

1. Eine immunogene Zusammensetzung, die protektive, neutralisierende Antikörperantworten auf ein *Flavivirus* in einem Mäusewirt gegen das homologe *Flavivirus* hervorruft, wobei der Stamm des *Flavivirus* ausgewählt ist aus der Gruppe bestehend aus einem Denguevirusstamm, einem Stamm des Japan-Enzephalitis-Virus (JEV), einem Stamm des Gelbfiebervirus (GFV) und einem Stamm des Zeckenenzephalitisvirus (TBE),
wobei die Zusammensetzung ein Adjuvans enthält; und
einen Teil des Hüllproteins (E) des *Flavivirus*-Stammes, gegen den diese Antworten gerichtet sein sollen, wobei der Teil 80% E ist, **dadurch gekennzeichnet dass** diese 80% E den Teil des Hüllproteins darstellen, der 80% seiner Länge ausmacht, ausgehend von Aminosäure 1 an seinem N-Terminus, und wobei der Teil in einem DNA-Konstrukt kodiert ist, das funktionell stromabwärts von einem Sekretionsleiter gebunden ist und als rekombinant erzeugtes Protein von *Drosophila*-Zellen sekretiert wird.

2. Immunogene Zusammensetzung gemäss Anspruch 1, **dadurch gekennzeichnet dass** der Sekretionsleiter der Sekretionsleiter des humanen Gewebeplasminogenaktivator-Präpropeptids (tPA_{L}) ist.

3. Immunogene Zusammensetzung gemäss Anspruch 1, **dadurch gekennzeichnet dass** diese *Drosophila*-Zellen *D. melanogaster* Schneider-Zellen sind.

4. Immunogene Zusammensetzung gemäss Anspruch 1 oder 3, **dadurch gekennzeichnet dass** das *Flavivirus* ein Denguevirus ist.

5. Immunogene Zusammensetzung gemäss Anspruch 1, **dadurch gekennzeichnet dass** dieses Adjuvans ein Alaun-Adjuvans ist.

6. Die Verwendung eines Teils des Hüllproteins (E), wobei der Teil 80% E ist, **dadurch gekennzeichnet dass** diese 80% E den Teil des Hüllproteins darstellen, der 80% seiner Länge ausmacht, ausgehend von Aminosäure 1 an seinem N-Terminus, und wobei der Teil in einem DNA-Konstrukt kodiert ist, das funktionell stromabwärts von einem Sekretionsleiter gebunden ist und als rekombinant erzeugtes Protein von *Drosophila*-Zellen sekretiert wird, zur Herstellung einer pharmazeutischen Zusammensetzung zum Schutz eines Subjekts gegen einen *Flavivirus*-Stamm, wobei dieser Stamm ausgewählt ist aus der Gruppe bestehend aus einem Denguevirusstamm, einem GFV-Stamm, einem JEV-Stamm und einem TBE-Stamm, **dadurch gekennzeichnet dass** die Zusammensetzung einem Subjekt, das einen solchen Schutz benötigt, in einer wirksamen Menge verabreicht wird.

7. Verwendung nach Anspruch 6, **dadurch gekennzeichnet dass** das *Flavivirus* ein Denguevirus ist.

8. Immunogene Zusammensetzung gemäss Anspruch 1, die protektive, neutralisierende Antikörperantworten auf ein *Flavivirus* in einem Mäusewirt hervorruft, wobei die Antworten Schutz gegen intrazerebrale Infektion durch das homologe *Flavivirus* vermitteln, wobei dieser *Flavivirus*-Stamm ausgewählt ist aus der Gruppe bestehend aus einem Denguevirusstamm, einem JEV-Stamm, einem GFV-Stamm und einem TBE-Stamm.

9. Immunogene Zusammensetzung gemäss Anspruch 8, **dadurch gekennzeichnet dass** diese *Drosophila*-Zellen *Sc*hneider-Zellen sind.

10. Immunogene Zusammensetzung gemäss Anspruch 8, **dadurch gekennzeichnet dass** dieses *Flavivirus* ein Denguevirus ist.

11. Immunogene Zusammensetzung gemäss Anspruch 8, **dadurch gekennzeichnet dass** der Sekretionsleiter der Sekretionsleiter des humanen Gewebepiasminogenaktivator-Präpropeptids (tPA_{L}) ist.

12. Immunogene Zusammensetzung gemäss Anspruch 8, **dadurch gekennzeichnet dass** das Adjuvans ein Alaunadjuvans ist.

13. Die Verwendung eines Teils des Hüllproteins (E), wobei der Teil 80% E ist, **dadurch gekennzeichnet dass** diese 80% E den Teil des Hüllproteins darstellen, der 80% seiner Länge ausmacht, ausgehend von Aminosäure 1 an seinem N-Terminus, und wobei der Teil in einem DNA-Konstrukt kodiert ist, das funktionell stromabwärts von einem Sekretionsleiter gebunden ist und als rekombinant erzeugtes Protein von *Drosophila*-Zellen sekretiert wird, zur Herstellung einer pharmazeutischen Zusammensetzung zur Erzeugung einer neutralisierenden Antikörperantwort gegen einen *Flavivirus*-Stamm in einem Subjekt, wobei dieser Stamm ausgewählt ist aus der Gruppe bestehend aus einem Denguevirusstamm, einem GFV-Stamm, einem JEV-Stamm und einem TBE-Stamm, wobei die Zusammensetzung einem Subjekt in einer wirksamen Menge verabreicht wird, das die Erzeugung dieser Antwort benötigt.

14. Verwendung gemäss Anspruch 13, **dadurch gekennzeichnet dass** dieses *Flavivirus* ein Denguevirus ist.

15. Ein Expressionssystem zur rekombinanten Herstellung und Sekretion eines Teils des Hüllproteins (E) eines *Flavivirus* ausgewählt aus der Gruppe bestehend aus Denguevirus, Japan-Enzephalitis-Virus (JEV), Zeckenenzephalitisvirus (TBE) und Gelbfiebervirus (GFV), wobei das Expressionssystem *Drosphila*-Zellen umfasst, die modifiziert sind, um ein DNA-Molekül zu enthalten, welches umfasst
(a) eine erste Nukleotidsequenz, den Teil des E Proteins des *Flavivirus*-Stammes, gegen den Schutz erreicht werden soll, kodierend, wobei der Teil die N-terminalen 80% des Proteins von Rest 1 bis Rest 395 ist, und
(b) eine zweite Nukleotidsequenz, eine Sekretionsleitsequenz oder eine Sekretionssignalsequenz kodierend, die funktionell an die erste Nukleotidsequenz gebunden und so positioniert ist, um ein Fusionsprotein zu erzeugen, wenn die erste und zweite Nukleotidsequenz in einer eukaryotischen Zelle exprimiert werden, **dadurch gekennzeichnet dass** der Sekretionsleiter der Sekretionsleiter des humanen Gewebeplasminogenaktivator-Präpropeptids (tPA_{L}) ist und optional den Prämembranleiter des E Proteins einschliesst,
wobei die kodierenden Sequenzen funktionell an Kontrollsequenzen gebunden sind, die in der Lage sind, die Expression der kodierenden Sequenzen in eukaryotischen Zellen zu bewirken.

16. Ein Verfahren zur Herstellung eines Teils eines E Proteins eines *Flavivirus* ausgewählt aus der Gruppe bestehend aus Denguevirus, Japan-Enzephalitis-Virus (JEV), Zeckenenzephalitisvirus (TBE) und Gelbfiebervirus (GFV), wobei das Verfahren umfasst
(a) Kultivierung der *Drosophila*-Zellen gemäss Anspruch 15 in Kulturmedium unter Bedingungen förderlich zur Expression der kodierenden Nukleotidsequenz, so dass die Zellen diesen Teil des E Proteins des *Flavivirus*-Stammes, gegen den Schutz erzielt werden soll, in das Medium sekretieren, wobei der Teil die N-terminalen 80% des Proteins von Rest 1 bis Rest 395 ist; und
(b) Gewinnung des Teils des E Proteins aus dem Kulturmedium.

17. Expressionssystem gemäss Anspruch 15, **dadurch gekennzeichnet dass** die N-terminalen 80% des E Proteins von Rest 1 bis Rest 395 Denguevirus E Protein sind.

18. Verfahren gemäss Anspruch 16, **dadurch gekennzeichnet dass** die N-terminalen 80% des E Proteins von Rest 1 bis Rest 395 Denguevirus E Protein sind.

19. Expressionssystem gemäss Anspruch 15, **dadurch gekennzeichnet dass** die *Drosophila*-Zellen *Drosophila* Schneider-Zellen sind.

20. Verfahren gemäss Anspruch 16, **dadurch gekennzeichnet dass** die *Drosophila*-Zellen *Drosophila* Schneider-Zellen sind.

21. Expressionssystem gemäss Anspruch 17, **dadurch gekennzeichnet dass** die *Drosophila*-Zellen *Drosophila* Schneider-Zellen sind.

22. Verfahren gemäss Anspruch 18, **dadurch gekennzeichnet dass** die *Drosophila-*Zellen *Drosophila* Schneider-Zellen sind.

## Revendications

1. Composition immunogène qui génère des réponses protectrices par anticorps neutralisant à un *Flavivirus* chez un hôte murin contre le *Flavivirus* homologue, ladite souche de *Flavivirus* étant choisie dans le groupe consistant en une souche de la dengue, une souche de virus de l'encéphalite japonaise (JEV), une souche de virus de la fièvre jaune (YF), et une souche de virus de l'encéphalite transmise par des tiques (TBE)
laquelle composition contient un adjuvant et
une portion de la protéine d'enveloppe (E) de la souche de *Flavivirus* contre laquelle on vise lesdites réponses, laquelle portion est de 80% de E, tandis que ledit 80% de E représente la portion de la protéine d'enveloppe qui constitue 80% de sa longueur à partir de l'acide aminé 1 à son extrémité N-terminale et laquelle portion est codée dans un construit d'ADN lié de manière opérationnelle en aval d'une séquence de tête de sécrétion et sécrété sous forme d'une protéine produite par recombinaison à partir de cellules de *Drosophila*.

2. Composition immunogène selon la revendication 1, dans laquelle la séquence de tête de sécrétion est la séquence de tête de sécrétion prépropeptidique de l'activateur tissulaire du plasminogène humain (tPA_{L}).

3. Composition immunogène selon la revendication 1, dans laquelle lesdites cellules de *Drosophila* sont des cellules de Schneider de *D. melanogaster.*

4. Composition immunogène selon la revendication 1 ou 3, dans laquelle le *Flavivirus* est un virus de la dengue.

5. Composition immunogène selon la revendication 1, dans laquelle ledit adjuvant est un adjuvant d'alun.

6. Utilisation d'une portion de la protéine d'enveloppe (E), laquelle portion est de 80% de E, tandis que lesdits 80 % de E représentent la portion de la protéine d'enveloppe qui constitue 80 % de sa longueur à partir de l'acide aminé 1 à son extrémité N-terminale, et laquelle portion est codée dans un construit d'ADN lié de manière opérationnelle en aval d'une séquence de tête de sécrétion et sécrétée sous forme d'une protéine produite par recombinaison à partir de cellules de *Drosophila* pour la préparation d'une composition pharmaceutique pour la protection d'un sujet contre une souche de *Flavivirus,* ladite souche étant choisie dans le groupe consistant en une souche de la dengue, une souche de YF, une souche de JEV et une souche de TBE, tandis que la composition est administrée à un sujet ayant besoin d'une telle protection en une quantité efficace.

7. Utilisation selon la revendication 6, dans laquelle ledit *Flavivirus* est un virus de la dengue.

8. Composition immunogène selon la revendication 1, qui génère des réponses protectrices par anticorps neutralisant à un *Flavivirus* dans un hôte murin, lesquelles réponses confèrent une protection contre un défi intracérébral par le *Flavivirus* homologue, ladite souche de *Flavivirus* étant choisie dans le groupe consistant en une souche de la dengue, une souche de virus de l'encéphalite japonaise (JEV), une souche de virus de la fièvre jaune (YF), et une souche de virus de l'encéphalite transmise par des tiques (TBE).

9. Composition immunogène selon la revendication 8, dans laquelle lesdites cellules de *Drosophila* sont des cellules de Schneider.

10. Composition immunogène selon la revendication 8, dans laquelle ledit *Flavivirus* est un virus de la dengue.

11. Composition immunogène selon la revendication 8, dans laquelle la séquence de tête de sécrétion est une séquence de tête de sécrétion prépropeptidique de l'activateur tissulaire du plasminogène humain (tPA_{L}).

12. Composition immunogène selon la revendication 8, dans laquelle l'adjuvant est un adjuvant d'alun.

13. Utilisation d'une portion de la protéine d'enveloppe (E), laquelle portion est de 80% de E, tandis que lesdits 80 % de E représentent la portion de la protéine d'enveloppe qui constitue 80 % de sa longueur à partie de l'acide aminé 1 à son extrémité N-terminale, et laquelle portion est codée dans un construit d'ADN lié de manière opérationnelle en aval d'une séquence de tête de sécrétion et sécrétée sous forme d'une protéine produite par recombinaison à partir de cellules de *Drosophila* pour la préparation d'une composition pharmaceutique pour le déclenchement d'une réponse par anticorps neutralisant chez un sujet contre une souche de *Flavivirus*, ladite souche étant choisie dans le groupe consistant en une souche de la dengue, une souche de YF, une souche de JEV et une souche de TBE, tandis que la composition est administrée à un sujet ayant besoin du déclenchement de ladite réponse en une quantité efficace.

14. Utilisation selon la revendication 13, dans laquelle ledit *Flavivirus* est un virus de la dengue.

15. Système d'expression pour la production par recombinaison et la sécrétion d'une portion d'une protéine d'enveloppe (E) d'un *Flavivirus* choisi dans le groupe consistant en le virus de la dengue, le virus de l'encéphalite japonaise (JEV), le virus de l'encéphalite transmise par des tiques (TBE) et le virus de la fièvre jaune (YF), lequel système d'expression comprend des cellules de *Drosophila* modifiées pour contenir une molécule d'ADN qui comprend
(a) une première séquence nucléotidique codant pour ladite portion de ladite protéine E de la souche de *Flavivirus* contre laquelle une protection est visée, laquelle portion est constituée des 80% N-terminaux de la protéine du résidu 1 au résidu 395, et
(b) une deuxième séquence nucléotidique qui code pour une séquence de tête séccrétoire ou une séquence-signal sécrétoire liée de manière opérationnelle à ladite première séquence nucléotidique et positionnée de manière à produire une protéine de fusion quand ladite première et ladite deuxième séquences nucléotidiques sont exprimées dans une cellule eucaryote, tandis que ladite séquence de tête sécrétoire est une séquence de tête de sécrétion prépropeptidique de l'activateur tissulaire du plasminogène humain (tPA_{L}) et comporte en option la séquence de tête de prémembrane de la protéine E,
lesdites séquences codantes étant liées de manière opérationnelle à des séquences de contrôle capables d'influencer l'expression des dites séquences nucléotidiques codantes dans des cellules eucaryotes.

16. Procédé pour la production d'une portion d'une protéine E d'un *Flavivirus* choisi dans le groupe consistant en le virus de la dengue, le virus de l'encéphalite japonaise (JEV), le virus de l'encéphalite transmise par des tiques (TBE) et le virus de la fièvre jaune (YF), lequel procédé comprend
(a) la culture des cellules de *Drosophila* selon la revendication 15 dans un milieu de culture dans des conditions favorables pour l'expression de la séquence nucléotidique codante de telle manière que les cellules sécrètent ladite portion de la protéine E de la souche de *Flavivirus* contre laquelle une protection est visée, laquelle portion est constituée des 80% N-terminaux de la protéine du résidu 1 au résidu 395 dans le milieu; et
(b) la récolte de la portion de la protéine E à partir du milieu de culture.

17. Système d'expression selon la revendication 15, dans lequel les 80% N-terminaux de la protéine E du résidu 1 au résidu 395 sont constitués de la protéine E du virus de la dengue.

18. Procédé selon la revendication 16, dans lequel les 80% N-terminaux de la protéine E du résidu 1 au résidu 395 sont constitués de la protéine E du virus de la dengue.

19. Système d'expression selon la revendication 15, dans lequel les cellules de *Drosophila* sont des cellules de *Drosophila* de Schneider.

20. Procédé selon la revendication 16, dans lequel les cellules de *Drosophila* sont des cellules de *Drosophila* de Schneider.

21. Système d'expression selon la revendication 17, dans lequel les cellules de *Drosophila* sont des cellules de *Drosophila* de Schneider.

22. Procédé selon la revendication 18, dans lequel les cellules de *Drosophila* sont des cellules de *Drosophila* de Schneider.
